(19) Europäisches Patentamt
European Patent Office
Office européen des brevets

(11) EP 1 165 505 B1

(12) **EUROPEAN PATENT SPECIFICATION**

(45) Date of publication and mention of the grant of the patent:
**08.09.2004 Bulletin 2004/37**

(21) Application number: **00921654.0**

(22) Date of filing: **03.04.2000**

(51) Int Cl.[7]: **C07C 323/62**, C07D 233/61, C07D 207/26, C07D 295/18, C07D 213/74, C07D 211/22, C07D 207/14, C07D 211/46, C07D 211/62, C07D 211/60, C07D 401/04, C07D 217/02, C07D 307/68, C07D 295/26, C07D 317/66, C07D 209/08, C07D 295/205, C07D 401/12, A61K 31/165, A61K 31/33, A61P 29/00

(86) International application number:
**PCT/US2000/008895**

(87) International publication number:
**WO 2000/059880 (12.10.2000 Gazette 2000/41)**

(54) **CELL ADHESION-INHIBITING ANTIINFLAMMATORY AND IMMUNE-SUPPRESSIVE COMPOUNDS**

ZELLADHÄSION INHIBIERENDE ENTZÜNDUNGSHEMMENDE UND IMMUNSUPPRESSIVE VERBINDUNGEN

COMPOSES INHIBITEUR DE L'ADHESION CELLULAIRE, ANTI-INFLAMMATOIRES ET IMMUNOSUPPRESSEURS

(84) Designated Contracting States:
**AT BE CH CY DE DK ES FI FR GB GR IE IT LI LU MC NL PT SE**
Designated Extension States:
**AL LT LV MK RO SI**

(30) Priority: **02.04.1999 US 286645**
**29.12.1999 US 474517**
**31.03.2000 US 541795**

(43) Date of publication of application:
**02.01.2002 Bulletin 2002/01**

(73) Proprietor: **ABBOTT LABORATORIES**
**Abbott Park, IL 60064-6050 (US)**

(72) Inventors:
- **LINK, James**
  **Evanston, IL 60201 (US)**
- **LIU, Gang**
  **Gurnee, IL 60031 (US)**
- **PEI, Zhonghua**
  **Libertyville, IL 60048 (US)**
- **VON GELDERN, Thomas, W.**
  **Richmond, IL 60071 (US)**
- **WINN, Martin**
  **Deerfield, IL 60015 (US)**
- **XIN, Zhili**
  **Lake Bluff, IL 60044 (US)**
- **WANG, Sheldon**
  **Carmel, IN 46032 (US)**
- **BOYD, Steven, A.**
  **Longmont, CO 80503 (US)**
- **ZHU, Gui-Dong**
  **Gurnee, IL 60030 (US)**
- **FREEMAN, Jennifer, C.**
  **Grayslake, IL 60030 (US)**
- **GUNAWARDANA, Indrani, W.**
  **Libertyville, IL 60048 (US)**
- **STAEGER, Michael, A.**
  **Greenfield, WI 53221 (US)**
- **JAE, Hwan-Soo**
  **Glencoe, IL 60022 (US)**
- **LYNCH, John, K.**
  **Kenosha, WI 53142 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 165 505 B1

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

(56) References cited:
**WO-A-00/39081** **WO-A-98/54207**
**US-A- 5 208 253**

- **A. FRANKE, ET AL.: "Synthetische Juvenilhormone. 1. p-Substituierte beta-Methyl-Zimtsäurederivate" HELVETICA CHIMICA ACTA, vol. 58, no. 1, 29 January 1975 (1975-01-29), pages 268-278, XP002142990 Verlag Helvetica Chimica Acta, Basle, CH ISSN: 0018-019X**

## Description

### Technical Field

The present invention relates to compounds that are useful for treating inflammatory and immune diseases, to pharmaceutical compositions comprising these compounds, and to the use of these compounds for preparing medicaments for inhibiting inflammation or suppressing immune response in a mammal.

### Background

Inflammation results from a cascade of events that includes vasodilation accompanied by increased vascular permeability and exudation of fluid and plasma proteins. This disruption of vascular integrity precedes or coincides with an infiltration of inflammatory cells. Inflammatory mediators generated at the site of the initial lesion serve to recruit inflammatory cells to the site of injury. These mediators (chemokines such as IL-8, MCP-1, MIP-1, and RANTES, complement fragments and lipid mediators) have chemotactic activity for leukocytes and attract the inflammatory cells to the inflamed lesion. These chemotactic mediators which cause circulating leukocytes to localize at the site of inflammation require the cells to cross the vascular endothelium at a precise location. This leukocyte recruitment is accomplished by a process called cell adhesion.

Cell adhesion occurs through a coordinately regulated series of steps that allow the leukocytes to first adhere to a specific region of the vascular endothelium and then cross the endothelial barrier to migrate to the inflamed tissue (Springer, T.A., 1994, Traffic Signals for Lymphocyte Recirculation and Leukocyte Emigration: The Multistep Paradigm, Cell 76: 301-314; Lawrence, M. B., and Springer, T. A., 1991, Leukocytes' Roll on a Selectin at Physiologic Flow Rates: Distinction from and Prerequisite for Adhesion Through Integrins, Cell.65: 859-873; von Adrian, U., Chambers, J. D., McEnvoy, L.M., Bargatze, R.F., Arfos, K.E, and Butcher, E.C., 1991, Two-Step Model of Leukocyte-Endothelial Cell Interactions in Inflammation, Proc. Natl. Acad. Sci. USA 88: 7538-7542; and Ley, K., Gaehtgens, P., Fennie, C., Singer, M.S., Lasky, L.H. and Rosen, S.D., 1991, Lectin-Like Cell Adhesion Molecule 1 Mediates Rolling in Mesenteric Venules *in vivo*, Blood.77: 2553-2555). These steps are mediated by families of adhesion molecules such as integrins, Ig supergene family members, and selectins which are expressed on the surface of the circulating leukocytes and on the vascular endothelial cells. The first step consists of leukocytes rolling along the vascular endothelial cell lining in the region of inflammation. The rolling step is mediated by an interaction between a leukocyte surface oligosaccharide, such as Sialylated Lewis-X antigen (SLe*), and a selectin molecule expressed on the surface of the endothelial cell in the region of inflammation. The selectin molecule is not normally expressed on the surface of endothelial cells but rather is induced by the action of inflammatory mediators such as TNF-$\alpha$ and interleukin-1. Rolling decreases the velocity of the circulating leukocyte in the region of inflammation and allows the cells to more firmly adhere to the endothelial cell. The firm adhesion is accomplished by the interaction of integrin molecules that are present on the surface of the rolling leukocytes and their counter-receptors (the Ig superfamily molecules) on the surface of the endothelial cell. The Ig superfamily molecules or CAMs (Cell Adhesion Molecules) are either not expressed or are expressed at low levels on normal vascular endothelial cells. The CAM's, like the selectins, are induced by the action of inflammatory mediators like TNF-alpha and IL-1. The final event in the adhesion process is the extravasation of leukocytes through the endothelial cell barrier and their migration along a chemotactic gradient to the site of Inflammation. This transmigration is mediated by the conversion of the leukocyte integrin from a low avidity state to a high avidity state. The adhesion process relies on the induced expression of selectins and CAM's on the surface of vascular endothelial cells to mediate the rolling and firm adhesion of leukocytes to the vascular endothelium.

The interaction of the intercellular adhesion molecule ICAM-1 (cd54) on endothelial cells with the integrin LFA-1 on leukocytes plays an important role in endothelial-leukocyte contact. Leukocytes bearing high-affinity LFA-1 adhere to endothelial cells through interaction with ICAM-1, initiating the process of extravasation from the vasculature into the surrounding tissues. Thus, an agent which blocks the ICAM-1/LFA-1 interaction suppresses these early steps in the inflammatory response. Consistent with this background, ICAM-1 knockout mice have numerous abnormalities in their inflammatory responses.

The present invention discloses compounds which bind to the interaction-domain (I-domain) of LFA-1, thus interrupting endothelial cell-leukocyte adhesion by blocking the interaction of LFA-1 with ICAM-1, ICAM-3, and other adhesion molecules. These compounds are useful for the treatment or prophylaxis of diseases in which leukocyte trafficking plays a role, notably acute and chronic inflammatory diseases, autoimmune diseases, tumor metastasis, allograft rejection, and reperfusion injury. The compounds of this invention are diaryl sulfides, which are substituted with a cinnamide moiety. The cinnamide functionality may be placed either ortho- or para- to the linking sulfur atom, although para-substitution is preferable. Appropriate substitution of both aromatic rings is tolerated, and can be used to modulate a variety of biochemical, physicochemical and pharmacokinetic properties. In particular the amide moiety is readily modified; a variety of secondary and tertiary amides are active, and alternatively a heterocyclic ring may be

attached at this position. Modifications of this amide functionality are particularly useful in modulating physicochemical and pharmacokinetic properties.
Moreover, A. Franks et al., Helv. Chimica Acta, vol. 58, No. 1, 268-298 (1975) discloses p-substituted, β-methyl cinnamic derivatives and their use as synthetic juvenile hormone derivatives; WO 98/54907 discloses anti-inflammatory tyrosine derivatives; US Patent No. 5,208,253 discloses 3-alkyloxy, aryloxy or arylalkyloxy-benzo[b]thiophenyl-2-carboxamides suitable as agents which block leukocyte adherence to vascular endothelian; and WO 00/39081 (relevant under Art. 54(3) EPC) discloses cinnamides having cell adhesion anti-inflammatory activity.

## Summary of The Invention

The present invention provides

(1) compounds of formula (I) below,

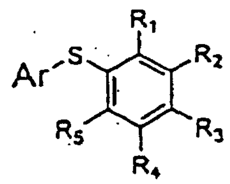

I

wherein $R_1$, $R_2$, $R_3$, $R_4$ and $R_5$ are independently selected from

a. hydrogen,
b. halogen,
c. alkyl,
d. haloalkyl,
e. alkoxy,
f. cyano,
g. nitro,
h. carboxaldehyde, and

with the proviso that at least one of $R_1$ or $R_3$ is a "*cis*-cinnamide" or a "*trans*-cinnamide", defined as

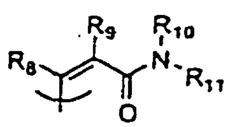

"*cis*-cinnamide"

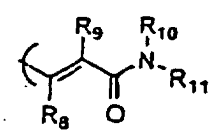

"*trans*-cinnamide",

wherein
$R_8$ and $R_9$ are independently selected from

a. hydrogen,
b. alkyl,
c. carboxy alkyl,
d. alkylaminocarbonyl alkyl, and
e. dialkylaminocarbonyl alkyl,
and
$R_{10}$ and $R_{11}$ are independently selected from

a. hydrogen,
b. alkyl,
c. cycloalkyl,
d. alkoxycarbonylalkyl,
e. hydroxyalkyl,
f. an aryl group being a mono- or bicyclic carbocyclic ring system having one or two aromatic rings which can be fused to a cyclohexane, cyclohexene, cyclopentane or cyclopentene ring, and having substituents independently selected from alkyl, halogen, hydroxy and alkoxy,
g. heterocyclyl representing a 4-, 5-, 6- or 7-membered ring which contains one, two or three heteroatoms independently selected from nitrogen, oxygen and sulfur, which can be fused to one or two rings independently selected from an aryl ring as defined below, a cyclohexane ring, a cyclohexene ring, a cyclopentane ring, a cyclopentene ring or another monocyclic heterocyclic ring,
h. heterocyclylalkyl, where the heterocyclyl is as defined above,
i. heterocyclylamino, where the heterocyclyl is as defined above,
j. a heterocyclyl group as defined in g. above having substituents independently selected from alkyl, halogen, hydroxy, alkoxy, carboxy, carboxyalkyl and alkoxycarbonyl,
k. a heterocyclylalkyl group as defined in h. above having substituents independently selected from alkyl, halogen, hydroxy, alkyoxy, carboxy, carboxyalkyl and alkoxycarbonyl, or

where $NR_{10}R_{11}$ is a heterocyclyl or substituted heterocyclyl group where the heterocyclyl group is as defined g. above and the substituents are independently selected from

1) alkyl,
2) alkoxy,
3) alkoxyalkyl,
4) cycloalkyl,
5) an aryl group being a mono- or bicyclic carbocyclic ring system having one or two aromatic rings which can be fused to a cyclohexane, cyclohexene, cyclopentane or cyclopentene ring,
5) an aryl group being a mono- or bicyclic carbocyclic ring system having one or two aromatic rings which can be fused to a cyclohexane, cyclohexene, cyclopentane or cyclopentene ring,
6) a heterocyclyl group as defined above,
7) heterocyclylcarbonyl, where the heterocyclyl is as defined above,
8) heterocyclylalkylaminocarbonyl, where the heterocyclyl is as defined above,
9) hydroxy,
10) hydroxyalkyl,
11) hydroxyalkoxyalkyl,
12) carboxy,
13) carboxyalkyl,
14) carboxycarbonyl,
15) carboxaldehyde,
16) alkoxycarbonyl,
17) arylalkoxycarbonyl, where the aryl is as defined above,
18) aminoalkyl,
19) aminoalkanoyl,
20) carboxamido,
21) alkoxycarbonylalkyl,
22) carboxamidoalkyl,
23) cyano,
24) tetrazolyl,
25) tetrazolyl substituted with alkoxy, alkyl, halogen, hydroxy, carboxy, carboxyalkyl or alkoxycarbonyl,
26) alkanoyl,
27) hydroxalkanoyl,
28) alkanoyloxy,
29) alkanoylamino,
30) alkanoyloxyalkyl,
31) alkanoylaminoalkyl,
32) sulfonate,
33) alkylsulfonyl,

34) alkysulfonylaminocarbonyl,
35) arylsulfonylaminocarbonyl, where the aryl is as defined above, and
36) heterocyclylsulfonylaminocarbonyl, where the heterocyclyl group is as defined above,

and wherein Ar is a substituted aryl or substituted heteroaryl group, which has one or two aromatic rings and which can be fused to a cyclohexane, cyclohexene, cyclopentane or cyclopentene ring, where substitutions are independently selected from

a. a heterocyclyl group as defined above having substuents independently selected from alkyl, halogen, hydroxyl, alkoxy, carboxy, carboxyalkyl and alkoxycarbonyl,
b. a heterocyclylalkyl group as defined above having substituents independently selected from carboxy, carboxyalkyl and alkoxycarbonyl,
c. carboxyalkoxy,
d. carboxythioalkoxy,
e. carboxycycloalkoxy,
f. thioalkyl; and
g. carboxyalkylamino;

or a pharmaceutically acceptable salt thereof;
(2) a compound selected from the group consisting of:

(2-Methoxyphenyl) [2,3-dichloro-4-(E-(((4-hydroxylaminocarbonyl)piperidin-1-yl)carbonyl)ethenyl)phenyl] sulfide;
(2-Methoxyphenyl) [2,3-dichloro-4-(E-((N-carboxymethyl-N-phenylamino)carbonyl)ethenyl)phenyl] sulfide;
(2-Methoxyphenyl) [3-chloro-6-hydroxy-4-(E-((3-carboxypiperidin-1-yl)carbonyl)ethenyl)phenyl]sulfide;
(2-Methoxyphenyl) [2,3-dichloro-4-(E-(4-((1-(2-phenyl-1-carboxyethyl)amino)carbonyl)piperidin-1-yl)carbonyl)ethenyl)phenyl]sulfide;
(2-Methoxyphenyl) [2,3-dichloro-4-(E-(4-((1-(2-hydroxy-1-carboxyethyl)amino)carbonyl)piperidin-1-yl)carbonyl)ethenyl)phenyl]sulfide;
(3-(4-Pyrrolidin-1-yl)piperidin-1-yl)phenyl) [2,3-dichloro-4-(E-(((3-(2-pyrrolidinon-1-yl)propylamino)carbonyl) ethenyl)phenyl]sulfide;
[3-(4-(Spiro-2,2-dioxolanyl)piperidin-1-yl)phenyl] [2,3-dichloro-4-(E-((4-morpholinyl)carbonyl)ethenyl)phenyl] sulfide;
(2-(2-Carboxy)ethenyl)phenyl) [2,3-dichloro-4-(E-((4-morpholinyl)carbonyl)ethenyl)phenyl]sulfide;
[2-(4-Acetylpiperazin-1-yl)phenyl] [2,3-dichloro-4-(E-[(4-carboxypiperidin-1-yl)carbonyl]ethenyl)phenyl] sulfide; and
(2-Methoxyphenyl) [2,3-dichloro-4-(E-((((4-carboxyphenyl)methyl)amino)carbonyl)ethenyl)phenyl]sulfide.

(3) a pharmaceutical composition comprising a compound of formula (I) as defined in (1) above or a compound as defined in (2) above;
(4) use of a compound as defined in (1) or (2) above for preparing a medicament for inhibiting inflammation;
(5) use of a compound as defined in (1) or (2) above for preparing a medicament for suppressing immune response;
(6) a compound of formula II

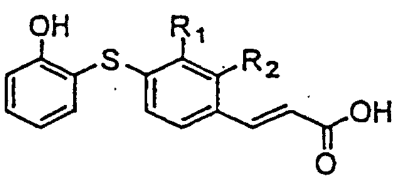

II

wherein $R_1$ and $R_2$ are independently selected from

i. hydrogen,

j. halogen,

k. alkyl,

l. haloalkyl,

m. alkoxy,

n. cyano,

o. nitro, and

p. carboxaldehyde ; and

(7) a process for preparing a compound of forrnula II

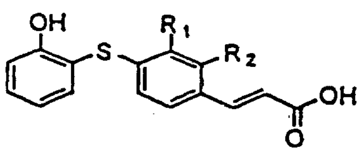

. II

which comprises:

a) reacting a compound of formula II'

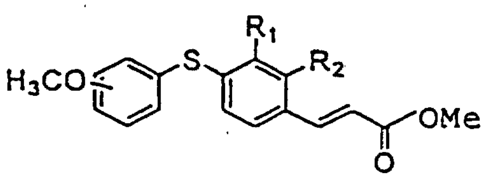

II'

with lithium hydroxide, and

b) cleaving the resulting methyl ether ,

where $R_1$ and $R_2$ are as defined in (6) above.

Detailed Description

The term "alkanoyl" as used herein refers to an alkyl group attached to the parent molecular group through a carbonyl group.

The term" alkanoylamino" as used herein refers to an alkanoyl group attached to the parent molecular group though an amino group.

The term "alkanoylaminoalkyl" as used herein refers to an alkanoylamino group attached to the parent molecular group through an alkyl group.

The term "alkanoyloxy" as used herein refers to an alkanoyl group attached to the parent molecular group through an oxygen radical.

The term " alkanoyloxyalkyl" as used herein refers to an alkanoyloxy group attached to the parent molecular group through an alkyl group.

The term "alkoxy" as used herein refers to an alkyl group attached to the parent molecular group through an oxygen atom.

The term "alkoxyalkoxy" as used herein refers to an alkoxy group attached to the parent molecular group through an alkoxy group.

The term "alkoxyalkyl" as used herein refers to an alkoxy group attached to the parent molecular group through an alkyl group.
The term "alkoxycarbonyl" as used herein refers to an alkoxy group attached to the parent molecular group through a carbonyl group.
The term "alkoxycarbonylalkyl" as used herein refers to an alkoxycarbonyl . group attached to the parent molecular group through an alkyl group.
The term "alkyl" as used herein refers to a saturated straight or branched chain group of 1-10 carbon atoms derived from an alkane by the removal of one hydrogen atom.
The term " alkyl(alkoxycarbonylalkyl)amino" as used herein refers to an amino group substituted with one alkyl group and one alkoxycarbonylalkyl group.
The term " alkyl(alkoxycarbonylalkyl)aminoalkyl" as used herein refers to an alkyl(alkoxycarbonylalkyl)amino group attached to the parent molecular group through an alkyl group.
The term "alkylene" as used herein refers to a divalent group of 1-10 carbon atoms derived from a straight or branched chain alkane by the removal of two hydrogen atoms.
The term "alkylsulfonyl" as used herein refers to an alkyl radical attached to the parent molecular group through an $-SO_2-$ group.
The term "alkylsulfonylaminocarbonyl" as used herein refers to an alkylsulfonyl group attached to the parent molecular group through an aminocarbonyl group.
The term "amino" as used herein refers to a radical of the form $-NR_{18}R_{19}$, or to to a radical of the form $-NR_{18}-$, where $R_{18}$ and $R_{19}$ are independently selected from hydrogen, alkyl or cycloalkyl.
The term "aminoalkanoyl" as used herein refers to to an amino group attached to the parent molecular group through an alkanoyl group.
The term "aminoalkyl" as used herein refers to an amino group attached to the parent molecular group through an alkyl group.
The term "aminocarbonyl" as used herein refers to an amino group attached to the parent molecular group through a carbonyl group.
The term "aryl" as used herein refers to a mono- or bicyclic carbocyclic ring system having one or two aromatic rings. The aryl group can also be fused to a cyclohexane, cyclohexene, cyclopentane or cyclopentene ring. The aryl groups of this invention can be optionally substituted with alkyl, halogen, hydroxy, carboxy, or alkoxy substituents.
The term "arylalkoxy" as used herein refers to an aryl group attached to the parent molecular group through an alkoxy group.
The term "arylalkoxycarbonyl" as used herein refers to an arylalkoxy group attached to the parent molecular group through a carbonyl group.
The term "arylsulfonyl" as used herein refers to an aryl radical attached to the parent molecular group through an $-SO_2-$ group.
The term "arylsulfonylaminocarbonyl" as used herein refers to an arylsulfonyl group attached to the parent molecular group through an aminocarbonyl group.
The term "carboxaldehyde" as used herein refers to the radical -CHO.
The term "carboxaldehyde hydrazone" as used herein refers to the radical $- CH=N-NR_{20}R_{21}$, where $R_{20}$ and $R_{21}$ are independently selected from hydrogen, alkyl or cycloalkyl.
The terms "carboxamide" or "carboxamido" as used herein refer to an amino group attached to the parent molecular group through a carbonyl group.
The term "carboxamidoalkyl" as used herein refers to a carboxamido group attached to the parent molecular group through an alkyl group.
The term "carboxy" as used herein refers to the radical -COOH.
The term "carboxyalkyl" as used herein refers to a carboxy group attached to the parent molecular group through a alkyl group.
The term "carboxycarbonyl" as used herein refers to a carboxy group attached to the parent molecular group through a carbonyl group.
The term "cyano" as used herein refers to the radical -CN.
The term "cycloalkyl" as used herein refers to a monovalent saturated cyclic or bicyclic hydrocarbon group of 3-12 carbons derived from a cycloalkane by the removal of a single hydrogen atom. Cycloalkyl groups may be optionally substituted with alkyl, alkoxy, halo, or hydroxy substituents.
The terms "halo" or "halogen" as used herein refers to F, Cl, Br, or I.
The term "haloalkyl" as used herein refers to an alkyl group substituted with one or more halogen atoms.
The terms "heterocycle" or "heterocyclyl" represent a 4-, 5-, 6- or 7-membered ring containing one, two or three heteroatoms independently selected from the group consisting of nitrogen, oxygen and sulfur. The 4- and 5-membered rings have zero to two double bonds and the 6- and 7-membered rings have zero to three double bonds. The

term "heterocycle" or "heterocyclic" as used herein additionally refers to bicyclic, tricyclic and tetracyclic groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring. Heterocycles include acridinyl, benzimidazolyl, benzofuryl, benzothiazolyl, benzothienyl, benzoxazolyl, biotinyl, cinnolinyl, dihydrofuryl, dihydroindolyl, dihydropyranyl, dihydrothienyl, dithiazolyl, furyl, homopiperidinyl, imidazolidinyl, imidazolinyl, imidazolyl, indolyl, isoquinolyl, isothiazolidinyl, isothiazolyl, isoxazolidinyl, isoxazolyl, morpholinyl, oxadiazolyl, oxazolidinyl, oxazolyl, piperazinyl, piperidinyl, pyranyl, pyrazolidinyl, pyrazinyl, pyrazolyl, pyrazolinyl, pyridazinyl, pyridyl, pyrimidinyl, pyrimidyl, pyrrolidinyl, pyrrolidin-2-onyl, pyrrolinyl, pyrrolyl, quinolinyl, quinoxaloyl, tetrahydrofuryl, tetrahydroisoquinolyl, tetrahydroquinolyl, tetrazolyl, thiadiazolyl, thiazolidinyl, thiazolyl, thienyl, thiomorpholinyl, triazolyl.

Heterocyclics also include bridged bicyclic groups where a monocyclic heterocyclic group is bridged by an alkylene group such as

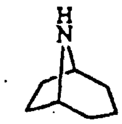, 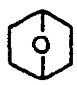, 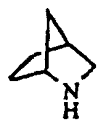.

Heterocyclics also include compounds of the formula

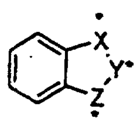

where X* and Z* are independently selected from $-CH_2-$, $-CH_2NH-$, $-CH_2O-$, -NH- and -O-, with the proviso that at least one of X* and Z* is not $-CH_2-$, and Y* is selected from - C(O)- and $-(C(R'')_2)_v$ -, where R" is hydrogen or alkyl of one to four carbons, and v is 1-3. These heterocycles include 1,3-benzodioxolyl, 1,4-benzodioxanyl, 1,3-benzimidazol-2-one and the like. The heterocycle groups of this invention can be optionally substituted with alkoxy, alkyl, halogen, hydroxy, carboxy, carboxyalkyl, or alkoxycarbonyl substituents.

The term "heterocyclylalkyl" as used herein refers to an heterocyclic group attached to the parent molecular group through an alkyl group.

The term "heterocyclylalkylamino" as used herein refers to an heterocyclylalkyl group attached to the parent molecular group through an amino group.

The term "heterocyclylalkylaminocarbonyl" as used herein refers to a heterocyclylalkyl amino group attached to the parent molecular group through a carbonyl group.

The term "heterocyclylamino" as used herein refers to a heterocyclyl group attached to the parent molecular group through a amino group.

The term "heterocyclylcarbonyl" as used herein refers to a heterocyclyl group attached to the parent molecular group through a carbonyl group.

The term "heterocyclylsulfonyl" as used herein refers to a heterocyclyl radical attached to the parent molecular group through an $-SO_2-$ group.

The term "heterocyclylsulfonylaminocarbonyl" as used herein refers to a heterocyclylsulfonyl group attached to the parent molecular group through an aminocarbonyl group.

The term "hydroxyalkanoyl" as used herein refers to an hydroxy radical attached to the parent molecular group through an alkanoyl group.

The term "hydroxyalkoxy" as used herein refers to an hydroxy radical attached to the parent molecular group through an alkoxy group.

The term "hydroxyalkoxyalkyl" as used herein refers to an hydroxyalkoxy group attached to the parent molecular group through an alkyl group.

The term "hydroxyalkyl" as used herein refers to an hydroxy radical attached to the parent molecular group through an alkyl group.

The term "hydroxyalkylaminocarbonyl" as used herein refers to an hydroxyalkyl group attached to the parent molecular group through an aminocarbonyl group.

The term "perfluoroalkyl" as used herein refers to an alkyl group in which all of the hydrogen atoms have been replaced by fluoride atoms.

The term "phenyl" as used herein refers to a monocyclic carbocyclic ring system having one aromatic ring. The phenyl group can also be fused to a cyclohexane or cyclopentane ring. The phenyl groups of this invention can be optionally substituted with alkyl, halogen, hydroxy or alkoxy substituents.

The term "sulfonate" as used herein refers to the radical $-SO_3H$

The term "tetrazole" or "tetrazolyl" as used herein refers to the heterocyclic radical $-CN_4H$.

The term "thioalkoxy" as used herein refers to an alkyl group attached to the parent molecular group through a sulfur atom.

Compounds of the present invention can exist as stereoisomers wherein asymmetric or chiral centers are present. These compounds are designated by the symbols "R" or "S," depending on the configuration of substituents around the chiral carbon atom. The present invention contemplates various stereoisomers and mixtures thereof. Stereoisomers include enantiomers and diastereomers, and mixtures of enantiomers or diastereomers are designated (± ). Individual stereoisomers of compounds of the present invention can be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns.

Geometric isomers can also exist, in the compounds of the present invention. The present invention contemplates the various geometric isomers and mixtures thereof resulting from the arrangement of substituents around a carbon-carbon double bond or arrangement of substituents around a carbocyclic ring. Substituents around a carbon-carbon double bond are designated as being in the Z or E configuration wherein the term "Z" represents substituents on the same side of the carbon-carbon double bond and the term "E" represents substituents on opposite sides of the carbon-carbon double bond. The arrangement of substituents around a carbocyclic ring are designated as cis or trans wherein the term "cis" represents substituents on the same side of the plane of the ring and the term "trans" represents substituents on opposite sides of the plane of the ring. Mixtures of compounds wherein the substituents are disposed on both the same and opposite sides of plane of the ring are designated cis/trans.

As is apparent from the foregoing descriptions, the compounds of Formula 1 are useful in a variety of forms, i.e., with various substitutions as identified. Examples of particularly desirable compounds are quite diverse, and many are mentioned herein. Included are compounds in which $R_1$ is a "cis-cinnamide" or a "trans-cinnamide", and $R_3$ is hydrogen; or where $R_3$ is a "cis-cinnamide" or a "trans-cinnamide", and $R_1$ is hydrogen, or $R_1$, $R_2$, and $R_4$ are each independently hydrogen or alkyl, and $R_5$ is halogen, haloalkyl or nitro. Further preferred compounds include those as above wherein $R_{10}$ and $R_{11}$ are each independently hydrogen, alkyl, cycloalkyl, alkoxycarbonylaalkyl, hydroxyalkyl, or heterocyclylalkyl, or where $NR_{10}R_{11}$ is heterocyclyl or substituted heterocyclyl, and where Ar is aryl, substituted aryl, heteroaryl, or substituted heteroaryl.

Compounds of the present invention include:

(2-Methoxyphenyl)[2,3-bis(trifluoromethyl)-4-(E-((4-phenylcarboxypiperidin-1-yl)carbonyl)ethenyl)phenyl]sulfide;
(2-Methoxyphenyl) (2,3-dichloro-4-(*E*-(((4-hydroxylaminocarbonyl)piperidin-1-yl)carbonyl)ethenyl)phenyl] sulfide;
(2-Methoxyphenyl) [2,3-dichloro-4-(*E*-((N-carboxymethyl-N-phenylamino)carbonyl)ethenyl)phenyl] sulfide;
(2-Methoxyphenyl) [3-chloro-6-hydroxy-4-(E-((3-carboxypiperidin-1-yl)carbonyl)ethenyl)phenyl]sulfide;
(2-Methoxyphenyl) [2,3-dichloro-4-(*E*-(4-((1-(2-phenyl-1-carboxyethyl)amino)carbonyl)piperidin-1-yl)carbonyl) ethenyl)phenyl]sulfide;
(2-Methoxyphenyl) [2,3-dichloro-4-(*E*-(4-((1-(2-hydroxy-1-carboxyethyl)amino)carbonyl)piperidin-1-yl)carbonyl) ethenyl)phenyl]sulfide;
(3-(1-(3-Carboxypiperidinyl)phenyl)[2,3-dichloro-4-(E-((1,2,5,6-tetrahydropyridin-1-yl)carbonyl)ethenyl)phenyl] sulfide;
(3-(4-Pyrrolidin-1-yl)piperidin-1-yl)phenyl) [2,3-dichloro-4-(E-(((3-(2-pyrolidinon-1-yl)propylamino)carbonyl)ethenyl)phenyl]sulfide;
[3-(4-(Spiro-2,2-dioxolanyl)piperidin-1-yl)phenyl][2,3-dichloro-4-(E-((4-morpholinyl)carbonyl)ethenyl)phenyl] sulfide;
[3-(3-Carboxylpiperidin-1-yl)phenyl] [2,3-dichloro-4-(*E*-[(4-carboxypiperidin-1-yl)carbonyl]ethenyl)phenyl] sulfide;
(2-(2-Carboxy)ethenyl)phenyl) [2,3-dichloro-4-(E-((4-morpholinyl)carbonyl)ethenyl)phenyl]sulfide;
[3-(4-Carboxylpipcridin-1-yl)phenyl] [2,3-dichloro-4-(*E*-[(1,2,3,6-tetrahydropyridine)-1-yl)carbonyl]ethenyl)phenyl] sulfide;
[3-(4-Carboxylpiperidinyl)phenyl] [2,3-dichloro-4-(*E*-[(4-morpholinyl)carbonyl]ethenyl)phenyl] sulfide;
[2-(4-Acetylpiperazin-1-yl)phenyl] [2,3-dichloro-4-(*E*-[(4-carboxypiperidin-1-yl)carbonyl]ethenyl)phenyl] sulfide;

3-(3-Carboxypiperidin-1-yl)phenyl) [2,3-dichloro-4-(*E*-[(4-morpholinyl)carbonyl)ethenyl)phenyl] sulfide;
[3-(4-Carboxypiperidin-1-yl)phenyl] [2,3-dichloro-4-(*E*-[(4-(dimethylaminosulfamoyl)piperazin-1-yl)carbonyl]ethenyl)phenyl] sulfide;
(2-Methoxyphenyl)[2,3-bis(trifluoromethyl)-4-(E-((3-carboxypiperidin-1-yl)carbonyl)ethenyl)phenyl]sulfide;
(2-Methoxyphenyl) [2,3-bis(trifluoromethyl)-4-(E-((2-carboxypyrrolidin-1-yl)carbonyl)ethenyl)phenyl ]sulfide;
[3-(4-Carboxypiperidin-1-yl)phenyl] [2,3-dichloro-4-(*E*-((4-((trifluoromethylsulfanyl)piperazin-1-yl)carbonyl)ethenyl)phenyl] sulfide;
(2-Methoxyphenyl)[2,3-dichloro-4-(*E*-(piperidin-1-ylcarbonyl)ethenyl)phenyl] sulfide;
(2-Hydroxyphenyl) [2,3-dichloro-4-(E-((4-morpholino)carbonyl)ethenyl)phenyl]suifide;
(2-Methoxyphenyl) [2,3-dichloro-4-(E-((((4-carboxyphenyl)methyl)amino)carbonyl)ethenyl)phertyl]sulfide;
(2-Methoxyphenyl) [2,3-dichloro-4-(E-(((4-pyrrolidin-1-yl)piperidin-1-yl)carbonyl)ethenyl)phenyl]sulfide;
(2-Hydroxyphenyl)[2,3-dichloro-4-(E-((4- carboxypiperidin-1-yl)carbonyl)ethenyl)phenyl]sulfide;
[3-(4-Carboxypiperidin-1-yl)phenyl] [2,3-dichloro-4-(*E*-((4-[(methylsulfonyl)piperazin-1-yl)carbonyl)ethenyl)phenyl] sulfide;
(2-Aminophenyl) (2,3-dichloro-4-(*E*-((4-morpholinyl)carbonyl)ethenyl)phenyl] sulfide;
(3-(4-carboxypiperidin-1-yl)phenyl)[2,3-dichloro-4-(*E*-((S-oxothiomorpholin-1-yl)carbonyl)ethenyt)pltenyl] sulfide;
[3-(4-Carboxypiperidin-1-yl)phenyl] [2,3-dichloro-4-(*E*-((4-hydroxypiperidin-1-yl)carbonyl)ethenyl)phenyl] sulfide;
(2-Glycoxyphenyl) [2,3-dichloro-4-(E-((4-morpholino)carbonyl)ethenyl)phenyljsulfide;
(2-(4-Butyroxy)phenyl)(2,3-dichloro-4-(E-{(4-morpholino)carbonyl)ethenyl)phenyllsulfide;
[3-(4-Carboxypiperidin-1-yl)phenyl] [2,3-dichloro-4-(*E*-((4-hydroxyethylpiperazin-1-yl)carbonyl)ethenyl)phenyl] sulfide;
[3-(4-Carboxypiperidin-1-yl)phenyl] (2,3-dichloro-4-(*E*-((4-furoylpiperazin-1-yl)carbonyl)ethenyl)phenyl] sulfide;
[3-(4-Carboxypiperidin-1-yl)phenyl][2,3-dichloro-4-(*E*-((pyrrolidin-1-yl)carbonyl)ethenyl)phenyl] sulfide;
[3-(4-Carboxypiperidin-1-yl)phenyl] [2,3-dichloro-4-(*E*-((diethylaminocarbonyl)ethenyl)phenyl] sulfide;
[3-(4-Carboxypiperidin-1-yl)phenyl][2,3-dichioro-4-(*E*-((4-ethylpiperazinyl)carbonyl)ethenyl)phenyl] sulfide;
[3-(4-Carboxypiperidin-1-yl)phenyl][2,3-dichloro-4-(*E*-((4-(aminocarbonyl)piperidin-1-yl)carbonyl)ethenyl)phenyl] sulfide;
[3-(4-Carboxypiperidin-1-yl)phenyl] [2,3-dichloro-4-(*E*-((4-(2-(ethoxyethyl)piperidin-1-yl)carbonyl)ethenyl)phenyl] sulfide;
[3-((4-Carboxymethyl)piperazin-1-yl)phenyl] [(2,3-dichloro-4-(E-(4-morpholinyl)carbonyl)ethenyl)phenyl] sulfide;
[3-(4-Carboxypiperidin-1-yl)phenyl] [2,3-bis(trifluoromethyl)-4-(E-((4-morpholino)carbonyl)ethenyl)phenyl]sulfide;
(3-Hydroxyphenyl) [2,3-dichloro-4-(E-((4-morpholino)carbonyl)ethenyl)phenyl]sulfide;
[3-(4-Butyroxy)phenyl] [2,3-dichloro-4-(E-((4-morpholino)carbonyl)ethenyl)phenyl]sulfide;
(2-Hydroxyphenyl) (2,3-bis(trifluoromethyl)-4-(E-((4-morpholino)carbonyl)ethenyl)phenyl]sulfide;
(3-Hydroxyphenyl)( 2,3-bis(trifluoromethyl)-4-(E-((4-morpholino)carbonyl)ethenyl)phenyl]sulfide;
[3-(4-Carboxypiperidin-1-yl)phenyl] [2,3-ditrifluoromethyl-4-(*E*-((4-hydroxypiperidin-1-yl)carbonyl)ethenyl)phenyl] sulfide;
[3-(4-Carboxypiperidin-1-yl)phenyl) [2,3-ditrifluoromethyl-4-(*E*-((1,2,5,6-tetrahydropyridin-1-yl)carbonyl)ethenyl) phenyl] sulfide;
[2-((4-Carboxy)butyloxy)phenyl] [2,3-dichloro-4=(E-((4-morpholino)carbonyl)ethenyl)phenyl]sulfide;
(2-Glycoxyphenyl) [2,3-bis(trifluoromethyl)-4-(E-((4-morpholino)carbonyl)ethenyl)phenyl]sulfide;
(2-(4-Butyroxy)phenyl)[2,3-bis(trifluoromethyl)-4-(E-((4-morpholino)carbonyl)ethenyl)phenyl]sulfide;
(3-(4-Carboxypiperidin-1-yl)phenyl] [2,3-ditrifluoromethyl-4-(*E*-((bis-(2-ethoxyethyl)amino)carbonyl)ethenyl)phenyl] sulfide;
[3-(4-Carboxypiperidin-1-yl)phenyl] [2,3-bis-(trifluoromethyl)-4-(*E*-((bis-(2-hydroxypropyl)amino)carbonyl)ethenyl) phenyl] sulfide;
[3-(4-Carboxypiperidin-1-yl)phenyl] [2,3-bis-(trifluoromethyl)-4-(*E*-((piperazin-1-yl)carbonyl)ethenyl)phenyl] sulfide;
(3-(4-Butyroxy)phenyl)[ 2,3-bis(trilluoromethyl)-4-(E-((4-morpholino)carbonyl)ethenyl)phenyl]sulfide;
[2-(3-Carboxypiperidin-1-yl)phenyl] (2,3-dichloro-4-(*E*-[(3-(2-pyrrolidinon-1-yl)propylaminocarbonyl)ethenyl)phenyl] sulfide;
[2-(3-Carboxypiperidin-1-yl)phenyl] [2,3-bis(trifluoromethyl)-4-(*E*-[(3-(2-pyrrolidinon-1-yl)propylaminocarbonyl) ethenyl)phenyl] sulfide;
[2-(3-Carboxypiperidin-1-yl)phenyl] [2,3-dichloro-4-(*E*-((4-(2-hydroxyethyl)piperazin-1-yl)carbonyl)ethenyl)phenyl] sulfide;
[2-(3-Carboxypiperidin-1-yl)phenyl] [2,3-bis(trifluoromethyl)-4-(*E*-((1,2,5,6-tetrahydropyridin-1-yl)carbonyl)ethenyl)phenyl] sulfide;
[2-(3-Carboxypiperidin-1-yl)phenyl] [2,3-bis(trifluoromethyl)-4-(*E*-((4-(2-hydroxyethyl)piperazin-1-yl)carbonyl) ethenyl)phenyl] sulfide;

[2-(3-Carboxypiperidin-1-yl)phenyl] [2,3-bis(trifluoromethyl)-4-(*E*-((4-(2-(hydroxyethoxy)ethyl)piperazin-1-yl)carbonyl)ethenyl)phenyl] sulfide; and
(3-(3-Propioxy)phenyl) [2,3-dichloro-4-(E-((4-morpholino)carbonyl)ethenyl)phenyl]sulfide.

Pharmaceutical Compositions

The present invention also provides pharmaceutical compositions which comprise compounds of the present invention formulated together with one or more pharmaceutically-acceptable carriers. The pharmaceutical compositions may be specially formulated for oral administration in solid or liquid form, for parenteral injection, or for rectal administration.

The pharmaceutical compositions of this invention can be administered to humans and other animals orally, rectally, parenterally, intracistemally, intravaginally, intraperitoneally, topically (as by powders, ointments, or drops), bucally, or as an oral or nasal spray. The term "parenteral" administration as used herein refers to modes of administration which include intravenous, intramuscular, intraperitoneal, intrasternal, subcutaneous and intraarticular injection and infusion.

Pharmaceutical compositions of this invention for parenteral injection comprise pharmaceutically-acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol,

and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid.

It may also be desirable to include isotonic agents such as sugars, sodium chloride, and the like, Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically-acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidone, sucrose, and acacia, (c) humectants such as glycerol, (d) disintegrating agents such as agar-agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (I) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions

which can be used include polymeric substances and waxes.

The active compounds can also be in micro-encapsulated form, if appropriate, with one or more of the above-mentioned excipients.

Liquid dosage forms for oral administration include pharmaceutically-acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, com, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters; microcrystalline cellulose, aluminum metahydroxide, bentonite, agar-agar, and tragacanth, and mixtures thereof.

Compositions for rectal or vaginal administration are preferably suppositories which can be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any nontoxic, physiologically-acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

The compounds of the present invention may be used in the form of pharmaceutically -acceptable salts derived from inorganic or organic acids. By "pharmaceutically-acceptable salt" is meant those salts which are, within the scope of sound medical judgment, suitable for use in contact with the tissues of humans and lower animals without undue toxicity, irritation, allergic response, and are commensurate with a reasonable benefit/risk ratio. Pharmaceutically-acceptable salts are well-known in the art. For example, S. M. Berge, *et al.* Describe pharmaceutically-acceptable salts in detail in J. Pharmaceutical Sciences, 1977, 66: 1 et seq. The salts may be prepared *in situ* during the final isolation and purification of the compounds of the invention or separately by reacting a free base function with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides.

Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically-acceptable basic addition salts include cations based on alkali metals or alkaline earth metals such as lithium, sodium, potassium, calcium, magnesium and aluminum salts and nontoxic quaternary ammonia and amine cations including ammonium, tetramethylammonium, tetraethylammonium, methylamine, dimethylamine, trimethylamine, triethylamine, diethylamine, ethylamine. Other representative organic amines useful for the formation of base addition salts include ethylenediamine, ethanolamine, diethanolamine, piperidine, piperazine .

Dosage forms for topical administration of a compound of this invention include powders, sprays, ointments and inhalants. The active compound is mixed under sterile conditions with a pharmaceutically-acceptable carrier and any needed preservatives, buffers, or propellants which may be required. Opthalmic formulations, eye ointments, powders and solutions are also contemplated as being within the scope of this invention.

. Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of 0.1 to 50 mg, more preferably of 5 to 20 mg of active compound per kilogram of body weight per day are administered orally or intravenously to a mammalian patient. If desired, the effective daily dose may be divided into multiple doses for purposes of administration, e.g. two to four separate doses per day.

Preparation of Compounds of the Invention

The compounds and processes of the present invention may be better understood in connection with the following synthetic Schemes which illustrate the methods by which the compounds of the invention can be prepared.

Scheme 1

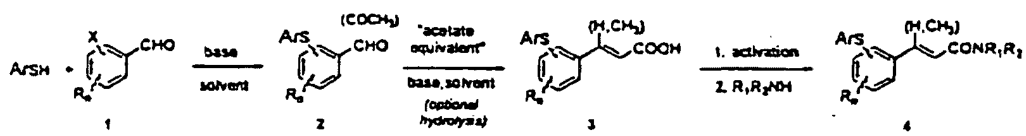


1 2 3 4

Scheme 1 describes the synthesis of a typical cinnamide-substituted diaryl sulfide 4 through an aldehyde intermediate **2**. Aldehyde **2** is prepared by reaction of a thiophenol (for example 2,4-dichlorothiophenol, 2-bromothiophenol, or the like) with halo-substituted benzaldehyde derivative **1** (e.g. 2-chlorobenzaldehyde, 3-chloro,4-fluorobenzaldehyde) in the presence of base (e.g. sodium carbonate, triethylamine,) and a polar solvent (e.g. dimethylformamide, dimethylsulfoxide,). The aldehyde group is homologated to the corresponding cinnamic acid **3**, using an acetate equivalent (for example, malonic acid, triethoxyphosphonoacetate) in the presence of an appropriate base and solvent. In some cases, it may be necessary to hydrolyze an intermediate ester (for example using sodium hydroxide in alcohol). The acid group is activated (for example using thionyl chloride, or dicyclohexylcarbodiimide and N-hydroxysuccinimide, or the like) and reacted with a primary or secondary amine (for example, 6-aminohexanol, pyrrolidone-3-propylamine) to provide the desired analog **4**. In one variant, a halo-acetophenone can replace benzaldehyde 2; the resultant cinnamides **4** are substituted with a methyl group at the 3-position.

Scheme 2

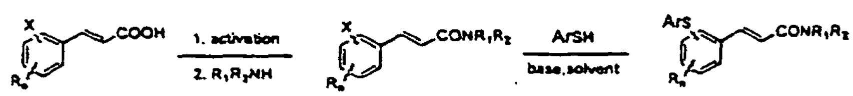


5 6 7

Alternatively, the order of these coupling steps may be reversed (Scheme 2). A substituted halocinnamic acid **5** (e.g. 3-chloro-2-nitrocinnamic acid) may be coupled with a primary or secondary amine (e.g. N-acetylpiperazine) as described above to give the corresponding amide **6**. The halo-group can then be displaced with a substituted thiophenol in the presence of base to provide the product **7**.

## Scheme 3

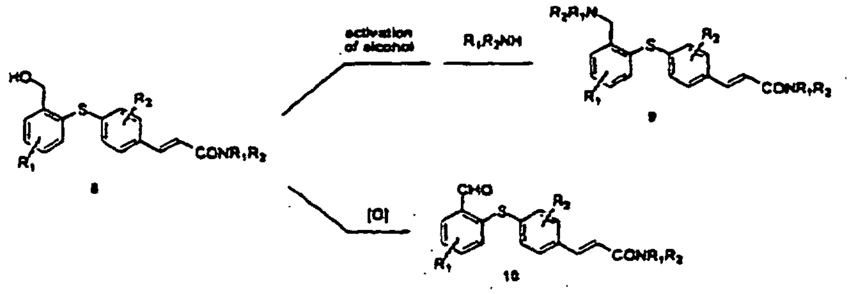


A number of the compounds described herein may be prepared from intermediate benzylic alcohols like **8** (Scheme 3) Activation of the alcohol moiety (for example, using phosphorus tribromide or methanesulfonyl chloride and lithium halide in dimethylformamide) and displacement with a primary or secondary amine (e.g. morpholine, N-formylpiperazine) provides analogs with structures related to **9**. Alternatively the alcohol may be oxidized (for example using TPAP or PCC) to give aldehyde **10**.

## Scheme 4

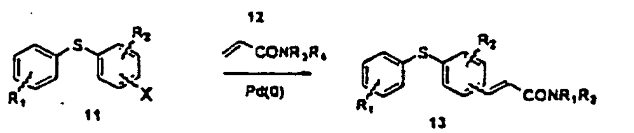


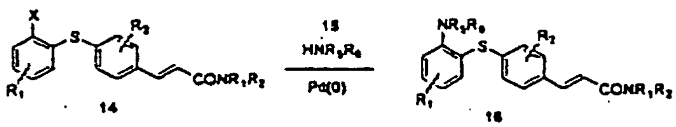


Cinnamides like **13** may be prepared from halo-substituted derivatives **11** by palladium-mediated coupling [e. g. using tetrakis (o-tolyl phosphine) palladium (0). $Pd_1(dba)_3$] with acrylamide derivatives **12** (Scheme 4). In similar manner, anilino-cinnamides like **16** can be prepared by palladium-mediated coupling of amines **15** with halo-cinnamides **14**.

## Scheme 5

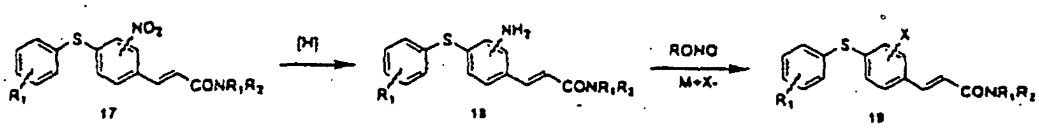


In some cases, functional groups on the aromatic rings can be modified to produce new analogs (Scheme 5). For example, a nitro group in compounds like **17** may be reduced (for example, with tin(II) chloride, or by catalytic hydrogenation.)

to the corresponding amine **18**. This amine may then itself be converted to a halogen, for example by diazotization using nitrous acid or t-butyl nitrite in the presence of a metal halide salt like cupric bromide, providing analog **19**.

Scheme 6

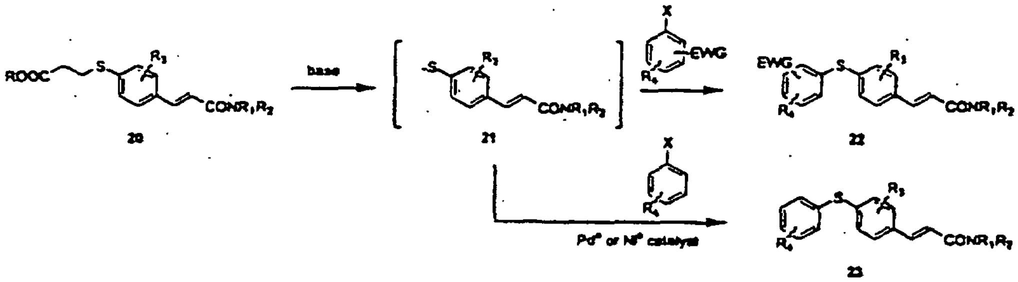


20 21 22 23

It is also possible to assemble cinnamide-substituted diaryl sulfides in a "reverse" sense (Scheme 6). Thus, for example, compound **20**, prepared as described in Scheme 1, may be deprotected by treatment with base (e.g. potassium t-butoxide) to provide thiolate anion **21**, which may be reacted with an activated haloarene (e.g. 2,3-dichlorobenzaldehyde, 3-chloro,4-fluorobenzaldehyde) to provide the corresponding product **22**. Alternatively, this same thiolate anion may be coupled with unactivated aryl halides (e.g. aryl bromide or Aryl iodides) using a metal-catalyzed Ullman coupling procedure (for example, using a palladium or nickel catalyst) to give product **23**.

A further method for producing diarylsulfide cinnamides is shown in Scheme 7, wherein the diaryl sulfide is formed through coupling of a suitably protected aryl thiol **28** to an activated cinnamate ester **27**. Substituted phenol **24** may be brominated to give bromophenol **25**. Heck-type coupling of bromide **25** with an appropriate olefinic substrate, for example methyl acrylate, is effected with palladium catalysis, leading to the cinnamate ester **26**. The phenol is then activated towards further reaction, for example by conversion to the corresponding triflate **27** under standard conditions. The required protected thiol **28** may be prepared by the method of XXX *(Tetrahedron Lett.* **1994**, *35*, 3221-3224), by coupling an aryl halide or triflate with triisopropylsilyl thiol under palladium catalysis. The two partners **27** and **28** are then reacted in the presence of a fluoride source, for example cesium fluoride, to provide the diarylsulfide cinnamate **29**. Hydrolysis is accomplished by basic media, such as lithium or sodium hydroxide in water-THF, and the resulting acid **30** is coupled to amines under standard amide-bond forming conditions (for example, EDC/HOBt) to produce the amides **31**.

Scheme 7

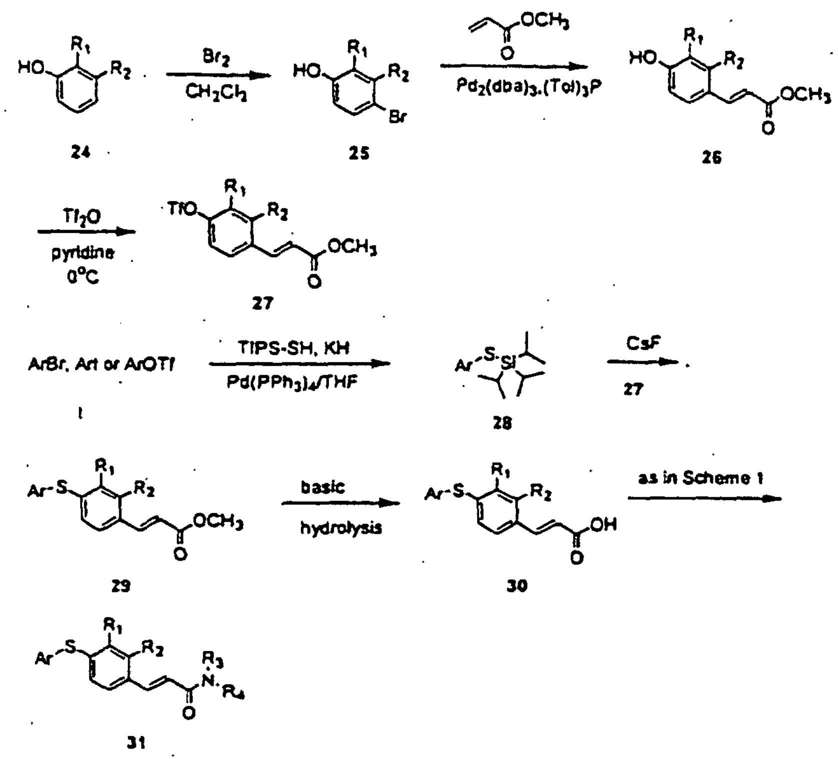


24 25 26 27 1 28 29 30 31

A method for preparing cinnamides bearing two arylthio groups is outlined in Scheme 8. Commercially available difluoro cinnamic acid **32** was coupled with an amine, using standard conditions, and this derived amide **33** was reacted with excess aryl thiol to provide the bis-sulfide **34.**

Scheme 8

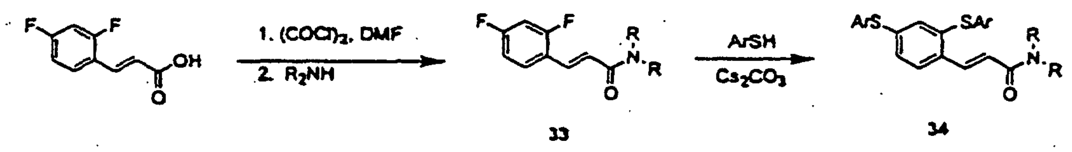


33 34

Compounds which contain trifluoromethyl groups on the cinnamide-portion of inhibitors were made by the method shown in Scheme 9. According to the method of XXX (Ref), Diels-Alder reaction between 1,1,1 ,4,4,4-hexafluoro-2-butyne and 2-methylfuran led to bicyclic ether **35**, which was rearranged with Lewis acid (for example, boron trifluoride etherate) to the phenol **36**. The methyl group is then converted to the corresponding aldehyde **37** by bromination followed by reaction with dimethylsulfoxide. Using the analogous procedures described for Scheme 1 above, the phenol was activated and condensed with thiols under basic conditions to afford diarylsulfide aldehydes **38**, and further converted to cinnamides **39** by the previously described procedures.

Scheme 9

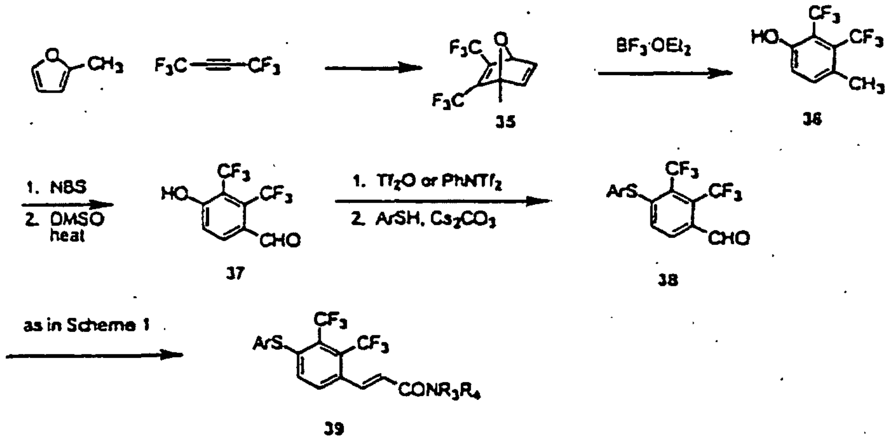


Cinnamides bearing more complex substituted piperidine amides can be produced by the methods outlined in Scheme 10 and 11. Cinnamic acids **40** are coupled to spiro-hydantoin piperidine **41**, and the derived amide **42** is first reacted with an activating reagent (for example di-tert-butyt dicarbonate), and then hydrolyzed to the amino acid **43**. The derived amino group may then be reacted further, for example with acid anhydrides or acid chlorides, to produce amides **44**.

Scheme 10

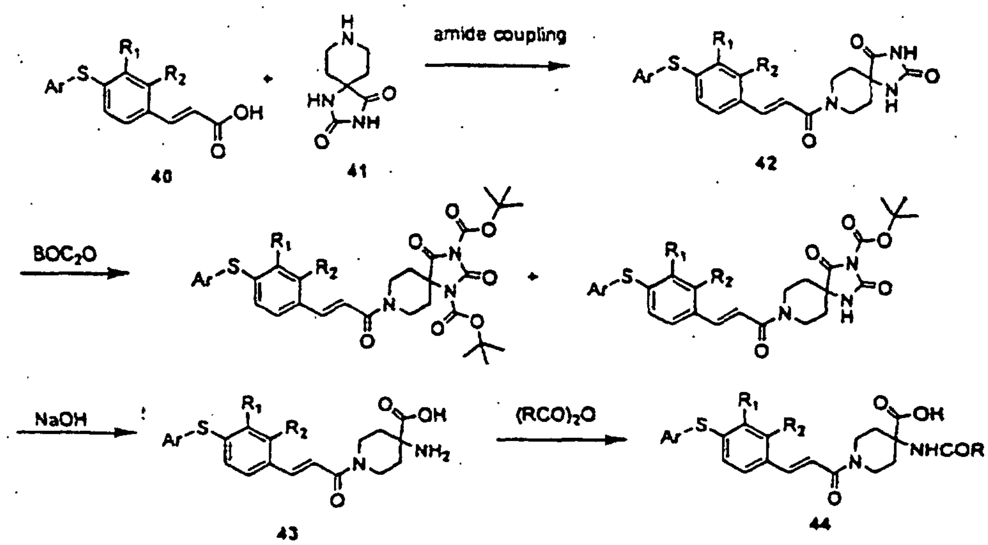


Further derivatives of piperidine amides can be obtained by coupling of piperidinone **45** with cinnamic acids **40,** as shown in Scheme 11. Standard coupling conditions lead to amide **46,** which is first reduced to the corresponding alcohol, then hydrolyzed to afford hydroxy acid **47**.

Scheme 11

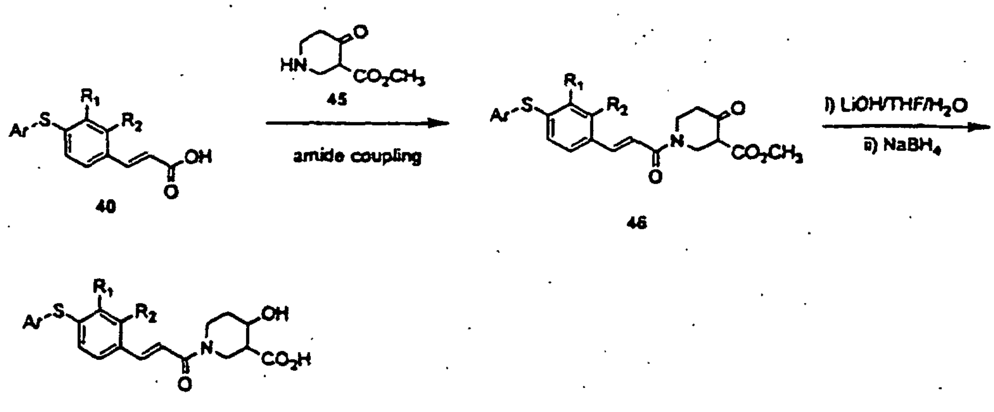


47

Also included in this invention are compounds derived from coupling of amines, or amino acid derivatives (such as a-amino esters) to the carboxylic acid group of cinnamides **48**, using standard coupling and hydrolysis methods, as outlined in Scheme 12. Thus, amides **49** are produced directly from amine coupling reactions. Amino acid esters are coupled to **48**, and the derived esters are hydrolyzed to the corresponding acids **50**.

Scheme 12

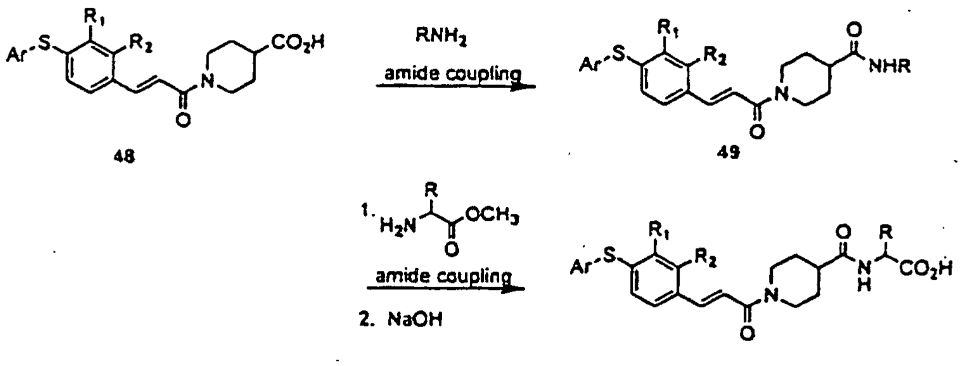


50

Inhibitors bearing substituted piperazine (or homopiperazine) cinnamides may be produced by the methods described in Scheme 13. The methods described may be utilized to produce piperazine amide **51**. Secondary amine **51** then serves as educt for preparing amides **52**, through standard coupling reactions. Alternatively, **51** may be converted to tertiary amines **53**, through standard reductive alkylation methods (for example, condensation with an aldehyde in the presence of a reducing agent such as sodium triacetoxyborohydride).

Scheme 13

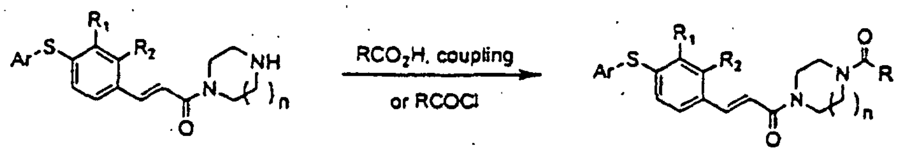


51 52

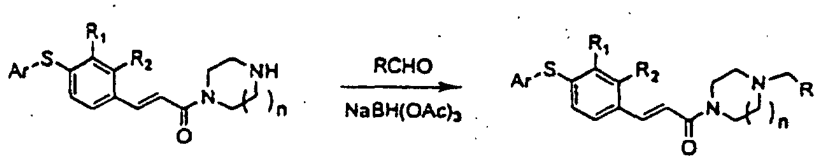


51 53

A process for preparing analogs with amino substitutions of the aryl portion of the sulfides is illustrated in Scheme 14. The intermediate triflate **27** is reacted with halo-substituted thiophenols **54** (X = Br, Cl, OTf, OTs) under basic catalysis, to provide the sulfide derivative **55**. The halogen or activated hydroxyl is then substituted with an amine, using the method of Buchwald (Old, D. W.; Wolfe, J. P.; Buchwald, S. L. *J. Am. Chem. Soc.* **1998**, *120,* 9722-9723). Similar transition-metal catalyzed reactions may be applied, for example, the method of Hartwig (Hamann, B. C.; Hartwig, J. F. *J. Am. Chem. Soc.* **1998**,*120,* 7369-7370). The $NR_3R_4$ group may constitute a cyclic or acyclic group, optionally substituted with additional functionalities that may enhance the activities of the compounds, and that further synthetic transformations familiar to those skilled in the art may be applied. For instance, ester groups may be hydrolyzed to the corresponding carboxylic acids or amides. The derived anilino sulfides may then be processed as described above to produce the cinnamides **56**.

Scheme 14

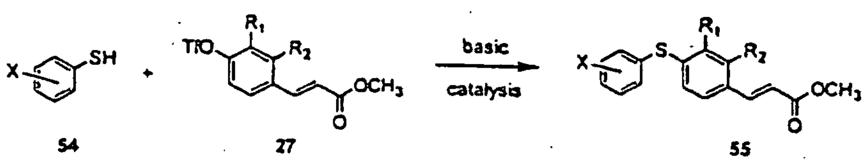


54 27 55

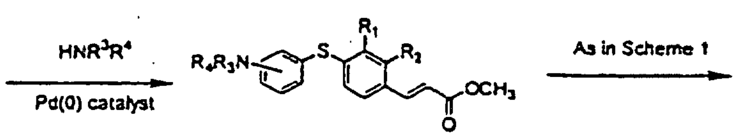


56

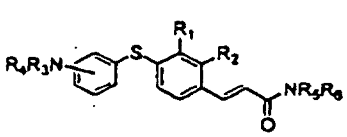

57

Scheme 15 presents a synthesis of a particular class of substituted aniline derivatives bearing a carboxylic acid. A cyclic amino acid **58** may be converted into the corresponding t-butyl ester **61**, through the intermediacy of carbamate **59** and ester **60**, using standard synthetic methods. The amino ester **61** was then reacted with 2-fluoronitrobenzene with mild basic catalysis (for example, cesium fluoride, potassium bicarbonate), to provide the aniline derivative **62**. The nitro group may then be transformed into an iodo-substituted derivative **64**, by first conversion to the aniline **63**, followed by standard diazotization and reaction of the diazonium salt with potassium iodide (among other similar methods for this Sandmeyer reaction). Using the method outlined in Scheme 7, the iodide **64** may be converted to the TIPS-protected arylthiol 65. In a sequence analogous with that described in Scheme 7, silyl thioether 65 may be reacted with cinnamide triflate **27** in the presence of a fluoride source (for example, cesium fluoride), and thus converted to the diarylsulfide **66**. Standard synthetic transformations (ester hydrolysis, amide coupling, and tert-butyl ester cleavage) provides the desired acid **68**, through intermediate ester **67**.

Compounds bearing elaborated ether groups on the arylsulfide ring were made according to Scheme 16. Methyl ether cinnamate esters such as **69** were hydrolyzed to the corresponding acids, and then the methyl ether was cleaved with boron tribromide (or alternatively using similar ether cleaving agents, such as trimethylsilyl iodide) to provide the hydroxy acids **70**. Standard coupling methods provided the amides **71**, which were then alkylated on the phenolic group using an appropriate alkyl halide **72** (where L is a linking group consisting of an acyclic or cyclic alkyl, or heterocyclic group) or lactone (m=1,2) in the presence of a base (such as potassium tert-butoxide, sodium hydride, or cesium carbonate). Alternatively, the phenolic group was alkylated with an ester-bearing alcohol **73**, using Mitsunobu conditions. The resulting ester-bearing ethers **74** were then hydrolyzed to the corresponding acids **75** using standard hydrolysis conditions. Alternatively, the ester of **74** may be *tert*-butyl, in which case acidic deprotection to acid **75** would be employed (for example, using trifluoroacetic acid in dichloromethane, or hydrochloric acid in dioxane).

Scheme 15

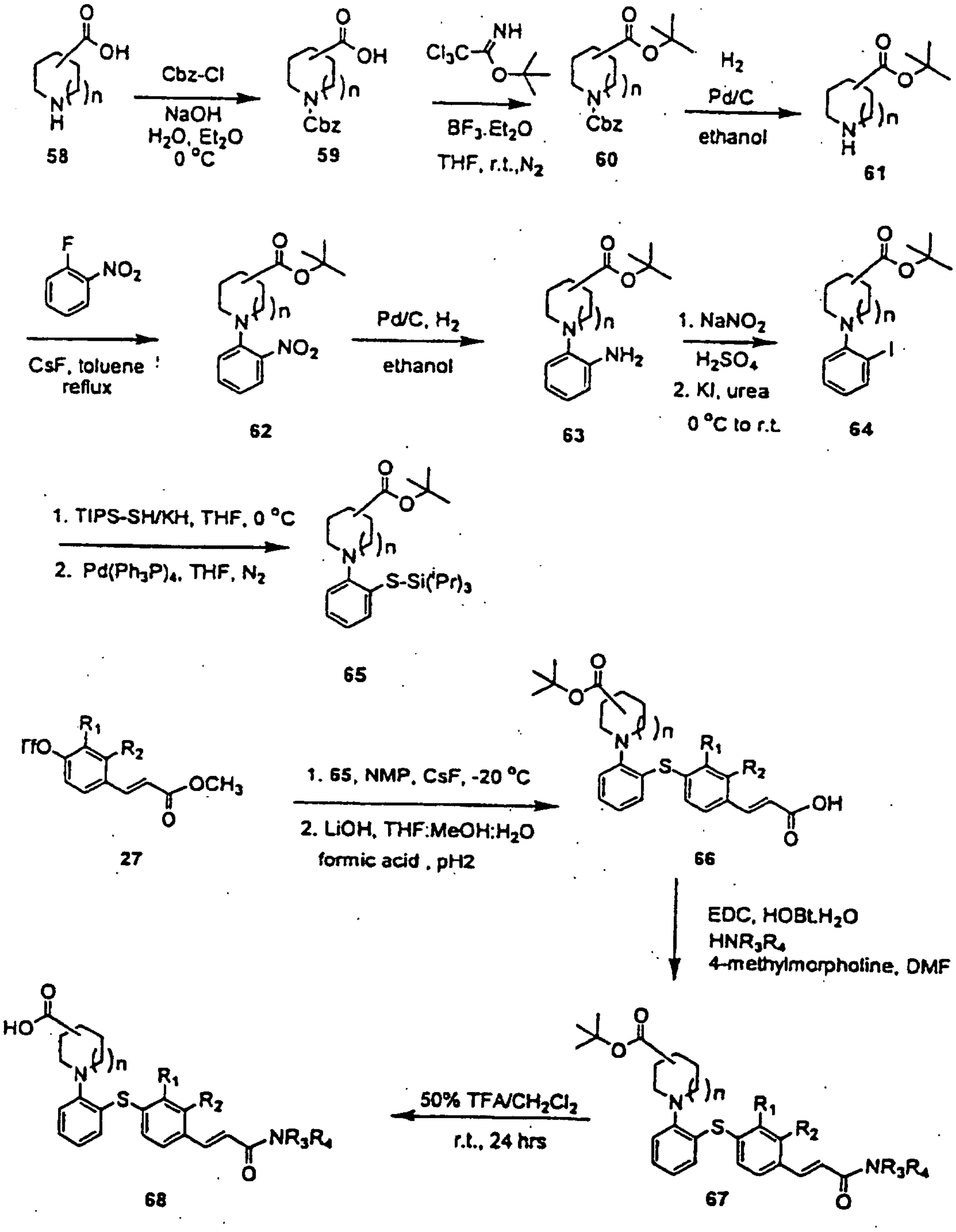
58
Cbz-Cl
NaOH
$H_2O$, $Et_2O$
0 °C
59
$BF_3.Et_2O$
THF, r.t.,$N_2$
60
$H_2$
Pd/C
ethanol
61
CsF, toluene
reflux
62
Pd/C, $H_2$
ethanol
63
1. $NaNO_2$
$H_2SO_4$
2. KI, urea
0 °C to r.t.
64
1. TIPS-SH/KH, THF, 0 °C
2. $Pd(Ph_3P)_4$, THF, $N_2$
65
27
1. 65, NMP, CsF, -20 °C
2. LiOH, THF:MeOH:$H_2O$
formic acid , pH2
66
EDC, HOBt.$H_2O$
$HNR_3R_4$
4-methylmorpholine, DMF
67
50% TFA/$CH_2Cl_2$
r.t., 24 hrs
68

Scheme 16

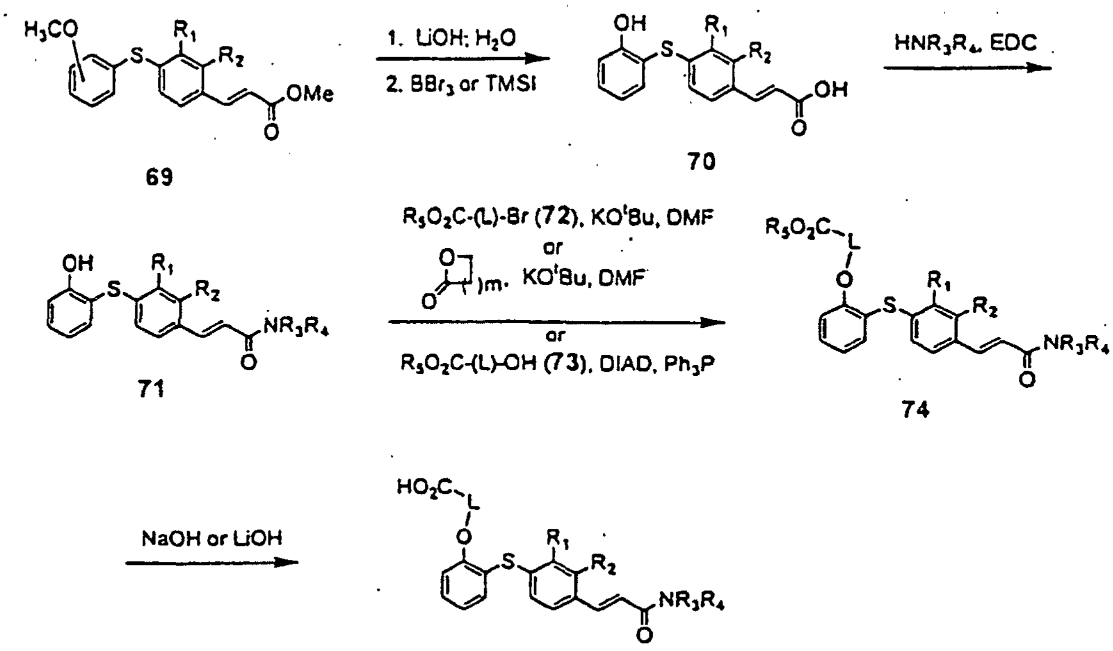


69

70

71

74

75

Related compounds bearing elaborated functionalized amino substituents were made according to Scheme 17. Triflate **27** was reacted with an amino thiophenol to produce the diarylsulfide cinnamide **76** in a similar manner to that described in Schemes 1, 2 and 7. The cinnamate ester was hydrolyzed to give acid **77,** which was coupled under standard conditions to provide amide **78.** The amino group of **78** then underwent reductive alkylation with an ester-bearing alkyl aldehyde, using standard conditions (or alternatively using sodium triacetoxyborohydride) to provide the secondary amine **79.** The ester functionality was hydrolyzed to the corresponding acid salt 80.

An alternative strategy for producing intermediate **78** is shown in Scheme **18.** Nitro-substituted *tert*-butyl ester derivative **81** (prepared according to Scheme 14, using the tert-butyl analog of cinnamate **27**) was cleaved to the carboxylic acid, converted to the cinnamide using standard conditions, and then the nitro group was reduced using iron powder in aqueous ammonium chloride solution.

Scheme 17

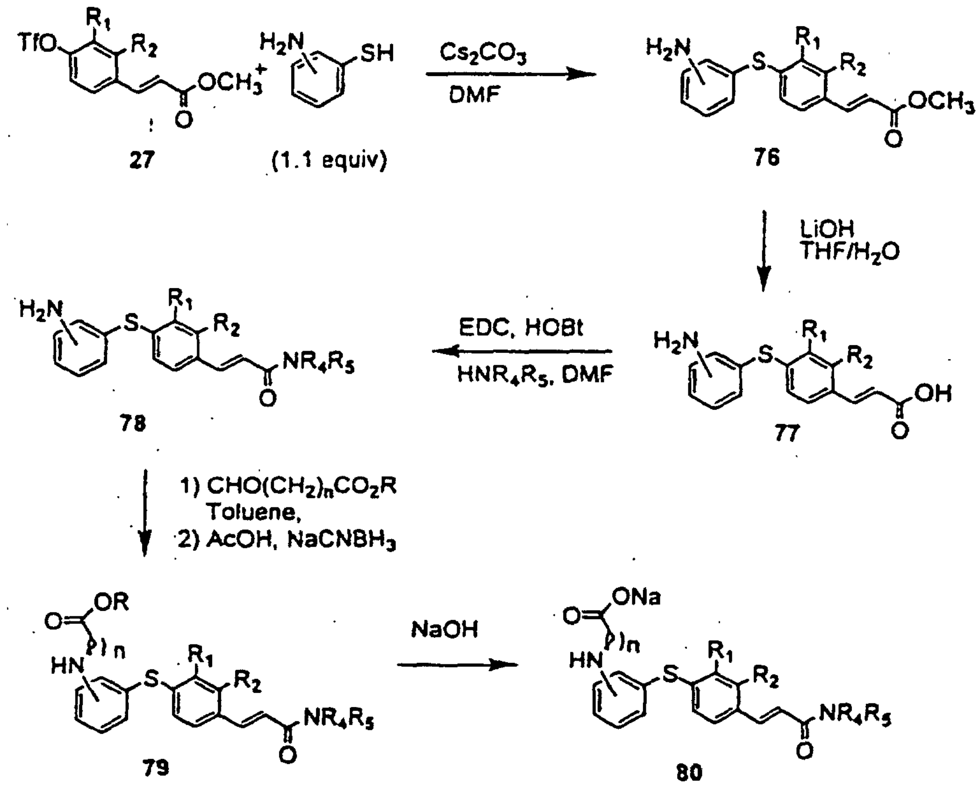


27, 76, 77, 78, 79, 80

Scheme 18

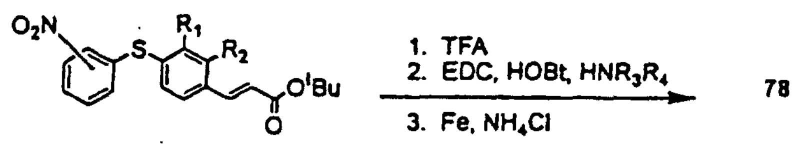


81

A modified method for the preparation of analogs bearing 2,3-bis-(trifluoromethyl)cinnamides is illustrated in Scheme 19. Commercially available acrylic acid **82** was esterified with ethyl iodide, and the ester **83** was condensed with 1,1,1,4,4,4-hexafluoro-2-butyne at 110 °C to give the bicyclic adduct **84**. The bicyclic ether was then converted to the corresponding phenol **85** using a Lewis acid (for example boron trifluoride-etherate). Phenol **85** was the utilized as illustrated in Scheme 7 or Scheme 14 to prepare the desired inhibitors.

Scheme 19

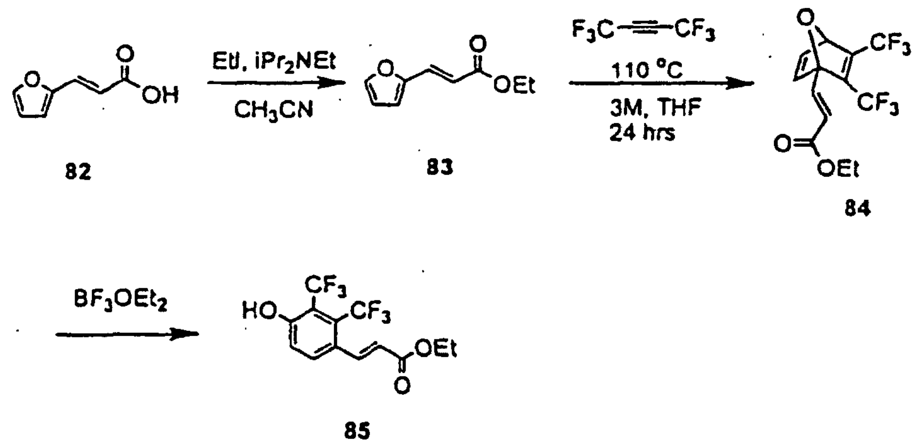


82 83 84 85

Scheme 20 illustrates an alternative method for preparing substituted anilinosulfides **57**. Cinnamate ester 55 was converted to the corresponding tert-butyl ester **87**, via reaction of acid **86** with tert-butyl trichloroacetimidate under Lewis acid catalysis. The bromide **87** was then coupled with an appropriately functionalized amine (illustrated in Scheme 20 with ethyl pyrrolidinecarboxylates) using palladium catalysis (for example, using the conditions of Buchwald or Hartwig noted for Scheme 14). The resultant substituted anilines **88** were then first cleaved to acids **89** using acidic conditions (TFA, HCl, or similar known deprotections for tert-butyl esters), then the acids **89** were coupled to amines $HNR_3R_4$ using standard conditions to provide amides **90**. The ethyl ester group of **90** was then hydrolyzed using lithium or sodium hydroxide in aqueous media to produce acids **91**.

Compounds with a 2,6-disubstitution pattern on the cinnamide ring system were made according to the method of Scheme 21. Commercially available 4,6-dichlorosalicylaldehyde was condensed with arylthiols under basic conditions to provide the diarylsulfide **92**. The phenolic group was protected with allyl bromide, providing the O-allyl derivative **93**. The method outlined in Scheme 1 was used to prepare the corresponding cinnamic acid **94**, then the allyl group was removed using panadium(0)-catalyzed transfer to morpholine, thus producing hydroxy cinnamic acid **95**. The acid group was coupled to a cyclic amino ester (n=0, 1, 2; R = Me, Et) under standard conditions to yield the amide **96**. Basic hydrolysis conditions reveal the acid 97.

Scheme 20

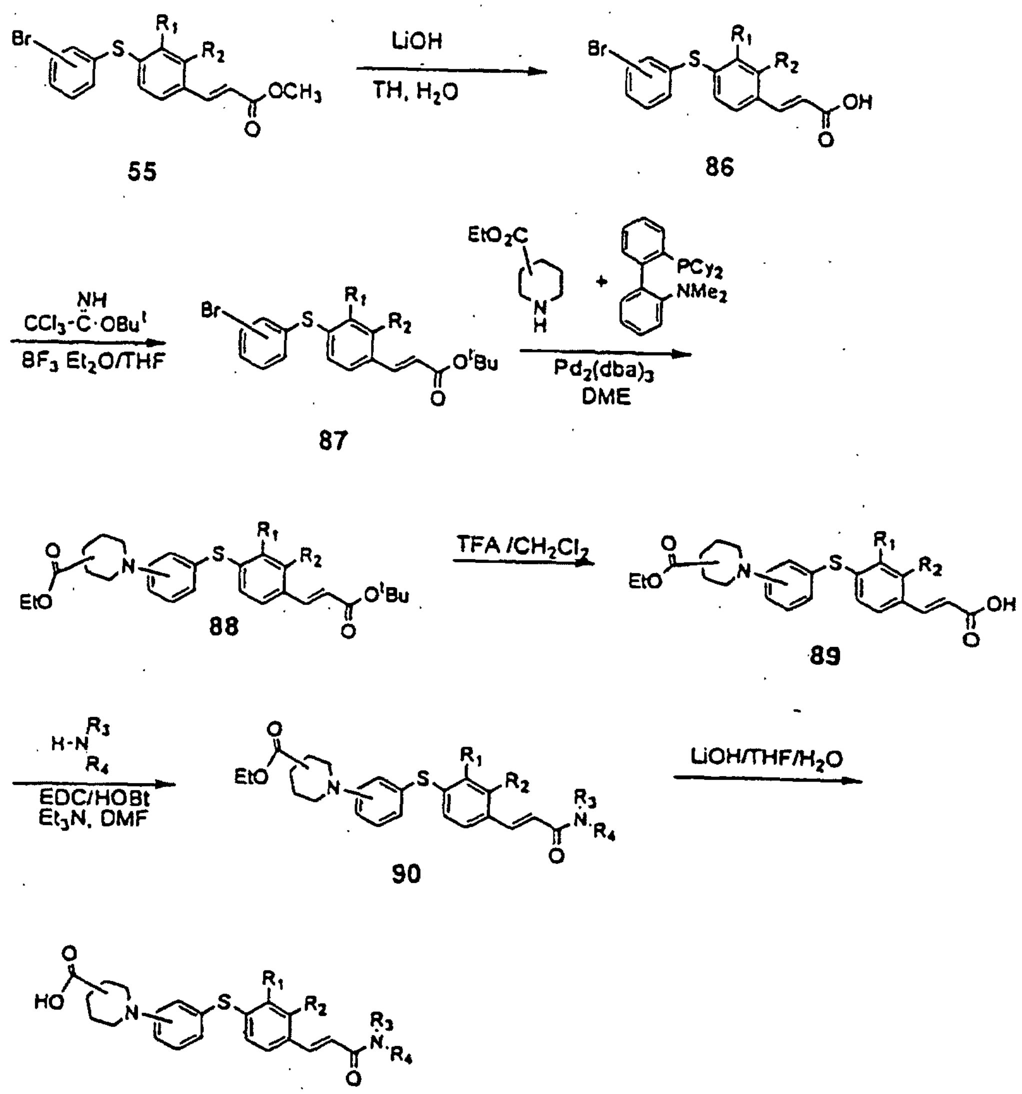


55 86 87 88 89 90 91

Scheme 21

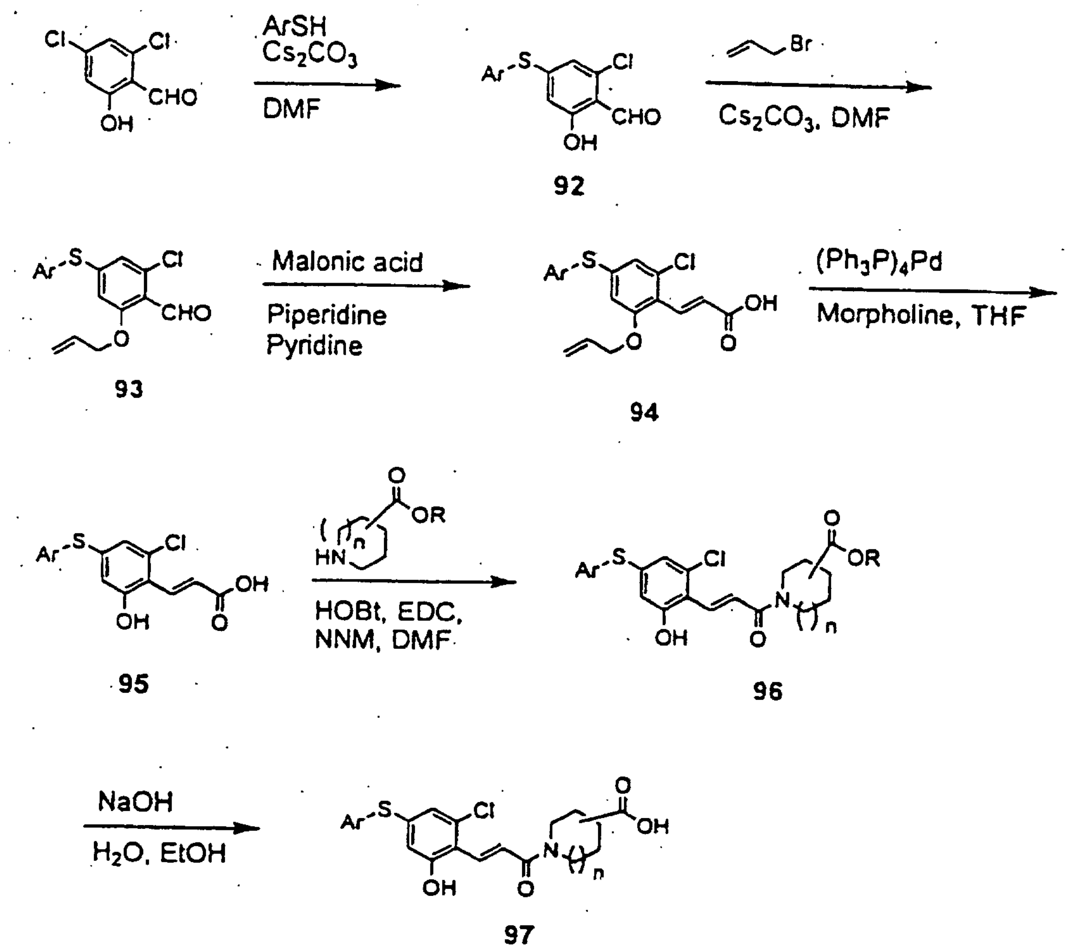


92 93 94 95 96 97

## EXAMPLES

The compounds and processes of the present invention may be better understood in connection with the following Examples, which are intended as an illustration of the invention.

Example 1 (Reference Example)

(2,4-Dichlorophenyl)[2-(*E*-((6-hvdroxyhexylamino)carbonyl)ethenyl)phenyl] sulfide

Example-1A

2-[(2,4-Dichlorophenyl)thiol]benzaldehyde

To a stirred solution of 2,4-dichlorothiophenol (2.0 g, 11.2 mmol) in 25 mL of anhydrous DMF was added potassium carbonate (3.09 g, 22.4 mmol), followed by 2-chlorobenzaldehyde (1.26 mL, 11.3 mmol). The mixture was then heated under nitrogen atmosphere at 70 °C for 5 hours. The reaction mixture was then allowed to cool to room temperature and partitioned between ether and water. The aqueous layer was extracted with ether once and the combined organic layer was washed with water and brine, dried over sodium sulfate and condensed in vacuo. The crude product was purified via silica gel flash chromatography, eluting with 5-10 % ether/hexanes, to give 2.62 g (9.25 mmol, 83%) of the desired aldehyde as a colorless oil, which solidified slowly upon standing at room temperature.

Example 1B

*trans*-2-[(2,4-Dichlorophenyl)thio]cinnamic acid

A mixture of the aldehyde (1.50 g, 5.3 mmol) from Example 1A, malonic acid (1.21 g, 11.6 mmol), piperidine (78.6 µL, 0.80 mmol) in 8.0 mL of anhydrous pyridine was heated at 110 °C for 2 hours. Gas evolution ceased during this period. Pyridine was then removed under vacuum. Water and 3N aq. HCl were then added with stirring. The desired cinnamic acid was then collected through filtration, washed with cold water and dried in a vacuum oven overnight to give 1.56 g (4.8 mmol, 91 %) of white solid.

Example 1C

(2,4-Dichlorophenyl)[2-(*E*-((6-hydroxyhexylamino)carbonyl)ethenyl)phenyl] sulfide

A suspension of the acid (284 mg, 0.87 mmol) from Example 1B in 5 mL of methylene chloride was stirred with $(COCl)_2$ (84 µL, 0.97 mmol), and one drop of DMF under nitrogen atmosphere for 90 minutes. The solvent was then removed under vacuum. The residue $(COCl)_2$ was removed with benzene (2x) in vacuo. To a separate flask, previously filled with 6-amino-1-hexanol (12 mg, 0.10 mmol), Hunig's base (22.8 µL, 0.13 mmol) and DMAP (1.1mg, 0.008 mmol) in 2.0 mL of $CH_2Cl_2$, the acid chloride (30 mg, 0.087 mmol) in 1.0 mL of $CH_2Cl_2$ was then dropped in slowly. After 30 minutes, the reaction mixture was poured into 3N HCl and extracted with ethyl aceetate (EtOAc). The organic layer was washed with brine, dried with $Na_2SO_4$, condensed under reduced pressure. The crude product was purified by preparative TLC to give 21.0 mg (90 %) of the title compound as a colorless oil. $^1$H NMR ($CDCl_3$, 300 MHz) δ 1.31-1.48 (m, 4H), 1.48-1.70 (m, 4H), 3.37 (q, *J* = 6.7 Hz, 2H), 3.65 (t, *J* = 6.3 Hz, 2H), 5.63 (br s, 1H), 6.36 (d, *J* = 15.9 Hz, 1 H), 6.7 1 (d, *J*= 9.3 Hz, 1H), 7.05 (dd, *J* = 2.4, 8.7 Hz, 1H), 7.31-7.49 (m, 4H), 7.65 (dd, *J* = 2.1, 7.5 Hz, 1H), 7.99 (d, *J* = 15.9 Hz, 1H). MS ($DCI/NH_1$) $(M+NH_4)^+$ at *m*/*z* 441, 443, 445.

Example 9 (Reference Example)

(2,4-Dichlorophenyl)(2-chloro-4-( *E*-((4-acetylpiperazin-1-yl)carbonyl) ethenyl)phenyl] sulfide

The title compound was prepared by the procedures described in Example 1 substituting 2-chlorobenzaldehyde with 3-chloro-4-fluoro-benzadehyde, and 6-amino-1-hexanol with 1-acetylpiperazine. White solid; $^1$H NMR ($CDCl_3$, 300 MHz) δ 2.15 (s, 3H), 3.50-3.58 (m, 2H), 3.58-3.85 (m, 6H), 6.85 (d, *J* = 15.3 Hz, 1H), 6.96 (d, *J* = 8.7 Hz, 1H), 7.24-7.36 (m, 3 H), 7.54 (d, *J* = 2.4 Hz, 1H), 7.61 (d, *J* = 15.3 Hz, 1 H), 7.61 (d, *J* = 2.1 Hz, 1 H). MS ($DCI/NH_3$) $(M+H)^+$ at *m*/*z* 486, 488, 490, 492. Analysis calculated for $C_{21}H_{19}N_2O_2Cl_3S_1 \cdot 0.85H_2O$: C, 51.99; H, 4.30; N, 5.77. Found: C, 52.03; H, 4.27; N, 5.67.

Example 32 (Reference Example)

(2,4-Dichlorophenyl)[2-nitro-4-( *E*-((4-acetylpiperazin-1-yl)carbonyl) ethenyl)phenyl] sulfide

Example 32A

1-Chloro-2-nitro-4-( E-((4-acetylpiperazin-1-yl)carbonyl)ethenyl) benzene

To a stirred solution of *trans-4-chloro-3-nitrocinnamic* acid (1.50 g, 6.59 mmol) and 1-acetylpiperazine (0.89 g, 6.94 mmol) in 20 mL of DMF at room temperature was added EDAC (1.4 g, 7.30 mmol). The mixture was then stirred at room temperature for 2 hours. TLC indicated the complete consumption of the acid. Water was then added to quench the reaction and to precipitate out the product. Cinnamide was then collected through filtration and washed with cold water. The light yellow product was dried in vacuum oven overnight at 40 °C to give 2.04 g (6.03 mmol, 91.6 %) of the title compound.

Example 32B

(2,4-Dichlorophenyl)[2-nitro-4-(*E*-((4-acetylpiperazin-1-yl)carbonyl) ethenyl)phenyl] sulfide

To a stirred solution of 4-chloro-3-nitro-cinnamide (275 mg, 0.814 mmol) from Example 32A in 1.0 mL of DMF was added potassium carbonate (169 mg, 1.22 mmol), followed by the dropwise addition of 2,4-dichlorothiophenol

(146 mg, 0.815 mmol). The mixture was then stirred at room temperature for 60 minutes. Completion of the reaction was indicated by the TLC. Water was then added to precipitate the product. Filtration, washing with cold water, and drying in a vacuum oven afforded 350 mg (0.728 mmol, 89%) of the titled compound as light yellow solid. $^1H$ NMR ($d^6$-DMSO, 300 MHz) δ 2.05 (s, 3H), 3.42-3.50 (br m, 4H), 3.50-3.64 (br m, 2H), 3.64-3.79 (br m, 2H), 6.83 (d, *J* = 8.7 Hz, 1H), 7.44 (d, *J*= 15.3 Hz, 1H), 7.55 (d, *J*= 15.3 Hz, 1H), 7.63 (dd, *J*= 2.7, 8.7 Hz, 1H), 7.83 (d, *J*= 8.7 Hz, 1H), 7.93 (d, *J* = 8.7 Hz, 1H), 7.96 (d, *J*= 2.7 Hz, 1H), 8.69 (d, *J*= 1.8 Hz, 1H). MS (DCI/$NH_3$) $(M+H)^+$ at *m*/*z* 497, 499, 501. Analysis calculated for $C_{21}H_{19}N_3O_4$ $Cl_2$ $S_1$-0.82$H_2O$: C, 50.94; H, 4.20; N, 8.49. Found: C, 50.91; H, 4.21; N, 8.69.

Example 71 (Reference Example)

(2-Isopropylphenyl)[2-nitro-4-( E-((3-carbomethoxypiperazin-1-yl)carbonyl) ethenyl)phenyl] sulfide

Example 71A

1- *tert*-Butyoxycarbonyl -2-carbomethoxypiperazine

2-Carbomethoxypiperazine was treated with benzyl chloroformate (1.0 eq) in aqueous $NaHCO_3$ to give 1-benzyloxycarbonyl-3-carbomethoxypiperazine. This material was treated with di-*tert*-butyldicarbonate (1.1 eq) and triethylamine (1.0 eq) in THF to produce 1-*tert*-butyoxycarbonyl-4-benzyloxycarbonyl-2-carbomethoxypiperazine. Hydrogenation of this compound in methanol using 10% Pd-C gives the title compound after filtration and solvent removal.

Example 71B

(2-Isopropylphenyl)[2-nitro-4-( *E*-((3-carbomethoxypiperazin-1-yl)carbonyl) ethenyl) phenyl] sulfide

A mixture of (2-isopropylphenyl)[2-nitro-4-*E*-(carboxyethenyl)phenyl] sulfide (prepared according to the procedures of Example 32), the amine from Example 71A (1.0 eq), 2-(1H-benzotriazol-1-yl)-1,1,3,3-tetramethyluronium tetrafluoroborate (1.0 eq), and diisopropylethylamine (2.0 eq) in DMF was stirred at ambient temperature for 4 hr. Ethyl acetate was added, and the mixture was washed sequentially with 1N HCl, bicarb, and brine. The resultant yellow solid was treated with 1:1 TFA/dichloromethane at ambient temperature to give the title compound as a yellow solid. $^1H$ NMR (DMSO-$d_6$, 300MHz) δ 1.15 (d, *J* = 6.6 Hz, 6H); 2.52-3.16 (br m, 4H); 3.25-3.47 (m, $^1H$); 3.60-3.65 (br d, 3H); 3.60, 3.66(br s, br s, 3H); 6.61-6.67 (br m, 1H); 7.30-7.62 (m, 6H); 7.88-7.93 (br m, 1H); 8.58-8.65 (br m, 1H), MS (APCI) $(M+H)^-$ at m/z 470. Anal calcd for $C_{24}H_{27}N_3S_1O_5$: C, 61.39; H, 5.80; N, 8.95. Found: C, 61.51; H, 5.87; N, 8.68.

Example 84 (Reference Example)

(2-Bromophenyl)[2-trifluoromethyl-4-(*E*-((4-acetylpiperazin-1-yl)carbonyl) ethenyl) phenyl] sulfide

The tide compound was prepared by the procedures described in Example 9 substituting 2,4-dichlorothiophenol with 2-bromothiophenol, and 3,4-dichlorobenzaldehyde with 4-fluoro-3-trifluoromethylbenzaldehyde, to give a white solid. $^1H$ NMR ($d^6$-DMSO, 300 MHz) δ 2.04 (s, 3H), 3.43-3.80 (m, 8H), 7.21 (dd, *J*= 2.1, 8.4 Hz, 1H), 7.24 (d, *J*= 8.4 Hz, 1H), 7.33 (td, *J*= 2.1, 7.65 Hz, 1H), 7.42 (td, *J*= 1.8, 7.65 Hz, 1H), 7.45 (d, *J* = 15.6 Hz, 1H), 7.58 (d, *J* = 15.6 Hz, 1H), 7.78 (dd, *J* = 1.8, 8.4 Hz, 1H), 7.96 (dd, *J*= 1.8, 8.4 Hz, 1H), 8.25 (d, *J*= 1.8 Hz, 1H). MS ($APCI^+$) $(M+NH_4)^+$ at *m*/*z* 530, 532, 534.

Example 92 (Reference Example)

(3-Carboxamidophenyl)[2-nitro-4-(*E*-((4-acetylpiperazin-1-yl)carbonyl) ethenyl) phenyl]sulfide

Example 92A

(3-Carboxyphenyl)[2-nitro-4-( *E*-((4-acetylpiperazin-1-yl)carbonyl) ethenyl) phenyl] sulfide

The title compound was prepared by the procedures described in Example 32B substituting 2,4-dichlorothiophenol with 3-mercaptobenzoic acid.

Example 92B

(3-Carboxamidophenyl)[2-nitro-4-( *E*-((4-acetylpiperazin-1-yl)carbonyl) ethenyl) phenyl] sulfide

To a stirred solution of benzoic acid from Example 92A (40 mg, 0.088 mmol) in I mL of anhydrous DMF with HOBT (15 mg, 0.097 mmol) was added EDAC (19 mg, 0.097 mmol), followed by ammonium chloride (large excess). The pH of the solution was adjusted to 6 with addition of triethylamine. The resulting mixture was then stirred at ambient temperature for 6 h. Water was added to quenched the reaction. The product precipitated out after stirring for 30 min, which was then isolated by filtration and dried in vacuum oven to give a light yellow solid (25 mg, 63% yield). $^1$H NMR ($d^6$-DMSO, 300 MHz) δ 2.04 (s, 3H), 3.43-3.82 (m, 8H), 6.84 (d, *J*= 8.7 Hz, 1H), 7.43 (d, *J*= 15.6 Hz, 1H), 7.53 (d, *J*= 15.6 Hz, 1H), 7.56 (d, *J*= 1.8 Hz, 1H), 7.66 (t, *J* = 7.65 Hz, 1H), 8.06 (d, *J* = 7.80 Hz, 1H), 8.12 (s, 2H), 8.67 (d, *J* = 2.1 Hz, 1H). MS (ESI$^+$) $(M+Na)^+$ at *m*/*z* 477.

Example 97 (Reference Example)

(2-Ethoxyphenyl)-[2-chloro-4(E-(morpholin-1-yl)carbonyl]ethenyl)phenyl]sulfide

Example 97A

2-Ethoxybenzenethiol

To 7.82g of ethoxybenzene and 7.41g of tetramethylethylenediamine in 75 ml ether, cooled in an ice bath, a solution of 25.6 ml of a 2.5 M n-butyllithium solution in hexane, was added dropwise under a nitrogen atmosphere. The mixture was stirred for 1 hour at room temperature and then cooled to -65 degrees. Sulfur (2.28 g) was added in portions. The mixture was stirred for 3 hours at room temperature and then cooled in ice. $LiAlH_4$ (0.6,g) was added and the mixture was stirred I hour at room temperature. The mixture was again cooled in ice while 5 ml water was added dropwise followed by 15% HCI in water until all salts. The aqueous phase was separated and washed with ether. The combined ether layers was washed with HCI, then water. After drying with $Na_2SO_4$, the ether was evaporated to give 9.66 g of product. NMR analysis showed 70% pure material with 30% of a diaryl sulfide impurity. This mixture was carried forward to the next step.

Example 97B

(2 -Ethoxyphenyl)-[2-chloro-4(E-[(morpholin-1-yl)carbonyl]ethenyl)phenyl]sulfide

The title compound was prepared according to the procedures of Example 1, substituting the thiol of Example 97A, giving a white solid, m.p. 125-127C. $^1$H NMR ($CDCl_3$, 300 MHz) δ 1.25 (t, J=7Hz, 3H), 3.60-3.78 (m, 8H), 4.05 (q, 1=7Hz, 2H), 6.76 (d, J=15Hz, 1H), 6.82 (d, J=9H, 1H), 6.94-7.00 (m, 2H), 7.16 (dd, J=9Hz, 2Hz, 1H), 7.34-7.45 (m, 2H), 7.54 (d, J=2Hz, 1H), 7.58 (d, J=15Hz, 1H). Anal. Calcd. for $C_{21}H_{22}ClNO_3S$: C, 62.44; H, 5.49; N, 3.47. Found: C, 62.14; H, 5.70; N, 3.22.

Example 137 (Reference Example)

(2-Ethoxyphenyl)-[2-chloro-4(E-[(3-ethoxycarbonylpiperidin-1-yl)carbonyl]ethenyl] phenyl]sulfide

The title compound was prepared according to the procedures of Example 97. $^1$H NMR ($CDCl_3$, 300 MHz) δ 1.25 (t, J= 7 Hz, 6H), broad peaks totaling 9 protons at 1.50-1.62, 1.65-1.92, 2.01-2.15,2.45-2.55,2.95-3.05, 3.13-3.30,3.55-3.68, 3.90-4.10, 4.05 (q, J=7Hz, 2H), 4.15 (q, J=7Hz, 2H), 6.84 (d, J=9Hz, 1H), 6.80-6.95 (broad, 1H), 6.94-6.99 (m, 2H), 7.18 (dd, J=9Hz, 2Hz, 1H), 7.34-7.41 (m, 2H), 7.52 (d, J=15Hz, 1H), 7.55 (d, J=2Hz, 1H). Anal. Calcd. for $C_{25}H_{28}ClNO_4S$: C, 63.35; H, 5.95; N, 2.95. Found: C, 63.17; H, 6.02; N, 26.02; N, 2.81.

Example 155 (Reference Example)

(2-Ethoxyphenyl)-[2-chloro-4(E-[(3-carboxypiperidin-1-yl)carbonyl]ethenyl)phenyl] sulfide

The compound of Example 137 was hydrolyzed using an excess of aqueous 10% NaOH in methanol, stirring overnight. The reaction mixture was concentrated *in vacuo,* water was added, and the solution was extracted with ether, giving a white solid, m.p. 166-171. $^1$H NMR (DMSO, 300 MHz) δ 1.17 (t, J=7Hz, 3H), broad peaks totaling 9

protons at 1.32-1.48, 1.51-1.78, 1.90-2.04,'2.25-2.50, 2.90-2.90, 2.95-3.17, 3.45-3.51, 3.95.4.19, 4.4-4.51, 4.06 (q, J=7Hz, 1H), 6.80 (d, J=9Hz,1H), 7.01 (t, J=7Hz, 1H), 7.15 (d, J=8Hz, 1H), 7.26-7.40 (m, 2H), 7.40-7.48 (m, 1H), 7.51 (dd, J=9Hz, 2Hz, 1H), 7.99 (d, J=9Hz, 1H). Anal. Calcd. for $C_{23}H_{24}ClNO_4S$: C, 61.94; H, 5.42; N, 3.14. Found: C, 61.75; H, 5.65; N, 3.15. The resultant acid (303 mg, 0.631 mmol) was dissolved in 3 ml MeOH. A KOH solution (38 mg, 0.595 mmol, of 87.6% KOH ) in 1ml MeOH was added. The resulting solution was concentrated in vacuo, and 5 ml. ether was added. The mixture was stirred for one hour to form a powder, which was filtered and dried in the vacuum oven at 60C to yield 307 mg of a solid, water soluble product.

Example 164 (Reference Example)

(2-Ethoxyphenyl)-[2-chloro-4(*E*-[(3-ethoxycarbonylpiperidin-1-yl)carbonyl]ethenyl) phenyl]sulfide

The title compound was prepared according to the procedures of Example 97. $^1$H-NMR ($CDCl_3$) δ 1.25 (t, J=7Hz, 6H), broad peaks totaling 9 protons at 1.65-1.80, 1.95-2.04, 2.51-2.63, 2.90-3.00,3.15-3.30, 2.95-4.05, 4.42-4.55, 4.14 (q, J=7Hz, 2H), 4.15 (q, J=7Hz, 2H), 6.82 (d, J=15 Hz, 1H), 6.84 (d, J=9Hz, 1H), 6.93-6.99 (m, 2H), 7.17 (dd, J=9Hz, 2Hz, 1H), 7.34-7.41 (m, 2H), 7.52 (d, J=15Hz, 1H), 7.55 (d, J=2Hz, 1H). Anal. Calcd. for $C_{25}H_{28}ClNO_4S$:C, 63.35; H, 5.95; N, 2.95. Found: C, 63.09; H, 6.24; N, 2.77.

Example 165

(2-Ethoxyphenyl)-[2-chloro-4(*E*-[(4-carboxypiperidin-1-yl)carbonyl]ethenyl)phenyl] sulfide

The compound of Example 164 was hydrolyzed, and the salt formed, according to the procedures of Example 155. m.p. I6S-166C. $^1$H-NMR (DMSO 300 MHz) δ 1.25 (t, J=7Hz, 3H0, 1.35-1.58 (m, 2H), 1.80-1.95 (m, 2H), 2.50-2.60 (m, 1H), 1.78-1.91 (m, 1H), 3.13-3.24 (m, 1H), 4.05 (q, 7=7Hz, 2H), 4.12-4.35 (m, 2H), 6.80 (d, J=9Hz, 1H), 6.96-7.05 (t, J=8 Hz, 1H), 7.15 (d, J=9Hz, 1H), 7.28-7.48.(m, 4H), 7.51 (dd, J= 9Hz, 2Hz, 1H), 8.00 (d, J=2Hz).

Example 233 (Reference Example)

(Benzodioxan-6-yl)[2-trifluoromethyl-4-(*E*-((4-acetylpiperazin-1-yl)carbonyl)ethenyl) phenyl] sulfide

The title compound was prepared by the procedures described in Example 84, substituting 2-bromothiophenol with 6-mercaptobenzenedioxane. white solid; $^1$H NMR ($CDCl_3$, 300 MHz) δ 2.15 (s, 3H), 3.46-3.89 (m, 8H), 4.30 (dd, *J*= 2.1, 6.0 Hz, 4H), 6.84 (d, *J* = 15.0 Hz, I H), 6.92 (d, *J* = 8.4 Hz, 1H), 6.97-7. 10 (m, 3 H), 7.42 (d, *J* = 8.4 Hz, 1H), 7.64 (d,*J*= 15.0 Hz, 1H), 7.77 (s, 1H). MS ($ESI^+$) *m/z* 493 $(M+H)^+$.

Example 240 (Reference Example)

(Benzodioxan-6-yl)[2-trifluoromethyl-4-(E((3-(pyrrolidin-2-on-1-yl)prop-1-ylamino)carbonyl) ethenyl)phenyl]sulfide

The title compound was prepared by the procedures described in Example 233, substituting I-acetylpiperazine with 3-aminopropyl-1-pyrrolidin-2-one, giving a white solid. $^1$H NMR ($CDCl_3$, 300 MHz) δ 1.69-1.80 (m, 2H), 2.08 (p, *J* = 7.5 Hz, 2H), 2.44 (t, *J*= 7.5 Hz, 2H), 3.27-3.48 (m, 6H), 4.24-4.34 (m, 4H), 6.44 (d, *J*= 15.6 Hz, 1H), 6.90 (d, *J* = 8.4 Hz, 1H), 7.00 (d, *J* = 8.4 Hz, 1H), 7.01 (dd, *J* = 2.7, 8.4 Hz, 1H), 7.06 (d, *J* = 2.7 Hz, 1H), 7.08 (s, 1H), 7.40 (dd, *J* = 2.1, 8.4 Hz, 1H), 7.53 (d, *J*= 15.6 Hz, 1H), 7.75 (d, *J* = 2.1 Hz, 1H). MS ($ESI^+$) $(M+H)^+$ at *m/z* 507.

Example 281 (Reference Example)

(1-Methylindol-5-yl)[2-chloro-4-(*E*-((3-(1-pyrrolidin-2-onyl)propylamino) carbonyl)ethenyl) phenyl] sulfide

Example 281A

Triisopropylsilyl(1-methylindol-5-yl) sulfide

To a stirred solution of 5-bromo-*N*-methyl indole (300 mg, 1.43 mmol) in 5 mL of benzene in a sealed tube was charged with $Pd(PPh_3)_4$ (82 mg, 0.072 mmol), followed by KSTIPS (326 mg, 1.43 mmol). The mixture was flushed with $N_2$, the tube was capped, and the reaction mixture refluxed for 2 h. The reaction mixture was then allowed to cool down, partitioned between $Et_2O$ and water. The organic layer was washed with brine, dried over $Na_2SO_4$, concentrated

in vacuo. The residue was purified on a $SiO_2$ flash column chromatography eluting with 5% EtOAc/hexanes to give 400 mg (88 %) of the title compound as colorless oil.

Example 281 B

3-Chloro-4-((1-methylindol-5-yl)thio) benzaldehyde

To a stirred solution of thiolsilyl ether (1.0 g, 3.13 mmol) in 5 mL of DMF with 3-chloro-4-flurobenzaldehyde (500 mg, 3.13 mmol) at ambient temperature was added CsF (5.7 mg, 0.38 mmol). The mixture was stirred over night before it was poured in water and extracted with $Et_2O$ (2x25 mL). The combined organic layer was washed with water and brine, dried over $Na_2SO_4$, concentrated in vacuo. The residue was purified on a $SiO_2$ flash column chromatography eluting with 5-10 % EtOAc/hexanes to give 650 mg (71 %) of the title compound as white solid.

Example 281C

(1-Methylindol-5-yl)[(2-chloro-4-(*E*-((3-(1-pyrrolidin-2-onyl)propylamino) carbonyl)ethenyl)phenyl] sulfide

The title compound was prepared by the procedures described in Example 92, substituting the benzoic acid with cinnamic acid prepared from the above-described aldehyde, and ammonium with 3-aminopropyl-1-pyrrolidin-2-one, to give a white solid. $^1$H NMR ($CDCl_3$, 300 MHz) δ 1.74 (br m, 2H), 2.07 (br m, 2H), 2.44 (br m, 2H), 3.32 (br m, 2H), 3.40 (br m, 4H), 3.85 (s, 3H), 6.36 (d, *J*= 15.3 Hz, 1H), 7.14 (d, *J* = 3.0 Hz, 1H), 7.36 (dd, *J* = 1.5, 9.0 Hz, 1 H), 7.41 (d, *J* = 9.0 Hz, 1H), 7.50 (s, 1H), 7.89 (d, *J*= 1.5 Hz, I H). MS (ESI$^+$) (M+H)$^+$ at *m*/*z* 468, 470. Anal. Calcd for $C_{25}H_{26}ClN_3O_2S$· 1.37 $H_2O$: C, 60.95; H, 5.88; N, 8.53. Found: C, 60.97; H, 5.98; N, 8.46.

Example 285 (Reference Example)

(1-Methylindol-5-yl)[2-chloro-4-( *E*-((4-carboethoxypiperidin-1-yl)carbonyl)ethenyl) phenyl] sulfide

The title compound was prepared by the procedures described in Example 281C, substituting aminopropyl pyrrolidinone with ethyl isonipecotate, giving a white solid. $^1$H NMR ($CDCl_3$, 300 MHz) d 1.26 *(t, J*= 7.5 Hz, 3H), 1.64-1.83 (m, 2H), 1.88-2.08 (m, 2H), 2.48-2.67 (m, 1 H), 2.86-3.40 (m, 2H), 3.85 (s, 3H), 3.89-4.24 (m, 1H), 4.15 (q, *J*= 7.5 Hz, 2H), 4.24-4.65 (m, 1H), 6.53 (d, *J*= 3.0 Hz, 1H), 6.58 (d, *J*= 8.1 Hz, 1H), 6.81 (d, *J*= 15.3 Hz, 1H), 7.07 (d, *J* = 8.1 Hz, 1H), 7.14 (d, *J*= 3.0Hz, 1H), 7.37 (dd, *J*= 1.5, 9.0 Hz, 1H), 7.50 (d, *J* = 9.0 Hz, 1H), 7.50 (d, *J*= 15.3 Hz, 1H), 7.88 (d, *J*= 1.5 Hz, 1 H). MS (ESI$^+$) (M+H)$^+$ at *m*/*z* 483, 485.

Example 310 (Reference Example)

(2-Methoxyphenyl)-[2,3-dichloro-4( *E*- [morpholin-1-yl)carbonyl]ethenyl)phenyl] sulfide

Example 310A

2,3-Dichloro-4-trifluoromethanesulfonyloxy-benzaldehyde

2,3-Dichloro-4-hydroxy-benzaldehyde (9.10 g , J. Med. Chem. 19 (4), 534, 1994) was dissolved in 45 ml pyridine at room temperature. The solution was placed in an ice bath and immediately, 15.63 g of trifluoromethanesulfonic anhydride was added slowly. [Note: If the pyridine solution is cooled to zero before addition of triflic anhydride the aldehyde crystallizes out and the mixture cannot be stirred.] After the addition is complete the dark mixture was stirred for 1 hour at room temperature. It was then poured into a stirred mixture of ice water, 100 ml of concentrated HCl and ether. (Note: Not everything is soluble in this mixture] The ether layer was separated, dried over sodium sulfate, and the solvent removed. Warm heptane was added to this residue, and any insoluble material was filtered. The solution was concentrated to give 8.74 g (57% yield) of product as an orange oil which solidified in the refrigerator.

Example 310B

2,3-Dichloro-4-(2-methoxyphenylthio)-benzaldehyde

2,3-Dichloro-4-trifluoromethanesufonyloxy-benzaldehyde (2.50 g) was dissolved in 6 ml acetonitile. 2-Methoxybenzenethiol (2.55 g of 70% pure material, 50% excess) was added. With cooling 2.50 g diisopropylethylamine

was added slowly. The solution was removed from the ice bath, whereon a solid formed. The solution was warmed in a 50C waterbath for 5 minutes. More acetonitrile (5 ml) was added and the mixture was cooled in ice, and then filtered to get 2.047 g of product, m.p. 137-139C.

Example 310C

2,3-Dichloro-4-(2-methoxyphenythio)cinnamic acid

A mixture of 2,3-dichloro-4-(2-methoxyphenylthio)-benzaldehyde (2.03g), 1.44 g malonic acid, 5 ml pyridine, and 0.100 g piperidine was heated to 115° C for 1.5 hours. The mixture was cooled, and ice and HCl were added. The resulting solid was filtered, washed with water and dissolved in tetrahydrofuran. This solution was dried over sodium sulfate, the solvent removed and ether added to give 1.733 g of product, m.p. 187.188 °C.

Example 310D

(2-Methoxyphenyl)-[2,3-dichloro-4(*E*-(morpholin-1-yl)carbonyl])ethenyl)phenyl] sulfide

The title compound was prepared according to the procedure of Example 1, substituting the cinnamic acid of Example 310C, giving a white solid, m.p. 161-162°C, $^1$H-NMR ($CDCl_3$ 300 MHz) δ 3.83 (s, 3H), 6.55 (d, J=9Hz, 1H), 6.70 (broad d, J=15 Hz, 1H), 6.99-7.05 (m, 2H), 7.26 (d, J=9 Hz, 1H), 7.43-7.50 (m, 2H), 8.07 (broad d, J=15 Hz, 1H) Anal. Calcd. for $C_{20}H_{19}Cl_2NO_3S$: C, 56.61; H, 4.51; N, 3.30. Found: C, 56.75; H, 4.57; N, 2.61.

Example 319 (Reference Example)

(2-Methoxyphenyl)-[2,3-dichloro-4(*E*-[(4-carboxypiperidin-1-yl)carbonyl]ethenyl) phenyl]sulfide

The title compound was prepared according to the procedures of Example 310. $^1$H-NMR ($CDCl_3$ 300 MHz) δ 1.66-1.83 (m, 2H), 1.95-2.09 (m, 2H), 2.57-2:69 (m, 1H), 2.94-3.08 (m, 1), 3.15-3.31 (m, 1H), 3.72 (s, 3H), 3.90-4.05 (m, 1H), 4.41-4.55 (m, 1H), 6.55 (d, J=9Hz, 1H), 6.73 (d, J=15Hz, 1H), 7.00-7.05 (m, 2H), 7.27 (d, J=8Hz, 1H), 7.44-7.50 (m, 2H), 7.92 (d, J=15Hz, 1H). Anal. Calcd. for $C_{22}H_{21}Cl_2NO_4S$: C, 56.66; H, 4.54; N, 3.00 . Found: C, 56,89; H, 4.84; N, 2.64.

Example 325 (Reference Example)

(Benzodioxan-6-yl)[2,3-dichloro-4-(E-((3-(pyrrolidin-2-on-1-yl)prop-1-ylamino)carbonyl) ethenyl)phenyl]sulfide

The title compound was prepared by the procedures described in Example 240, substituting 4-fluoro-3-trifluoromethylbenzaldehyde with 2,3-dichloro-4-trifluoromethanesulfoxybenzaldehyde, giving a white solid. $^1$H NMR ($CDCl_3$, 300 MHz) δ 1.71-1.82 (m, 2H), 2.08 (p, *J*= 7.5 Hz, 2H), 2.46 (t, *J*= 7.5 Hz, 2H), 3.2603.50 (m, 6H), 4.23-4.36 (m, 4H), 6.36 (t, *J*= 15.6 Hz, 1H), 6.60 (d, *J* = 8.7 Hz, 1H), 6.44 (d, *J* = 8.7 Hz, 1H), 7.03 (dd, *J* = 2.4, 8.7 Hz, 1H), 7.09 (d, *J* = 2.4 Hz, 1H), 7.31 (d, *J* = 8.7 Hz, 1H), 7.94 (d, *J* = 15.6 Hz, 1H). MS ($ESI^+$) $(M+H)^+$ at *m/z* 507, 509, 511. Anal. Calcd for $C_{24}H_{24}Cl_2N_2O_4S \cdot 1.87\ H_2O$: C, 53.27; H, 5.17; N, 5.18. Found: C, 53.30; H, 5.17; N, 4.83.

Example 328 (Reference Example

(Benzodioxan-6-yl)[2,3-dichloro-4-(*E*-((4-carboethoxypiperidin-1-yl) carbonyl)ethenyl) phenyl] sulfide

The title compound was prepared by the procedures described in Example 325, substituting aminopropyl pyrrolidinone with ethyl isonipecotate, giving a white solid. $^1$H NMR ($CDCl_3$, 300 MHz) δ 1.26 (t, *J* = 7.2 Hz, 3H), 1.69 (td, *J* = 3.9. 10.8 Hz, 1H), 1.74 (td, *J*= 3.9, 10.8 Hz, 1H), 1.82-2.05 (m, 2H), 2.50-2.63 (m, 1H), 2.84-3.31 (m, 2H), 3.81-4.06 (m, 1H), 4.15 (q, *J* = 7.2 Hz, 2H), 4.24-4.34 (m, 4H), 4.34-4.59 (m, 1H), 6.61 (d, *J* = 8.7 Hz, 1H), 6.74 (d, *J* - 15.6 Hz, 1H), 6.94 (d, *J* = 8.7 Hz, 1H), 7.03 (dd, *J*= 2.7, 8.7 Hz, 1H), 7.08 (d, *J* = 2.7 Hz, 1H), 7.29 (d, *J*= 8.7 Hz, 1H), 7.90 (d, *J* = 15.6 Hz, 1H), MS ($ESI^+$) $(M+H)^+$ at *m/z 522,* 524, 526. Anal. Calcd for $C_{25}H_{25}Cl_2NO_5S$: C, 57.48; H, 4.82; N, 2.68. Found: C, 57.82; H, 4.96; N, 2.28.

Example 330 (Reference Example)

(Benzodioxan-6-yl)[2,3-dichloro-4-(*E*-((4-carboxypiperidin-1-yl)carbonyl)ethenyl phenyl] sulfide

The title compound was prepared by the procedures described in Example 155, substituting the ethyl ester from Example 137 with the ethyl ester from Example 328, and KOH with NaOH, to produce a white solid. $^1$H NMR (d$^6$-DMSO, 300 MHz) δ 1.33-1.55 (m, 2H), 1.62-1.78 (m, 2H), 1.93-2.07 (m, 1H), 2.90 (brt, *J* = 10.5 Hz, 1 H), 3.16 (brt, *J*= 10.5 Hz, 1H), 3.96 (br d, *J*= 13.5 Hz, 1H), 4.09 (br d, *J*= 13.5 Hz, 1 H), 4.26-4.42 (m, 4H), 6.60 (d, *J*= 9.0 Hz, 1H), 7.04-7.08 (m, 2H), 7.13 (d, *J*= 1.5 Hz, 1 H), 7.22 (d, *J* = 15.3 Hz, 1H), 7.70 (d, *J* = 15.3 Hz, 1H), 7.86 (d, *J* = 9.0 Hz, 1H). MS (ESI$^+$) (M+H)$^+$ at *m/z* 516, 518, 520. Anal. Calcd for $C_{23}H_{20}Cl_2N_1NaO_5S \cdot 0.36\ Et_2O$: C, 54.06; H, 4.38; N, 2.58. Found: C, 53.99; H, 4.37; N, 2.22.

Example 339 (Reference Example)

(1-Methylindol-5-yl)[2,3-dichloro-4-(*E*-((4-carboethoxypiperidin-1-yl) carbonyl)ethenyl) phenyl]sulfide

The title compound was prepared by the procedures described in Example 285, substituting 4-fluoro-3-chlorobenzaldehyde with 2,3-dichloro-4-trifluoromechanesulfoxybenzaldehyde, providing a white solid. $^1$H NMR ($CDCl_3$, 300 MHz) δ 1.25 (t, *J*= 7.2 Hz, 3H), 1.62-1.79 (m, 2H), 1.87-2.04 (m, 2H), 2.41-2.63 (m, 1H), 2.85-3.41 (m, 2H), 3.85 (s, 3H), 3.87-4.10 (m, 1H), 4.15 (q, *J*= 7.2 Hz, 2H), 4.32-4.60 (m, 1H), 6.46 (d, *J*= 8.7 Hz, 1H), 6.54 (d, *J* = 3.0 Hz, 1H), 6.71 (d, *J*= 15.3 Hz, 1H), 7.15 (d, *J* = 3.0 Hz, 1H), 7.17 (d, *J* = 8.7 Hz, 1H), 7.36 (dd, *J* = 2.4, 8.4 Hz, 1H), 7.42 (d, *J* = 8.4 Hz, 1H), 7.88 (d, *J*= 2.4 Hz, 1H), 7.90 (d, *J*= 15.3 Hz, 1H). MS (ESI$^+$) (M+H)$^+$ at *m/z* 517, 519, 521. Anal. Calcd for $C_{26}H_{26}Cl_2N_2O_3S \cdot 0.12\ H_2O$: C, 60.10; H, 5.09; N, 5.39. Found: C, 60.09; H, 5.21; N, 5.54.

Example 340 (Reference Example)

(1-Methylindol-5-yl)[2,3-dichloro-4-( *E*-((4-carboxypiperidin-1-yl) carbonyl)ethenyl) phenyl] sulfide

The title compound was prepared by the procedures described in Example 155, substituting the ethyl ester from Example 137 with ethyl ester from Example 339, and KOH with NaOH, to give a white solid. $^1$H NMR$^-$(d$^6$-DMSO, 300 MHz) δ 1.31-1.53 (m, 2H), 1.62-1.76 (m, 2H), 1.94-2.09 (m, 1H), 2.88 (brt, *J*= 10.5 Hz, 1H), 3.13 (brt, *J* = 10.5 Hz, 1H), 3.86 (s, 3H), 3.93 (br d, *J*= 13.2 Hz, 1H), 4.09 (br d, *J* = 13.2 Hz, 1H), 6.41 (d, *J*= 8.7 Hz, 1H), 6.53 (dd, *J*= 0.9, 3.0 Hz, 1H), 7.04 (d, *J*= 15.3 Hz, 1H), 7.32 (dd, *J* = 2.1, 8.7 Hz, 1H), 7.48 (d, *J*=3.0 Hz, 1H), 7.64 (d, *J* = 8.7 Hz, 1H), 7.69 (d, *J* = 15.3 Hz, 1H), 7.73 (d, *J* = 8.7 Hz, 1 H), 7.88 (d, *J* = 2.1 Hz, 1H). MS (ESI$^+$) (M+H)$^+$ at *m/z* 489, 491, 493. Anal. Calcd for $C_{24}H_{21}Cl_2N_2NaO_3S \cdot 0\ H_2O$: C, 56.37; H, 4.14; N, 5.48. Found: C, 56.44; H, 4.38; N, 5.20.

An alternative method for preparing Example 340 is given below.

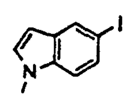

Example 340A

1-Methyl-5-iodoindole

To a solution of 5-iodoindole (75 g, 0.31 mol) in dry THF (750mL), at -78°C was added sodium hydride (60% in mineral oil, 14.85 g, 0.37 mol) in one portion. The suspension was stirred at -78°C for 1 hour after which iodomethane (28.8 mL, 0.46 mol) was added. The reaction mixture was stirred overnight with a slow elevation on temperature to room temperature(no more dry ice was added). Ether (600mL) and hexane (1.2L) were added and the mixture was washed with brine (1.6L) and water (1.5L), dried over $Na_2SO_4$ and filtered. The solution was concentrated and the residual brown solid was recrystallized from hexane to give the tide compound (66 g). The impure fraction from the mother liquor was flash chromatographed (8% EtOAc in hexane) to give an additional quantity of desired product (12.5 g, combined yield of 99%). MS ($DCI/NH_3$) m/e 258 (M+H)$^+$.

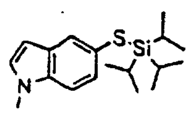

Example 340B

1-Methyl-S-triisopropylsilyl-5-indolethiol

Potassium hydride (35% in mineral oil, 12.03 g, 0.105 mol) was charged to a 250 mL RBF and was washed with dry THF (2x50mL). The resultant KH powder was then suspended in dry THF (75 mL), and cooled to 5 °C. Triisopropylsilylthiol (20.0 g, 0.105 mol) was slowly added via syringe over a period of 15 minutes. Vigorous escape of hydrogen gas was observed with addition of the thiol. The suspension was stirred at 5°C for 1 hour and became homogenous. After another hour stirring at room temperature, this solution was cannulated to a THF solution (100mL) containing Example 340A (24.5 g, 95.5 mmol) and tetrakis(triphenylphosphine)palladium(0) (2.2 g, 1.91 mmol). The yellow suspension was stirred at 70°C for 1 hour. After cooled, ether and hexane were added, and the mixture was washed with brine, dried ($Na_1SO_4$ and concentrated. The residual oil was purified by flash chromatography (silica gel, 3% EtOAc in hexane) to give the title compound (26.7 g, 88%). MS ($DCI/NH_3$) m/e 320 $(M+H)^+$.

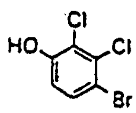

Example 340C

4-Bromo-2,3-dichlorophenol

To a solution of 2,3-dichlorophenol (200 g, 1.227 mol) in dichloromethane (800 mL), at 0 °C was added dropwise bromine (196.1g, 1.227 mol) from a dropping funnel within 1 hour. The red solution was stirred overnight (0°C - rt), and washed with 10% $NaHSO_3$. The organic phase was dried over $Na_2SO_4$, and concentrated. The residual white solid was recrystallized from hexane to give example 340C as white needles (207g, 70%). MS ($DCI/NH_3$) *m*/*e* 241 $(M+H)^+$.

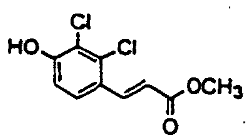

Example 340D

Methyl 2,3-dichloro-4-hydroxyphenylacrylate

A 1 L RBF was charged with Example 340C (48.4 g, 0.2 mol), $Pd_2(dba)_3$ (4.6 g, 5 mmol), $(Tol)_3P$ (4.66 g, 15.2 mmol), and purged with nitrogen. Dry DMF (300 mL), methyl acrylate (51.66 g, 0.6 mol) and triethylamine (84 mL, 0.6 mol) were then added. The reaction mixture was purged with nitrogen and stirred at 100°C (oil bath) for 16 hours. After cooled to room temperature, a lot of white crystalline material formed. Ethyl acetate (500 mL) and brine (not saturated, 800 mL) were added, and stirred. The white crystalline material dissolved. A little insoluble black solid (Pd) was filtered off. To the solution was then added, with stirring, saturated NaCl solution (2 L) and hexane (500 mL). The mixture was stirred for 1 hour. The formed yellowish solid was collected by filtration, washed with water (400 mL), acetonitrile (50 mL) and 1:1 ethyl acetate/hexane (500 mL), and dried to give pure desired compound (44.99g, 91%). MS ($DCVNH_3$) *m*/*e* 247 $(M+H)^+$.

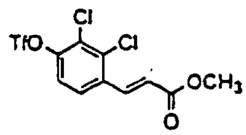

Example 340E

Methyl 2,3-dichloro-4-trifluoromethane sulfonyloxylphenylacrylate

To a suspension of Example 340D (18.62 g, 75.4 mmol) in pyridine (150 mL) at 5°C was added trifluoromethylsulfonyl anhydride (25.53 g, 90 mmol) very slowly. The suspension was stirred at 5 °C for 1 hour and became homogeneous. The solution was kept at 5 °C for 2 hours and at room temperature for 20 minutes. Ether (700mL) was added and the mixture was washed 10%HCl (700 mL)/brine (300 mL), 10%HCl (100 mL)/brine (900 mL), and brine (500 mL). The organic phase was dried ($Na_2SO_4$) and concentrated to give the title compound (24.86 g, 87%). MS ($DCINH_3$) m/e 379 $(M+H)^+$.

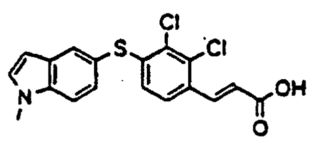

Example 340F

(1-Methylindol-5-yl)[2,3-dichloro-4-(*E*-(carboxyethenyl)phenyl] sulfide

To a solution of Example 340B (38.5 g, 0.12 mol) and Example 340E (30.3 g, 0.08 mol) in dry N-methylpyrrolidinone (300 mL) was added CsF (18.2 g, 0.12 mol) at 5°C under nitrogen atmosphere. After I hour stirring at the same temperature, the cooling bath was removed, and the mixture was stirred at room temperature for 0.5 hour. Ethyl acetate (800 mL) was added, and the mixture was washed with brine and water, and concentrated. The residual oil was separated by flash chromatography (20% EtOAc/hexane) to give a yellow solid (30 g).

This yellow solid was dissolved in THF (150 mL), and was added a solution of LiOH (4.0 g, 0.16 mol) in $H_2O$ (50 mL). The mixture was stirred at room temperature for 1 hour and more water (100 mL) was added to form a transparent solution. After overnight stirring the solution was acidified with 10 % aq. HCl. The mixture was concentrated under reduced pressure to about 100 mL. The formed solid material was collected by filtration, washed with water (200 mL), acetonitrile (30 mL), 1:1 ether/hexane, and dried to give the title compound (22.3 g, overall 74%). MS ($DCI/NH_3$) *m*/*e* 378 $(M+H)^+$.

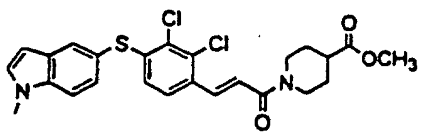

Example 340G

(1-Methylindol-5-yl)[2,3-dichloro-4-(*E*-((4-carbomethoxypiperidin-1-yl)carbonyl)ethenyl)phenyl]sulfide

To a solution of Example 340F (9.5 g, 25.1 mmol) and methyl isonipecotate (7.19 g, 50.2 mmol) in DMF (70 mL) was added EDC (9.64 g, 50.2 mmol), HOBt (6.78 g, 50.2 mmol) and triethylamine (7.0 mL, 50.2 mmol). The reaction mixture was stirred at room temperature for 15 hours. Ethyl acetate (800 mL) was added, and the mixture was washed with brine, and concentrated. The residue was purified by flash chromatography (60% EtOAc in hexane) to give example 340G as white powder (10.86 g, 94%). MS ($ESI^+$) *m*/*z* 503 $(M+H)^+$.

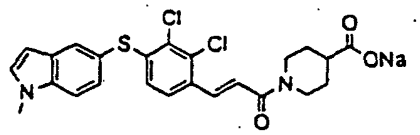

Example 340

(1-Methylindol-5-yl)[2,3-dichloro-4-(*E*-((4-carboxypiperidin-1-yl)carbonyl)ethenyl)phenyl] sulfide, sodium salt

To a suspension of Example 340G (11.8 g, 23.6 mmol) in THF (150 mL) was added a solution of lithium hydroxide monohydrate (1.98 g, 47.2 mmol) in $H_2O$ (30 mL). The mixture was stirred at room temperature overnight. Water (120 mL) was added and formed transparent solution was stirred for another hour before 10% HCl (30 mL) was added. The mixture was concentrated under reduced pressure to about 120 mL. The formed solid material was collected by filtration, washed with water, acetonitrile, and dried to give a white solid (11.0 g).

10.50 grams of the solid was suspended in methanol (60 mL), and was treated with a solution NaOH (0.8S9g) in methanol (20 mL). After all of the solid material went into solution, the solvent was removed under reduced pressure. The residual yellow oil was triturated with ether, and dried to give the title compound as yellow powder (11.33 g, 95%).

Example 348 (Reference Example)

(2-Isopropylphenyl)[2,3-difluoro-4-(*E*-((4-carboxypiperidin-1-yl)carbonyl)ethenyl) phenyl] sulfide

Prepared according to the procedures of Example 71, giving a yellow solid. $^1H$ NMR (DMSO-$d_6$, 300MHz) $\delta$ 1.18 (d, J = 6.8Hz, 6H); 1.30-1.91 ( br m, 4H); 2.50-3.50 (br m, 4H); 4.02-4.34 (br m, 2H); 6.62-6.72 (m, 1H); 7.23-7.73 (m, 7H). MS (APCI) $(M+H)^-$ at m/z 446.

Example 359 (Reference Example)

(Benzodioxan-6yl)[2,3-bis(trifluoromethyl)-4-(E-((4-carboxypiperidin-1-yl)carbonyl)ethenyl)phenyl]sulfide

Example 359A

1-Methyl-2,3-bis(trifluoromethyl)-7-oxabicyclo[2.2.1]hepta-2,5-diene

Hexafluoro-2-butyne (21.0 g, 0.13 mol) was transferred into a reaction bottle and added 2-methylfuran (12.86 g, 0.157 mol). This resulting mixture bottle was sealed and heated for 15 hr. at 120 °C. After cooling, the excess 2-methylfuran was rotoevaporated in vacuo at rt, to give crude title product (29 g, 92%), which was used directly.

Example 359B

4-Methyl-2,3-bis(trifluoromethyl)phenol

A mixture of Example 359A (12.0 g, 0.05 mol) and boron trifluoride-diethyl ether complex (150 ml) was stirred at room temp overnight, then neutralized carefully with 20% aqueous potassium carbonate, then the mixture was extracted with ether. The ether layer was dried over $MgSO_4$ and evaporated under reduced pressure to afford 10.4g (85%) of the title compound.

Example 359C

4-(4-Bromobenzene sufonyloxy-2,3-bis(trifluoromethyl)]benzylbromide

The phenol compound of Example 359B (10 g, 0.04 mol) was treated with 4-bromobenzenesulfonyl chloride (11.0 g, 0.043 mol) and Hunig's base (5.56 g, 0.043 mol) in $CH_2Cl_2$ (150 ml). The solution was washed with water, brine and dried over $MgSO_4$. After evaporating the solvent, N-bromosuccinimide (7.3 g, 0.04 mol) and benzoyl peroxide (200 mg) were added and the mixture was suspended in $CCl_4$ (100ml). The resulting mixture was refluxed for 13 h. When the reaction was cooled, the white solid was filtered and washed with $CCl_4$ to afford the crude title compound.

This crude product was used for next step without further purification.

Example 359D

4-Hydroxy-2,3-bis(trifluoromethyl)benzaldehyde

The crude product of Example 359C was dissolved in 60 ml of DMSO and 20 ml of $CH_2Cl_2$, and 12 g of trimethylamine N-oxide added. The resulting mixture was stirred at rt for 2.5 hr. The reaction mixture was poured into an ice cold 50% saturated aqueous NaCl solution (200 ml) and extracted with ether (3X100 ml). The combined organic layer was washed with brine and dried over $Na_2SO_4$. After evaporation of solvent, the product was purified by column chromatography, eluted with hexane:EtOAc (3:2) to provide 3.0 g of the title compound, plus 4.0 g of recovered 4-(4-bromobenzenesulfonyloxy-2,3-bis(trfluoromethyl)]toluene.

Example 359E

(Benzodioxan-6-yl)-[2,3-bis(trifluoromethyl)-4-(E-carboethenyl)phenyl]sulfide

The title compound was prepared according to the procedures described in Example 330, substituting the compound of Example 359D for 4-hydrox-2,3-dichlorobenzaldehyde.

Example 359F

(Benzodioxan-6yl)[2,3-bis(trifluoromethyl)-4-(E-((4-carboxypineridin-1-yl)carbonyl)ethenyl)phenyl]sulfide

The tide compound was prepared from Example 359E by the procedures described in Example 330, giving a white solid. $^1H$ NMR ($CD_3OD$, 300MHz) δ 1.65(br s, 2H), 1.93-2.04 (m, 2H), 2.57-2.65 (m, 1H), 2.95-3.05 (m, 1H), 3.25 (m, 1H), 4.12 (m, 1H), 4.28 (m, 4H), 4.41 (m, 1H), 6.92-7.03(m, 4H), 7.25 (d, J=9Hz, 1H), 7.72 (d, J=9Hz, 1H), 7.72-7.81 (m, 1H), MS (ESI) *m/e* 562 $(M+H)^+$. Anal calcd for $C_{25}H_{21}NO_5F_6S$: C, 53.48; H, 3.77; N, 2.49. Found: C, 53.42; H, 3.69; N, 2.25.

Example 363, (Reference Example)

(1-Methylindol-5-yl) [2,3-dichloro-4-(*E*-((4-(carbo-2,3-dihydroxypropylamino)piperidin-1-yl)carbonyl)ethenyl)phenyl] sulfide

To a solution of Example 340H (100mg, 0.2 mmol) and 3-amino-1,2-propanediol (37.4 mg, 0.41 mmol) in DMF (3 mL) was added EDC (78 mg, 0.41 mmol), HOBt (55 mg, 0.41 mmol) and triethylamine (0.057 mL, 0.41 mmol). The reaction mixture was stirred at room temperature for 15 hours. Ethyl acetate (60 mL) was added, and the mixture was washed with brine. The aqueous phase was extracted with 1.0% MeOH in methylene chloride. The combined organic phases were concentrated to dry. The residual material was triturated with water, filtered, washed with water, acetonitrile and ethyl acetate, and dried to give example 363 (92 mg, 80%). $^1H$ NMR (300 MHz, DMSO-$d_6$) δ 1.44 (m, 1H), 1.72 (m, 1H), 2.41 (m, 1H); 2.70 (t, 1H), 3.00 (m, 2H), 3.20 (m, 2H), 3.27 (m, 2H), 3.50 (m, 2H), 3.90 (s, 3H), 4.18 (br d, 1H), 4.40 (br d, 1H), 4.50 (t, 1H), 4.77 (d, 1H), 6.40 (d, 1H), 6.58 (d, 1H), 7. 19 (d, 1 H), 7.35 (d, 1H), 7.50(d, 1H), 7.66 (d, 1H), 7.70 (m, 2H), 7.80 (t, 1H), 7.88 (s, 1H). MS ($ESI^+$) *m/z* 562 $(M+H)^+$. Anal. calcd for $C_{27}H_{29}Cl_2N_3SO_4 \cdot 0.25H_2O$: C, 57.19; H, 5.24; N, 7.41. Found: C, 57.07; H, 5.22; N, 7.13.

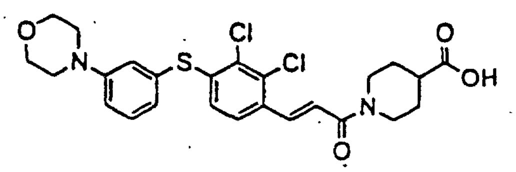

Example 384 (Reference Example)

[3-(4-Morpholino)phenyl] [2,3-dichloro-4-(*E*-((4-carhoxypiperidin-1-yl)carbonyl)ethenyl phenyl] sulfide

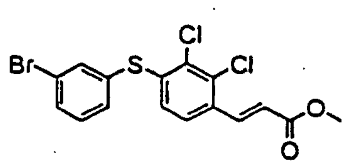

Example 384A

(3-Bromophenyl) [2,3-dichloro-4-(*E*-[methoxycarbonyl]ethenyl)phenyl] sulfide

To a solution of the resultant compound from Example 340E (12.0 g, 31.7 mmol) in N-methylpyrrolidinone (63 mL) at 0 °C (under dry $N_2$) was added 3-bromothiophenol (4.0 mL, 7.3 g, 38.8 mmol) and a solution of lithium *tert*-butoxide (3.1 g, 38.8 mmol), and the resulting solution was stirred for 3 h at 0 °C. The reaction was diluted with 500 mL EtOAc and extracted sequentially with 100 mL water, 3 x 60 mL of 1 N aq. NaOH, then 2 x 100 mL brine. The organic phase was dried over $Na_2SO4$, filtered, and concentrated in vacuo to produce the crude title compound (9.2 g). $^1H$ NMR (DMSO-$d_6$, 300 MHz) δ 3.75 (s, 3H), 6.67 (d, J=15 Hz, 1 H), 6.83 (d, J=9 Hz, 1H), 7.46-7.59 (m, 2H), 7.72-7.76 (m, 2H), 7.80 (t, J=2.5 Hz, 1H), 7.85 (d, J=9 Hz, 1H), 7.88 (d, J=15 Hz, 1H); MS (APCI) *m/e* 419 $(M+H)^+$.

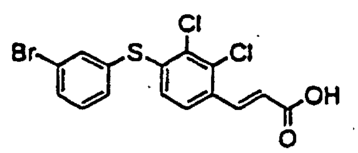

Example 384B

(3-Bromophenyl) [2,3-dichloro-4-(*E*-carboxyethenyl)phenyl) sulfide

Using the procedure for Example 340H, Example 348A was hydrolyzed to the title compound. $^1H$ NMR (DMSO-$d_6$, 300 MHz) δ 6.56 (d, J=16.5 Hz, 1H), 6.84 (d, J=9 Hz, 1H), 7.45-7.58 (m, 2H), 7.72 (m, 1H), 7.77-7.86 (m, 4H), 12.75 (br s, 1H); (ESI) *m/e* 401, 403 $(M-H)^-$.

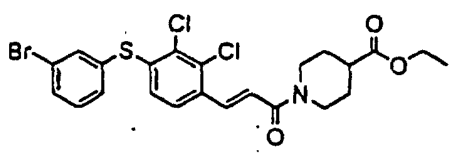

Example 384C

(3-bromophenyl)[2,3-dichloro-4-(*E*-[(4-ethoxycarbonylpiperidin-1-yl)carbonyl]ethenyl)phenyl]sulfide

The title compound (750 mg, 58%) was prepared from Example 384B (1.0 g, 2.48 mmol), using the procedures described in Example 340G substituting methyl isonipecotate with ethyl isonipecotate. $^1H$ NMR (300 MHz, DMSO-$d_6$) δ 1. 18 (t, J=7.5 Hz, 3H), 1.38-1.56 (m, 2H), 1.82-1.92 (m, 2H), 2.50-2.69 (m, 1H), 2.80-2.93 (m, 1H), 3.14-3.27 (m, 1H), 4.07 (t, J=7.5 Hz, 2H), 4.10-4.35 (m, 2H), 6.92 (d, J=9 Hz, 1H), 7.30 (d, J=15 Hz, 1H), 7.43-7.52 (m, 2H), 7.67-7.77 (m, 3H), 7.92 (d, J=9 Hz, 1H).

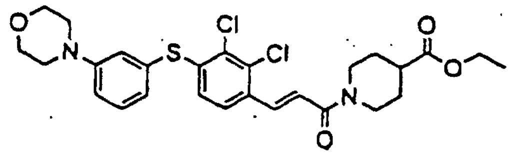

Example 384D

[3-(4-Morpholino )phenyl] [2,3-dichloro-4-(*E*-[(4-ethoxycarbonylpineridin-1-yl)carbonyl]ethenyl)phenyl sulfide

The procedure of D. W.; Wolfe, J. P.; Buchwald, S. L. *J Am. Chem. Soc.* **1998**, *120*, 9722-9723, was adapted. To a stirred solution of Example 384D (180 mg, 0.331 mmol) in ethylene glycol dimethyl ether (1 mL) containing 1-(N, N-dimethylarnino)-1'-(dicyclohexylphophino)biphenyl (7 mg, 5 mol%), $Pd_2(dba)_3$ (8 mg, 2.5 mol%), and morpholine (0.058 ml, 0.663 mmol) was added powdered $K_3PO_4$ (141 mg, 0.663 mmol). The reaction mixture was bubbled with $N_2$ for 5 min and heated at 90 °C in sealed tube for 18 h. Then the solvent was removed under reduced pressure and residue was diluted with methylene chloride (1 mL). The title compound (90 mg, 50%) was isolated by flash chromatography on silica gel eluting with 20% acetone-hexane. $^1$H NMR (DMSO-$d_6$, 300 MHz) δ 1.18 (t, J=7.5 Hz, 3H), 1.35-1.55 (m, 2H), 1.79-1.91 (m, 2H), 2.58-2.69 (m, 1 H), 2.70-2.94 (m, 2H), 3.16 (t, J=4.5 Hz, 2H), 3.15 (t, J=5 Hz, 4H), 3.73 (t, J=4.5 Hz, 4H), 3.78 (t, J=5 Hz, 2H), 4.08 (q, J=7.5 Hz, 2H), 4.11-4.36 (m, 2H), 6.70 (d, J=8.25 Hz, 1H), 6.97 (m, 1H), 7.10-7.27 (m, 2H),7.24 (d, J=15 Hz, 1H, 7.39 (m, 1H), 7.73 (d, J=15 Hz, 1H), 7.86 (d, J=8.25 Hz, 1H); MS (ESI) *m/e* 549, 551 $(M+H)^+$.

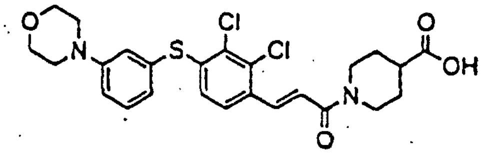

Example 384E

[3-(4-Morpholino)phenyl] (2,3-dichloro-4-(*E*-[(4-carboxypiperidin-1-yl)carbonyl]ethenyl)phenyl] sulfide

The title compound (38 mg, 67%) was prepared from Example 384D (60 mg, 0.1 mmol) using the procedures described in Example 340H. $^1$H NMR (DMSO-$D_6$, 400 MHz) δ 1.37-1.55 (m, 2H), 1.81-1.90 (m, 2H), 2.52-2.58 (m, 1H), 2.80-2.94 (m, 1H), 3.10-3.15 (m, 3H), 3.67-3.75 (m, 3H), 3.76-3.99(m, 3H), 4.04-4.16 (M, 1H), 4.22-4.33 (m, 1H), 6.71 (d, J=8Hz, 1 H), 6.96 (d, J=7Hz, 1 H), 7.07 (m, 1H), 7.12 (s, 1H), 7.24 (d, J=15Hz, 1H), 7.38 (t, j=7Hz, 1H), 7.73 (d, J=15Hz, 1H), 7.85 (d, J=8Hz, 1H). MS (ESI) *m/e* 521, 523 $(M+H)^+$, 519, 521 $(M-H)^-$.

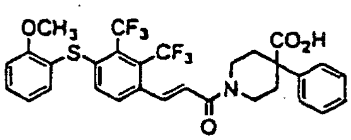

Example 385

(2-Methoxyphenyl)[2,3-bis(trifluoromethyl)-4-(E-((4-phenylcarboxypiperidin-1-yl)carbonyl)ethenyl)phenyl]sulfide

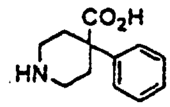

Example 385A

4-Phenylpiperidine-4-carboxylic acid

4-Cyano-4-phenylpiperidine hydrochloride (2.0g, 0.11 mol) was dissolved in 8 mL of conc. $H_2SO_4$ and 4mL of $H_2O$, then the solution was heated at reflux for 4 h. The solution was cooled and then NaOH was added to precipitate a white solid. The solid was collected, then dissolved in methanol, and the solution was filtered and concentrated to obtain a white solid. This dried solid was used for without purification for Example 385B.

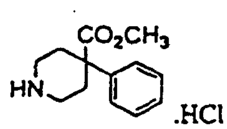

Example 385B

Methyl 4-phenylpiperidine-4-carboxylate hydrochloride

Dissolved the 4-phenylpiperidinecarboxylic acid in 10 mL of methanol and added 2 mL of thionyl chloride dropwise at room temperature. The resulting mixture was stirred overnight at room temperature. Evaporated solvent in vacuo, added toluene and evaporated excess thionyl chloride in vacuo. This white powder salt was used for next step without further purification.

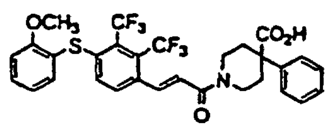

Example 385C

(2-Methoxyphenyl)[2,3-bis(trifluoromethyl)-4-(*E*-((4-phenylcarboxypiperidin-1-yl)carbonyl)ethenyl)phenyl]sulfide

The methyl ester of the title compound was prepared by the procedures described in Example 356, employing the compound of Example 359D as starting material, to give an oil. The resultant methyl ester was hydrolyzed with aq. NaOH in methanol at 60 °C for 4 h to give a white solid. $^1$H NMR ($CD_3OD$, 300 MHz) δ 1.88 (br t, J= 13.5 Hz, 2H, 2.59 (br d, J= 13.5 Hz, 2H), 3.13(br t, J= 13.5 Hz, 1H), 3.75 (s, (br t, J= 13.5 Hz, 2H), 2.59(br d. J=13.5 Hz, 2H), 3.13(br t, J= 13.5 Hz, 1H), 3.75 (s, H), 3H), 3.44 (br t,1=13.5 Hz, 1H), 4.12 (br d,1=13.5Hz, 1H), 4.42(br d, J=13.5 Hz, 1H), 6.35 (d, J=15 Hz, 1H), 7.0-7.46 (m, 7H), 7.43-7.55 (m, 3H), 7.62-7.85 (m, 2H); MS(ESI) *m/z* 610(M+H)$^+$. Anal calcd for $C_{30}H_{23}F_6NO_4S{\cdot}H_2O$: C, 57.49; H, 4.13; N, 2.20. Found: C, 57.12; H, 3.93; N, 1.77.

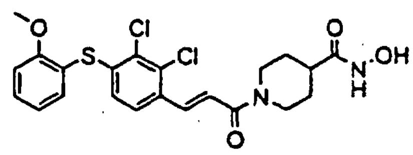

Example 386

(2-Methoxyphenyl) (2,3-dichloro-4-(E-(((4-hydroxylaminocarbonyl)piperidin-1-yl)carbonyl)ethenyl)phenyl] sulfide

To a suspension of Example 319 (300 mg, 0.64 mmol) in $CH_2Cl_2$ (10 mL) was added oxalyl chloride (67 μL) and 2 drops of DMF. The yellow suspension was stirred at room temperature for 2 h to give an orange solution which was then concentrated under reduced pressure, and dried under vacuum. An aliquot of the resulting acid chloride solution (2 mL) was added to a solution containing o-trimethylsilyloxyamine (101 mg, 0.96 mmol), Hunig's base (122 μL, 0.7 mmol) and DMAP (2 mg) in $CH_2Cl_2$ (3 mL). After the solution was stirred at room temperature for 1h, TBAF (1.0 M solution in THF, 1.5 mL) was then added. The brown solution was stirred at room temperature for another h, then it was purified by HPLC (Zorbax, C-18) to give the title compound as white solid (71 mg). $^1H$ NMR (300 MHz, DMSO-$d_6$) δ 1.50 (m, 2H), 1.70 (m, 2H), 2.28 (m, 1 H), 2.70 (m, 1H), 3.09 (m, 1 H), 3.79 (s, 3H), 4.23 (m, 1H), 4.45 (m, 1H), 6.55 (d, J = 8.8 Hz, I H), 7.08 (t, J = 7.4 Hz, 1H), 7.25 (m, 2H), 7.48 (d, J = 7.2 Kz, 1H), 7.54 (t, J = 8.2 Hz, 1H), 7.73 (d, J = 15.3 Hz, 1 H), 7.82 (d, J = 8.8 Hz, 1H), 8.55 (br s, 1H), 10.46 (s, 1H), MS (ESI$^+$) *m/z* 481 $(M+H)^+$.

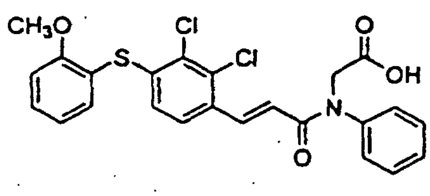

Example 387

(2-Methoxyphenyl)[2,3-dichloro-4-(*E*-((N-carboxymethyl-N-phenylamino)carbonyl)ethenyl)phenyl] sulfide

The title compound was prepared by the procedures described in Example 1C substituting Example 1B with (2-methoxy) [2,3-dichloro-4-(*E*-(2-carboxyethenyl)phenyl] sulfide and substituting 6-amino-1-hexanol with N-phenylglycine ethyl ester following by hydrolysis. $^1H$ NMR (300 MHz, DMSO-$d_6$) δ 3.76 (s, 3H), 4.40 (s, 2H), 6.35 (d, J = 15.5 Hz, 1H), 6.46 (d, J = 8.4 Hz, 1H), 7.05 (t, J = 7.3 Hz, 1H), 7.22 (m, 2H), 7.35 (t, *J* = 7.5 Hz, 3H), 7.44 (t, J = 7.2 Hz, 3H), 7.55 (t, 1= 7.4 Hz, 1H), 7.76 (d, J = 15.4 Hz, 1H); MS (ESI$^+$) *m/z* 488,490 $(M+H)^+$. Anal. calcd for $C_{24}H_{19}NCl_2O_4S$: C, 59.02; H, 3.92; N, 2.87. Found: C, 58.71; H, 4.10; N, 2.58.

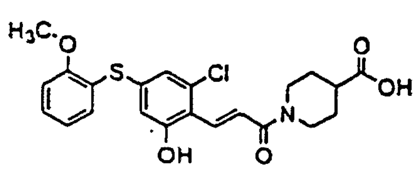

Example 388

(2-Methoxyphenyl) [3-chloro-6-hydroxy-4-(E-((3-carboxypiperidin-1-yl)carbonyl)ethenyl)phenyl]sulfide

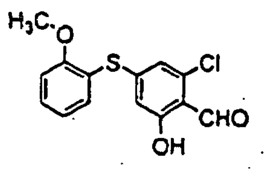

Example 388A

(2-Methoxyphenyl) ((3-chloro-6-hydroxy-4-formyl) pheny)sulfide

2-Methoxythiophenol (3.5 mL, 28.9 mmol) and 2,4-dichoro-6-hydroxybenzaldehyde (5.00g, 26.3 mmol) were processed as described in Example 1 to provide the title disulfide (6.71 g, 87%) as a pale yellow solid. $^1$H NMR (DMSO-$d_6$, 300 MHz) δ 10.18 (s, 1H), 7.61 (dd, *J*=7.4 Hz, *J*=1.7 Hz, 1H), 7.56 (dd, *J*=7.7 Hz, *J*=1.9 Hz, 1H), 7.25 (d, *J*=7.3 Hz, 1H), 7.11 (dt, *J*=7.7 *Hz, J*=1.5 Hz, 1H), 6.69 (d, *J*=1.8 Hz, 1H), 6.38 (d, *J*=1.5 Hz, 1H), 3.80 (s, 3H); MS (AFCI)*m*/*z* 294 $(M+H)^-$.

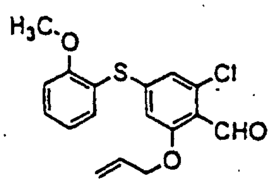

Example 388B

(2-Methoxyphenyl)(3-chloro-6-allyloxy-4-benzaldehyde)sulfide

Allyl bromide (2.0 mL, 22.8 mmol) was added to a stirred solution of Example 388A (6.71 g, 22.8 mmol), cesium carbonate (14.86 g, 45.6 mmol), and DMF (45 mL). After 21 h, the pale yellow solution was diluted with 1 N aqueous HCl (100 mL) and extracted with $Et_2O$ (2x75 mL). The ether extracts were combined, dried ($MgS_{O4}$), filtered, and concentrated to a yellow solid (7.20 g, 94%). $^1$H NMR (DMSO-$d_6$, 300 MHz) δ 10.28 (s, 1H), 7.58 (dd, *J*=8.4 Hz, *J*=1.7 Hz, 1 H), 7.52 (dd, *J*=7.8 Hz, *J*=1.7 Hz, 1H), 7.23 (d, *J*=8.1 Hz, *J*=1.0 Hz, 1H), 7.08 (dt, *J*=7.8 Hz, *J*=1.4 Hz, 1H), 6.82 (d, *J*=1.7 Hz, 1H), 6,52 (d, *J*=1.7 Hz, 1H), 5,97 (m, 1H), 5.33 (d, *J*=17.3 Hz, 1H), 5.28 (d, *J*=*10.8* Hz, 1H), 4.61 (m, 2H), 3.80 (s, 3H); MS (APCI) m/z 335 $(M+H)^+$.

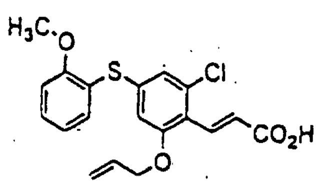

Example 388C

(2-Methoxy-phenyl) [3-chloro-6-allyloxy-4-((carboxy)ethenyl)phenyl]sulfide

Example 388B was processed as detailed in Example 1H. $^1$H NMR (DMSO-$d_6$, 300 MHz) δ 7.77 (d, *J*=16.3 Hz, 1H), 7.51 (dd, *J*=7.4 Hz, *J*=1.7 Hz, 1H), 7.43 (dd, *J*=7.4 Hz, *J*=1.7 Hz, 1H), 7.19 (dd, *J*=8.1 Hz, *J*= 1.0 Hz, 1H), 7.05 (dt, *J*=7.4 Hz, *J*=1.4 Hz, 1H), 6.82 (d, *J*= *1*.3 Hz, 1H), 6.72 (d, *J*=15.9 Hz, 1H), 6.66 (d, *J*=1.7 Hz, 1H), 6.00 (m,

1H), 5.30 (d, *J*=9.8 Hz, 1H), 5.26 (d, *J=3.1* Hz, 1H), 4.63 (m, 2H), 3.80 (s, 3H). MS (APCI) m/z 377 ($M+H^+$) 394 ($M+NH_4^+$).

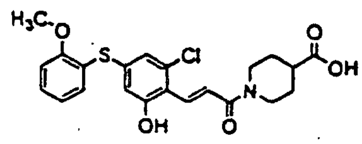

Example 388D

(2-Methoxyphenyl)[3-chloro-6-hydroxy-4-(E-((3-carboxypiperidin-1-yl)carbonyl)ethenyl)phenyl]sulfide

The allyl group of Example 388C was removed as reported in the literature (Honda, M.; Morita, H.; Nagakura, I. J. Org. Chem. 1997, 62, 8932.) and the carboxylic acid was converted to the amide as reported in Example 165 to provide the title compound as a white solid. $^1$H NMR(DMSO-$d_6$, 300MHz) δ 7.73 (d, *J*=16.3 Hz, 1H), 7.51 (d, *J*=7.4 Hz, 1H), 7.43 (d, *J*=7.4 Hz, 1H), 7.19 (d, *J*=7.9 Hz, 1H), 7.05 (dt, J=7.8Hz, J=1.1 Hz, 1H), 6.70(d, *J*=1.8Hz, 1H), 6.59(d, *J*=6.59Hz, 1H),4.30 (m, 1H), 3.95 (m, 2H), 3.80 (s, 3H), 2.85 (m, 2H), 1.87 (m, 2H), 1.45 (m,2H). MS (APCI) *m/z* 448 ($M+H^+$).

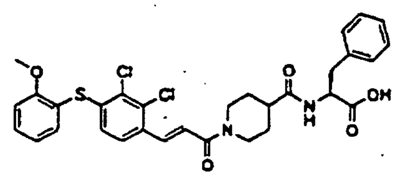

Example 389

(2-Methoxyphenyl) [2,3-dichloro-4-(*E*-(4-((1-(2-phenyl-1-carboxyethyl)amino)carbonyl)piperidin-1-yl)carbonyl) ethenyl)phenyl]sulfide

The methyl ester of the title compound was prepared by the procedure described in Example 363 using L-phenylalanine methyl ester as the coupling substrate. The methyl ester was then hydrolyzed as described in Example 340 to provide the title compound. HPLC (Supelco C-18 column, water:acetonitrile 100 :0-0: 100, 20 minute elution, flow rate 1.5 mL/min, RT = 13.97 min; 1H NMR (300 MHz, DMSO-d6) δ 1.45. (m, 2H), 1.56 (m, 1H), 1.68 (m, 1H), 2.41 (m, 1H), 2.71 (m, 1H), 2.83 (m, 2H), 3.08 (m, 2H), 3.79 (s, 3H), 4.12 (m, 1H), 4.30 (m, 1H), 4.41 (m, 1H), 6.55 (d, 1H), 7.09 (t, 1H), 7.22 (m, 6H), 7.48 (dd, 1H), 7.57 (m, 1H), 7.72 (d, 1H), 7.81 (d, 1H), 8.11 (m, 1H), 12.64 (br s, 1H); MS (ESI) m/e 613 $(M+H)^+$.

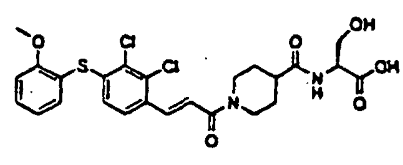

Example 390

(2-Methoxyphenyl) [2,3-dichloro-4-(*E*-(4-((1-(2-hydroxy-1-carboxyethyl)amino)carbonyl)piperidin-1-yl)carbonyl) ethenyl)phenyl]sulfide

The methyl ester of the title compound was prepared by the procedure described in Example 363 using L-serine methyl ester as the coupling substrate. The methyl ester was then hydrolyzed as described in Example 340 to give the title compound. HPLC (Supelco C-18 column, water:acetonitrile 100:0- 0:100, 20 minute elution, flow rate 1.5

mL/min, RT = 11.79 min; $^1$H NMR (300 MHz, DMSO-$d_6$) δ 1.48 (m, 2H), 1.72 (m, 2H), 2.55 (m, 2H), 2.71 (m, 1H), 3.10 (m, 1H), 3.62 (m, 2H), 3.79 (s, 3H), 4.22 (m, 2H), 4.41 (m, 1H), 6.55 (d, 1 H), 7.09 (t, 1H), 7.34 (m, 2H), 7.48 (m, 1H), 7.57 (m, 1H), 7.71 (d, 1H), 7.81 (d, 1H), 7.96 (br d, 1H); MS (ESI) *m/e* 553 $(M+H)^+$.

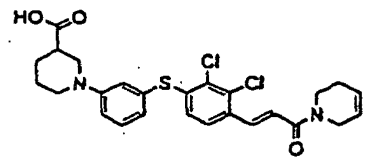

Example 391

(3-(1-(3-Carboxypiperidinyl)phenyl)[2,3-dichloro-4-(E((1,2,5,6-tetrahydropyridin-1-yl)carbonyl)ethenyl)phenyl]sulfide

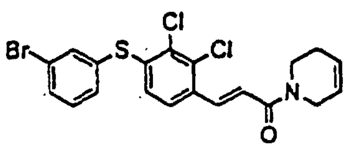

Example 391A

(3-bromophenyl)[2,3-dichloro-4-(*E*-[(1,2,3,6-tetrahydropyridin-1 yl)carbonyl]ethenyl)phenyl]sulfide

The title compound (1.2 g, 103%) was prepared from Example 384B (1.00 g, 2.48 mmol), using the procedures described in Example 340G substituting methyl isonipecotate with 1,2,3,6-tetrtahydropyridine. $^1$H NMR (300 MHz, DMSO-$d_6$) δ 2.09-2.2 (m, 2H), 3.61-3.68 (m, 1H), 3.70-3.77 (m, 1H), 4.03 (m, 1H), 4.18 (m, 1H), 5.69-5.78 (M, 1H), 5.80-5.93 (m, 1H), 6.93 (d, J=9 Hz, 1H), 7.20-7.37 (m, 1H), 7.43-7.56 (m, 3H), 7.67-7.79 (m, 2H), 7.88-7.97 (m, 1H); MS (ESI) *m/e* 470,472 $(M+H)^+$

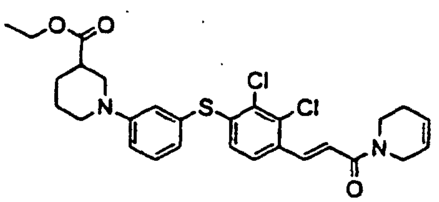

Example 391B

[3-(3-ethoxycarbonylpiperidine)] [2,3-dichloro4-(*E*-[(1.2,3.6-tetrahydropyridin-1-yl)carbonyl]ethenyl)phenyl] sulfide

The title compound (50 mg, 46%0 was prepared by the procedures described in Example 384D, substituting morpholine with ethyl nipecotate. $^1$HNMR(300 MHz, DMSO-$d_6$) δ 1.17 (t, J=6.8 Hz, 3H), 1.5-1.76 (m, 3H), 1.82-1.95 (m, 1H), 2.06-2.19 (m, 2H), 2.56-2.67 (m, 1H), 2.84-2.96 (m, 1H), 3.06-3.13 (m, 1H), 3.43-3.52 (m, 1H), 3.61-3.74 (m, 2H), 3.99-4.18 (m, 4H), 5.66-5.91 (m, 2H), 6.73 (d, J=9 Hz, 1H), 6.92 (d, J=7.5 Hz, 1H), 7.06-7.12 (m,2H), 7.31-7.39 (m, 2H), 7.75 (d, J=15 Hz, 1H), 7.80-7.91 (m, 1H); MS (ESI) *m/e* 545, 547 $(M+H)^+$.

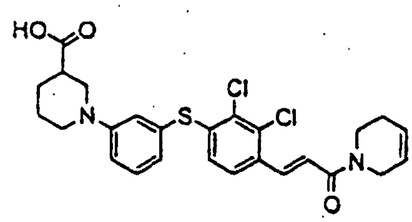

Example 391C

[3-(3-carboxylpiperidine)] [2,3-dichloro-4-(*E*-[(1,2,3,6-tetrahydropyridine)-1-yl)carbonyl]ethenyl)phenyl]sulfide

The title compound (20 mg, 49%) was prepared from Example 39 1 B (43 mg, 0.08 mmol) using the procedures described in Example 340H. $^1$H NMR (DMSO-$d_6$, 500 MHz) δ 1.51-1.64 (m, 2H), 1.68-1.73 (m, 1H), 1.87-1.94 (m, 1H), 2.07-2.19 (m, 2H), 2.51-2.57 (m, 1H), 2.83-2.89 (m, 1H), 2.99-3.04 (m, 1H), 3.61-3.73 (m, 4H), 4.02 (br s, 1H), 4.15 (br s, 1H), 5.67-5.76 (m, 1H), 5.79-5.90 (m, 1H), 6.72 (d, J=7.5 Hz, 1H), 6.92 (J=6.25 Hz, 1H), 7.10-7.13 (m, 2H), 7.14-7.30 (m, 1H), 7.36 (t, J=7.5 Hz, 1H), 7.74 (d, J=15 Hz, 1H), 7.80-7.90 (m, 1H); MS (ESI) *m/e* 517, 519 $(M+H)^+$.

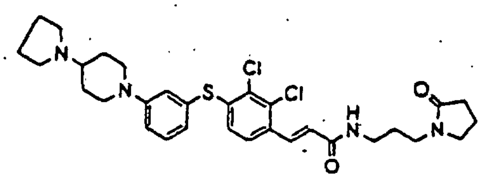

Example 392

(3-(4-pyrrolidin-1-yl)piperidin-1-yl)phenyl) [2,3-dichloro-4-(E-(((3-(2-pyrrolidinon-1-yl)propylamino)carbonyl)ethenyl] phenyl]sulfide

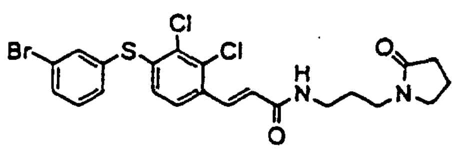

Example 392A

(3-bromophenyl) [2,3-dichloro-4-(E-(((3-(2-pyrrolidinon-1-yl)propylamino)carbonyl)ethenyl)phenyl]sulfide

The title compound (1.25 g, 95%) was prepared from Example 384B (1.00 g. 2.475 mmol), using the procedures described in Example 340G substituting methyl isonipecotate with 3-aminopropylpyrrolidine. MS (ESI) *mle* 529, $(M+H)^+$, 527, $(M-H)^-$.

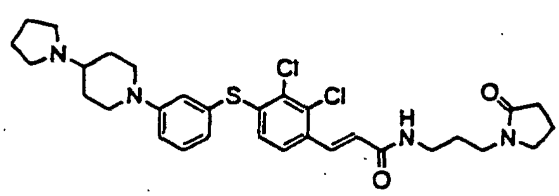

Example 392B

(3-(4-Pyrrolidin-1-yl)piperidin-1-yl)phenyl) [2,3-dichloro-4-(E-(((3-(2-pyrrolidinon-1-yl)propylamino)carbonyl)ethenyl) phenyl]sulfide

The title compound (32 mg, 27%) was prepared from Example 392A as described in Example 384D, substituting morpholine with 4-(1-pyrrolidinyl)piperidine. $^1$H NMR (500 MHz, DMSO-$d_6$) δ 1.60-1.67 (m, 4H), 1.84-2.90 (m, 5H), 2.91-2.03 (m, 1H), 2.04-2.11 (m, 3H), 2.20 (t, J=7.5 Hz, 2H), 2.75 (br t, J=12.5 Hz, 2H), 3.00-3.16 (m, XH), 3.21 (t, J=7.5Hz, XH), 3.33(m, 1H), 3.46-3.64 (m, 1H), 3.87 (br d, J=10Hz, 2H), 6.59 (d, J=15 Hz, 1H), 6.80 (d, J=8.75 Hz, 1H), 6.94 (d, J=7.5 Hz, 1H), 7.12-7.18 (m, 2H), 7.33 (t, J=7.5 Hz, 1H), 7.57 (d, J=8.75 Hz, 1H), 7.68 (d, J= 15 Hz, 1H), 8.24 (t, J=5Hz, 1H); MS (ESI) *m/e* 601, 603, $(M+H)^-$; 599, 601 $(M-H)^-$.

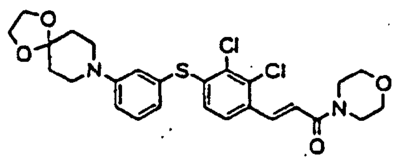

Example 393

[3-(4-Spiro-2,2-dioxolanyl)piperidin-1-yl)pheny] [2,3-dichloro-4-(E-((4-morpholinyl)carbonyl)etheny)phenyl]sulfide

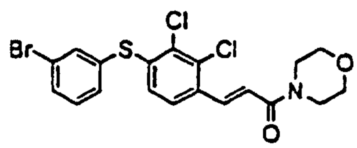

Example 393A

(3-bromophenyl) [2,3-dichloro-4-(*E*-((4-morpholino)carbonyl)ethenyl)phenyl] sulfide

The title compound (980 mg, 84%) was prepared from Example 384B (1.0.0 g, 2.48 mmol), using the procedures described in Example 340G substituting methyl isonipecotate with morpholine. $^1$H NMR (400 MHz, DMSO-$d_6$) 3.53-3.63 (m, 6H), 3.68 (br s, 2H), 6.93 (d, J=8 Hz, 1H), 7.27 (d, J=15 Hz, 1H), 7.44-7.52 (m, 2H), 7.67-7.74 (m, 2H), 7.78 (d, 7=15 Hz, 1H), 7.80 (d, J=8 Hz, 1H); MS (ESI) *m/e* 474 $(M+H)^+$.

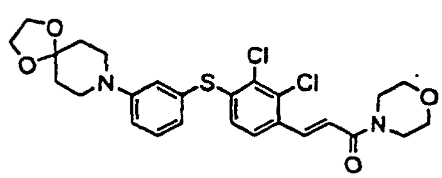

Example 393B

[3-(4-(Spiro-2.2-dioxolanyl)piperidin-1-yl)phenyl[2,3-dichloro-4[E-((4-morpholinyl)carbonyl)ethenyl)phenyl]sulfide

The title compound (32 mg, 27%) was prepared from Example 393A as described in Example 384D, substituting morpholine with 1,4-dioxa-8-azaspiro[4,5]decane. $^1$H NMR (500 MHz, DMSO-$d_6$) δ 1.68 (t, J=S Hz. 4H), 3.52-3.60 (m, 7H), 3.66 (br s, 2H), 3.91 (s, 4H), 6.71 (d, J=8.75 Hz, 1H), 6.91 (m, 1H), 7.11-7.13 (m, 2H), 7.22 (d, J=15 Hz, 1H), 7.35 (m, 1H), 7.76 (d, J=1 5 Hz, I H), 7.85 (d, J=8.75 Hz, 1H); MS (ESI) *m/e* 535, 537 $(M+H)^+$.

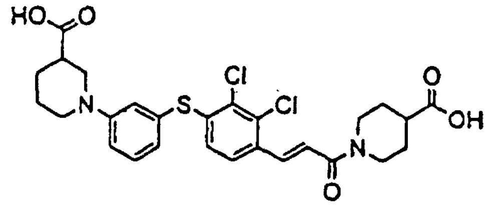

Example 394

[3-(3-Carboxylpiperidin-1-yl)phenyl] [2,3-dichloro-4-(*E*-[(4-carboxypiperidin-1-yl)carbonyl]ethenyl)phenyl] sulfide

The title compound (51 mg, 41%) was prepared from Example 384C as described in Example 384D, substituting morpholine with ethyl nipccotate followed by hydrolysis with LiOH as described in Example 340H. $^1$H NMR (400 MHz, DMSO-$d_6$) δ 1.39-1.60 (m, 4H), 1.67-1.76 (m,1H), 1.82-1.95 (m, 3H), 2.52-2.59 (m, 3H), 2.81-2.93 (m, 2H), 2.99-3.07 (m, 1H), 3.14-3.25 (m, 1H), 3.47-3.54 (m, 1H), 3.69 (dd, $J_1$=4 Hz, $J_2$=12 Hz, 1H), 4.05-4.17 (m, 1H), 4.24-4.34 (m, 1H), 6.72 (d, J=8 Hz, 1H), 6.92 (d, J=8 Hz, 1H), 7.11 (m, 2H), 7.23 (d, J=15 Hz, 1H), 7.34-7.40 (m, 1H), 7.73 (d. J=15 Hz. 1H), 7.85 (d,1=8 Hz, 1H); MS (ESI) *m/e* 563,565 $(M+H)^+$.

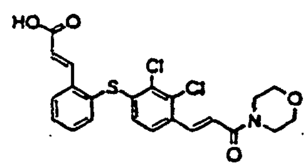

Example 395

(2-(2-Carboxy)ethenyl)phenyl)[2,3-dichloro-4-(E-((4-morpholinyl)carbonyl)ethenyl)phenyl]sulfide

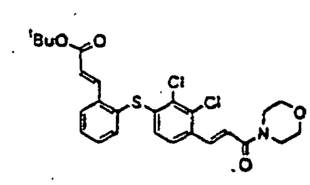

Example 395A

(2-(2-*Tert*-butyloxycarbonyl)ethenyl)phenyl) [2,3-dichloro-4-(*E*-((4-morpholinyl)carbonyl(ethenyl)phenyl]sulfide

A solution of Example (2-bromophenyl) (50 mg, 0.11 mmol), tris(benzylidineacetone)dipalladium[0] (5.1 mg, 0.0056 mmol), and tri-o-tolylphosphine (1 l mg, 0.035 mmol) in 0.2 mL DMF was degassed with nitrogen gas for 10 min, then triethylamine (50 μL, 36 mg, 0.36 mmol) and tert-butyl acrylate (50 μL, 44 mg, 0.34 mmol) were added to the solution, and the vessel was sealed under nitrogen and heated in a 100 °C oil bath for 17 h. The reaction was concentrated under hi-vacuum, and the residue was partially purified by preparative TLC eluting with 10% acetone-$CH_2Cl_2$, to provide 42 mg (0.080 mmol, 73%) of the title compound as a crude material. The compound was further purified by preparative HPLC (30-100% MeCN in 0.1% aqueous TFA, 40 min elution, C-1 8 reverse-phase Sorbax 10 mm column, producing 26 mg (0.051 mmol, 47%) of the title compound as a glass. $^1$H NMR (300 MHz, $CDCl_3$) δ 1.47 (s, 9H), 2.3-2.7 (v br s, 5H), 3.54-3.90 (2 br m, 8H), 6.32 (d, J=16 Hz, 1H), 6.46 (d, J=8 Hz, 1H), 6.69 (br d, J=15 Hz, 1H), 7.24 (br d, partially overlapped with $CHCl_3$, approx. 1H), 7.40-7.54 (m, 2H), 7.59 (dd, J=2,8 Hz, 1H), 7.75 (dd, J=2,8 Hz, 1H), 7.94 (br d, J=15 Hz, 1H), 7.98 (d, J=16 Hz, 1H); MS (ESI) *m/e* 520, 522 $(M+H)^+$.

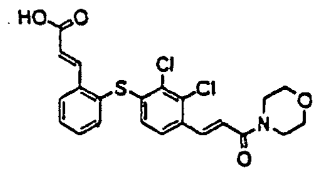

Example 395B

(2-(2-Carboxy)ethenyl)phenyl) [2,3-dichloro-4-(E-((4-morpholinyl)carbonyl)ethenyl)phenyl]sulfide

Example 395A (26 mg, 0.050 mmol) was dissolved in 1 mL chloroform and 1 mL TFA and the solution was stirred at ambient temperature for 1 h. The solvent was concentrated under reduced pressure to provide 25 mg (109%) of the title compound as an 85:15 mixture of *E*- and Z-cinnamide isomers. Data for major isomer: $^1$H NMR (300 MHz, $CDCl_3$) δ 3.55.3.85 (2 br m, 9H), 6.42 (d, J=16 Hz, 1H), 6.47 (d, J=8 Hz, 1H); 6.69 (d, J=15 Hz, 1H), 7.24 (d, partially overlapped with $CHCl_3$, approx. 1H), 7.43-7.56 (m, 2H), 7.78 (dd, J=2,8 Hz, 2H), 7.93 (d, J=15 Hz, 1H), 8.23 (d, J=16 Hz, 1H); MS (ESI) *m*/*e* 464, 466 $(M+H)^+$.

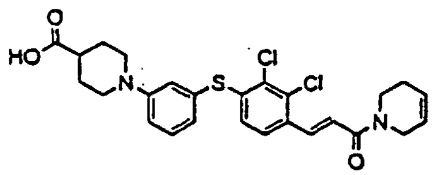

Example 396

[3-(4-Carboxylpiperidin-1-yl)phenyl][2,3-dichloro-4-(*E*-[(1,2,3,6-tetrahydropyridine)-1-yl)carbonyl]ethyl)phenyl]sulfide

The title compound (22 mg, 58%) was prepared from Example 391A as described in Example 384D, substituting morpholine with ethyl isonipecotate followed by hydrolysis with LiOH as described in Example 340H. $^1$H NMR (400 MHz, DMSO-$d_6$) δ 1.59-1.70 (m, 2H), 1.87-1.93 (m, 2H), 2.07-2.19 (m, 2H), 2.39-2.47 (m, 1H), 2.80-2.90 (m, 2H), 4.03 (br s,1H), 4.16.(br s, 1H), 5.68-5.76 (m, 1H), 5,79-5.90 (m, 1H), 6.72 (d, J=8 Hz, 1H), 6.93 (d. J=7 Hz, 1H), 7.13 (m, 2H), 7.17-7.3 (m, 1H), 7.36 (t, J=7 Hz, 1H), 7.75 (d, J=15 Hz, 1H), 7.80-7.90 (m, 1H); MS (ESI) *m*/*e* 517, 519 $(M+H)^+$.

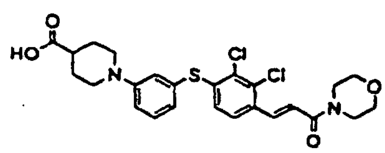

Example 397

[3-(4-Carboxylpiperidinyl)phenyl][ 2,3-dichloro-4-(*E*-[(4-morpholinyl)carbonyl]ethenyl)phenyl]sulfide

The title compound (39 mg, 79%) was prepared from Example 393A as described in Example 384D, substituting morpholine with ethyl isonipecotate followed by hydrolysis with LiOH as described in Example 340H. $^1$H NMR (400 MHz, DMSO-$d_6$) δ 1.56-1.68 (m, 2H), 1.86-1.92 (m, 2H), 2.38-2.46 (m, 1H), 2.77-2.86 (m, 2H, 3.52-3.61 (m, 6H), 3.65-3.72 (m, 4H), 6.71 (d, J=8 Hz, 1H), 6.91 )d, J=7 Hz, 1H), 7.10 (m, 2H), 7.21 (d, J=15 Hz, 1H), 7.34 (t, J=8 Hz, 1H), 7.76 (d, J=15 Hz, 1H), 7.83 (d, J=8 Hz, 1H); MS (ESI) *m*/*e* 521, 523 $(M+H)^+$.

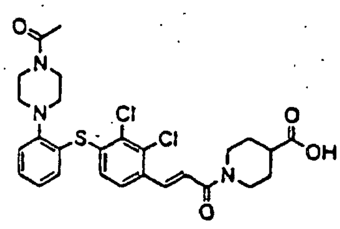

Example 398

[2-(4-Acetylpiperazin-1-yl)phenyl] [2,3-dichloro-4-(*E*-[(4-carboxypiperidin-1-yl)carbonyl]ethenyl)phenyl] sulfide

The title compound (19 mg, 83%) was prepared from Example 384C as described in Example 384D, substituting morpholine with 4-acetylpiperazine, followed by hydrolysis with LiOH as described in Example 340H. $^1$ H NMR (300 MHz, DMSO-$d_6$) δ 1.38-1.54 (m, 2H), 1.82-11.92 (m, 2M, 2.00 (s, 3 H), 2.51-2.60 (m, 1H), 2.87-3.00 (m, 5H), 3.13-3.27 (m, 1H), 3.36-3.46 (m, 4H), 4.06-4.18 (m, 1H), 4.22-4.36 (m, 1H), 6.91 (d, J=7.5 Hz, 1H), 7.10-7.17 (m, 1H), 7.20-7.25 (m, 2H), 7.28 (d, J=15 Hz, 1H), 7.39-7.45 (m, 1H), 7.77 (d, J=15 Hz, 1H), 7.89 (d, J=7.5 Hz, 1H); MS (ESI) *m*/*e* 562, 564 $(M+H)^+$.

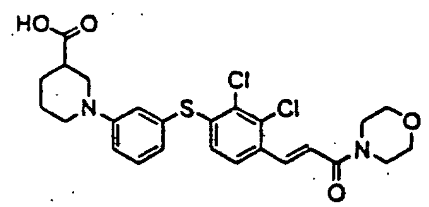

Example 399

[3-(3-Carboxypiperidin-1-yl)phenyl] [2,3-dichloro-4-(*E*-[(4-morpholinyl)carbonyl)ethenyl)phenyl] sulfide

The title compound (30 mg, 60%) was prepared from Example 393A as described in Example 384D, substituting morpholine with ethyl nipecotate followed by hydrolysis with LiOH as described in Example 340H. $^1$H NMR (400 MHz, DMSO-$d_6$) δ 1.51-1.60 (m, 1H), 1.66-1.72 (m, 1H), 1.87-1.94 (m, 1H), 2.79-2.87 (m, 1H), 2.96-3.02 (m, 1H), 3.44-3.72 (m, 12H), 6.71 (d, J=8 Hz, 1H), 6.90 (d, J=7 Hz, 1 H), 7.09 (m,2H), 7.21 (d, J=15 Hz, 1H), 7.32-7.38 (m, 1H), 7.76 (d, J=15 Hz, 1H), 7.84 (d. J=8 Hz, 1H); MS (ESI) *m*/*e* 521, 523 $(M+H)^+$.

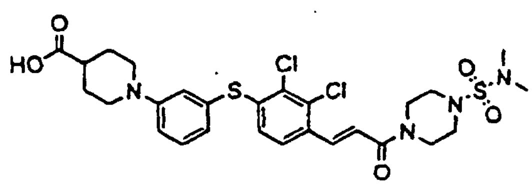

Example 400

[3-(4-Carboxypiperidin-1-yl)phenyl)[2,3-dichloro-4-(*E*-[(4-(dimethylaminosulfamoyl)piperazin-1-yl)carbonyl]ethenyl) phenyl]sulfide

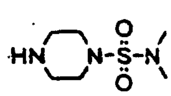

Example 400A

N,N-Dimethyl piperazinylsulfamide

To a solution of *tert*-butyl 1 -piperazinecarboxylate (2.5 g. 13.42 mmol) in tetrahydrofuran (21.5 ml, 0.25 M) at 0 °C was added triethylamine (2.25 mL, 16.11 mmol) followed by dimethylsulfamoyl chloride (1.73 mL, 16.11 mmol). The reaction mixture was stirred at 0 °C for 1h, diluted with ethyl acetate (100 mL) and washed with saturated $NaHCO_3$ solution (2x30 mL), followed by brine (2x30 mL). The dried ($Na_2SO_4$,) organic layer was evaporated to dryness under reduced pressure and the residue obtained was treated with 10% trifluoroacetic acid in methylene chloride (20 mL) at ambient temperature. After 48 h, methylene chloride was evaporated in vacuo to obtain a colorless syrup. This crude material was made basic (1 N NaOH, 50 mL), and the mixture was extracted sequentially with ethyl acetate (2x20 mL) and methylene chloride (2x30 mL). The combined organic layers were dried ($Na_2SO_4$) and evaporated to dryness under reduced pressure to obtain the title compound in quantitative yield. $^1H$ NMR (300 MHz, DMSO-$d_6$) δ 2.77 (s, 3H), 2.79 (s, 3H), 3.12-3.20 (m, 7H), 3.3 (m, 1H), 8.86 (br s, 1H); MS (ESI) *m*/*e* 194 $(M+H)^+$.

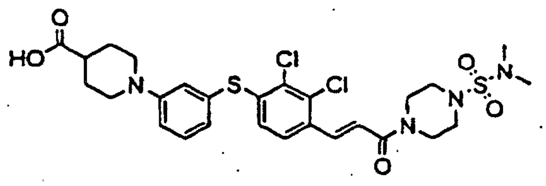

Example 400B

[3-(4-Carboxypiperidin-1-yl)phenyl] [2,3-dichloro-4-(*E*-[(4-(dimethylaminosulfamoyl)piperazin-1-yl)carbonyl]ethenyl) phenyl] sulfide

The title compound was prepared from Example 384B as described in Example 340G, substituting methy isonipecotate with Example 400A, followed by amination with ethyl isonipecotate as described in Example 396. $^1H$ NMR (500 MHz, MeOH-$d_4$) δ 2.79-2.88 (m, 2H), 2.01-2.08 (m, 2H), 2.48-2.53 (m, 1H), 2.84 (s, 6H), 2.91-2.99 (m, 2H), 3.24-3.29 (m, 2H), 3.66-3.73 (m, 2H), 3.77 (m, 6H), 6.80 (d, J=7.5 Hz, 1H), 7.05(d, J=7.5 Hz, 1H), 7.11 (d, J=15 Hz, 1H), 7.16-7.22 (m, 2H), 7.39 (t, J=7.5 Hz, 1H), 7.62 (d, J=7.5 Hz, 1H), 7.95 (d, J= 15 Hz, 1H); MS (ESI) *m*/*e* 625, 627 $(M-H)^-$.

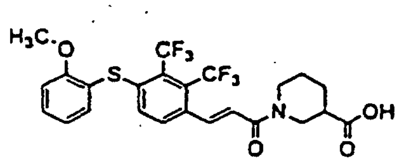

Example 401

(2-Methoxyphenyl)[2,3-bis(trifluoromethyl)-4-(E-((3-carboxypiperidin-1-yl)carbonyl)ethenyl)phenyl]sulfide

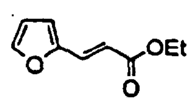

Example 401 A

Ethyl 2-Furylacrylate

Ethyl iodide (64 mL, 0.796 mol) was added to furylacrylic acid (100 g, 0.724 mol), diisopropylethyl amine (140 mL, 0.796 mmol), in acetonitrile (1100 mL), and the mixture was heated to 60 °C. After 18 h, the dark solution was cooled to room temperature and concentrated in vacuo. The resulting brown sludge was diluted with $Et_2O$ (500 mL), washed with 1N aqueous HCl (2x250 mL), washed with 0.2 N aqueous NaOH (2x250 mL), washed with saturated aqueous $NaHCO_3$ (1x250 mL), dried ($MgSO_4$), filtered, and concentrated to a black oil (114 g, 95%). $^1H$ NMR (DMSO-$d_6$, 300 MHz) δ 7.84 (d, *J*= 1.7 Hz, 1H), 7.46 (d, *J*=15.6 Hz, 1H), 6.97 (d, *J*=3.4 Hz, 1 H), 6.33 (dd, *J=3.4* Hz, *J*=1.7 Hz, 1 H), 6.22 (d, *J= 15.9* Hz, 1 H), 4.17 (q, *J*=7.1 Hz, 2H), 1.24 (t, *J*=7.1 Hz, 3H); MS (APCI) *m/z* 167 $(M+H)^+$.

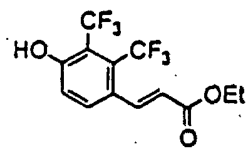

Example 401B

Ethyl *E*-2,3-bis(trifluoromethyl)-4-hydroxycinnamate

A solution of Example 401 A (20 g, 0.12 mol) in tetrahydrofuran (40 mL) at - 50 °C in a 600 mL Parr stirred reactor was treated with hexafluoroacetylene (24.4 g, 0.15 mol), the reactor sealed and heated to 110 °C for 22 hours, allowed to slowly cool to room temperature, and then concentrated to a brown oil (36 g). This oil was then treated with boron trifluoride etherate (33 mL, 0.275 mol) at room temperature for 17 hours, additional boron trifluoride etherate (16 mL, 0.135 mol) added, stirred six hours, cooled to 0 °C , diethyl ether (200 mL) added, followed by slow addition of 150 mL of 2M potassium carbonate (vigorous gas evolution). This mixture was diluted with additional diethyl ether, layers separated, organic layer washed with brine, dried ($MgSO_4$) and concentrated to give 39 grams of a brown semi-solid. This semi-solid was diluted with 75 mL of dichloromethane and then flash chromatographed on silica gel with 10-50% ethyl acetate/hexane to provide the title compound (22.8 g, 58%). mp 138.140 °C; $^1H$ NMR(300 MHz, d6 DMSO) δ 11.64 (bs, 1H), 7.95 (d, 1H), 7.78 (dq, 1H), 7.33 (d, 1H), 6.47 (d, 1H), 4.21 (q, 2H), 1.26 (t, 3H); MS (APCI-$NH_3$) m/e 329 $(M+H)^+$, 346 $(M+NH_4)^+$, 327 $(M-H)^-$. Analytical HPLC: 4.6X250 mm Zorbax C18 column, 1.5 mL/min, 254 nm, $CH_3CN:H_2O$ with 0.1% TFA, 0:100 ramp to 90:10 (0-10 min), 90:10 (10-18 min), ramp to 0:100 (18-20 min), Rt = 10.6 min (98.3 area%).

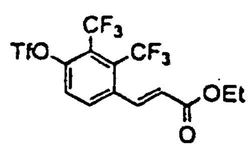

Example 401C

Ethyl *E*-4-(trifluoromethanesulfonyl)-2,3-*bis*(trifluoromethyl)cinnamate

Triflic anhydride (670 µL, 3.97 mmol) was added to a mixture of Example 401B (1.00 g, 3.05 mmol) and pyridine (6.5 mL). After 2 h, the dark solution was diluted with $Et_2O$ (75 mL), washed with 1N aqueous HCl (2x50 mL), washed with saturated aqueous $NaHCO_3$ (1x75 mL), dried ($MgSO_4$), filtered, and concentrated to a dark amber oil (1.35 g, 96%). $^1H$ NMR (DMSO-$d_6$, 300 MHz) δ 8.33 (d, *J*=8.8Hz, 1H), 8.11 (d, *J*=8.8 Hz, 1H), 7.87-7.78 (m, 1H), 6.67 (d, *J*=16.0 Hz, 1H), 4.24 (q, *J*=7.1 Hz, 2H), 1.27 (t, *J*=7.1 Hz, 3H); MS (APCI) *m/z* 478 $(M+NH_4)^+$, 495 $(M+Cl)^-$.

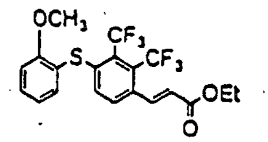

Example 401D

(2-Methoxyphenyl) [2,3-bis(trifluoromethyl)-4-(*E*-(ethoxycarbonyl)ethenyl)phenyl]sulfide

2-Methoxythiophenol (524 µL, 4.30 mmol) was added to Example 401C (1.69 g, 3.90 mmol), cesium carbonate (3.18 g, 9.75 mmol), and DMF (8 mL). After 15h, the dark solution was diluted with $Et_2O$ (100 mL), washed with water (1x50 mL), washed with 1 N aqueous HCl (2x100 mL), washed with saturated aqueous $NaHCO_3$ (1x100 mL), dried ($MgSO_4$), filtered, and concentrated to a dark oil. Flash silica gel column chromatography (85:15 hexane:ethyl acetate) provided the ethyl ester (1.16 g, 66%) as a yellow oil. The ester (858 mg) was subsequently hydrolyzed as previously detailed in Example 155 to provide the title compound (670 mg, 84%) as a white solid. $^1H$ NMR (DMSO-$d_6$, 300 MHz) δ 7.89 (d, J=8.8 Hz, 1H), 7.74-7.67 (m, 1 H), 7.55 (dd, *J*=7.5 Hz, *J*=1.7 Hz, 1H), 7.50 (dd, *J*=9.9 Hz, *J*=1.7 Hz, 1H), 7.20 (d, *J*=8.4 Hz, 1H), 7.19 (t, *J*=7.1 Hz, 1H), 7.07 (dt, *J*=7.5 Hz, *J*=1.3 Hz, 1H), 6.44 (d, *J*=15.6 Hz, 1H), 3.75 (s, 3H). MS (APCI) *m/z* 421 (M-H$^+$).

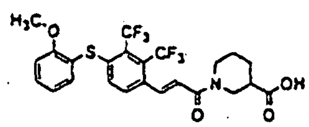

Example 401 E

(2-Methoxyphenyl) [2,3-bis(trifluoromethyl]-4-(E-((3-carboxypiperidin-1-yl)carbonyl ethenyl)phenyl)sulfide

Example 401D was processed as detailed in Examples 137 and 155 to provide the title compound (168 mg, 86%) as a white solid. $^1H$ NMR (DMSO-$d_6$, 300 MHz) δ 7.95 (d, 1H), 7.57 (m, 1H),7.50(t, 1H), 7.46 (d, 1H), 7.20 (d, 1H), 7.15(d,1H), 7.14 (d, 1H), 7.06 (t, 1H), 4.4 (m, 1H), 4.01 (m, 2H), 3.75 (s, 3H), 1.93 (m, 2H), 1.63 (m, 2H), 1.42 (m, 2H), MS (APCI) *m/z* 534 (M+H$^+$). Anal. calcd for $C_{24}H_{21}F_6NO_4S$+0.75M $H_2O$: C, 52.70; H, 4.15; N, 2.56. Found: C, 53.01; H, 3.78; N, 2.17.

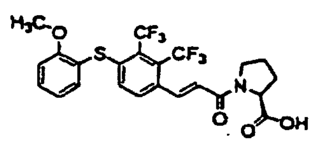

Example 402

(2-Methoxyphenyl) [2,3-bis(trifluoromethyl)-4-(E-((2-carboxypyrrolidin-1-yl)carbonyl)ethenyl)phenyl]sulfide

Example 401D was processed as reported in Example 401E, substituting L-proline methyl ester hydrochloride for the amine. $^1H$ NMR (DMSO-$d_6$, 300 MHz) δ 7.98 (d, *J*=8.2 Hz, 1H), 7.64 (m, 1H, 7.53 (d, *J*=8.2 Hz, 1H), 7.50 (t, *J*=7.4 Hz, 1H), 7.21 (d, 1H), 7.19 (d, 1H), 7.07 (t, *J*=7.8 Hz, 1H), 6.95 (d, *J*=15.0 Hz, 1H), 4.34 (m, 1H), 3.70 (m, 2H), 3.76 (s, 3H), 2.08 (m, 2H), 1.91 (m, 2H); MS (APCI) *m/z* 520. (M+H$^+$). Anal. calcd for $C_{23}H_{19}F_6NO_4S$: C, 53.18; H, 3.69; N, 2.70. Found: C, 52.88; H, 3.86; N, 2.43.

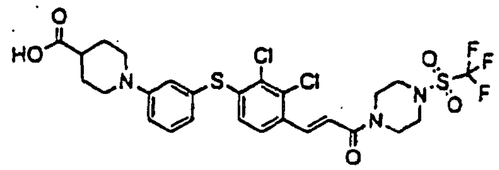

Example 403

[3-(4-Carboxypiperidin-1-yl)phenyl] [2,3-dichloro-4-(*E*((4-((trifluoromethylsulfonyl)piperazin-1-yl)carbonyl)ethenyl)phenyl]sulfide

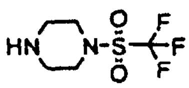

Example 403 A

Piperazine-1-trifluoromethylsulfonamide

The title compound (1.65 g, 72%) was prepared as described in Example 400A, substituting dimethylsulfamoyl chloride with trifluoromethanesulfonyl chloride (1.26 ml, 11.81 mmol). MS (ESI) *m*/*e* 219 $(M+H)^+$.

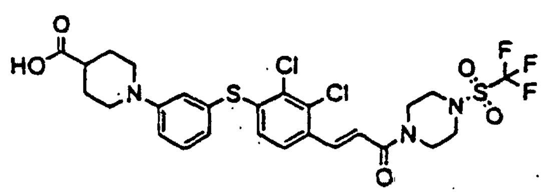

Example 403B

[3-(4-Carboxypiperidin-1-yl)phenyl] [2,3-dichloro-4-(*E*-((4-((trifluoromethylsulfonyl)piperazin-1-yl)carbonyl)ethenyl)phenyl] sulfide

Example 403B (51 mg, 38%) was prepared from Example 384B as described in Example 340G, substituting methy isonipecotate with Example 403A followed by amination with ethyl isonipecotate as described in Example 396. $^1H$ NMR (500 MHz, DMSO-$d_6$) δ 1.56-1.66 (m, 2H), 2.84-2.91 (m, 2H), 2.37-2.45 (m, 1 H), 2.77-2.86 (m, 2H), 3.63-3.70 ) m, 7H), 3.72-3.85 (m, 3H), 6.72 (d, J=8.75 Hz, 1H), 6.90 (d, J=7.5 Hz, 1H), 7.09 (m, 1H), 7.11 (s, 1H), 7.21 (d, J=15 Hz, 1H), 7.34 (t, J=7.5 Hz, 1H), 7.76 (d, J=15 Hz, 1 H), 7.81 (d, J=8.75 Hz, 1H); MS (ESI) *m*/*e* 650, 652 $(M-H)^-$.

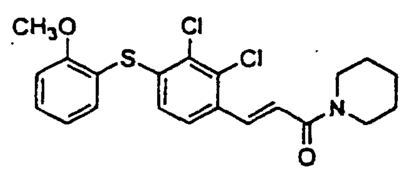

Example 404

(2-Methoxyphenyl)[2,3-dichloro-4-(*E*-(piperidin-1-ylcarbonyl)ethenyl)phenyl]sulfide

The title compound was prepared by the procedures described in Example 1C substituting Example 1B with (2-methoxy) [2,3-dichloro-4-( *E*-(2-carboxyethenyl)phenyl] sulfide and substituting 6-amino-1-hexanol with piperidine. $^1$H NMR (300 MHz, DMSO-$d_6$) δ 1.48 (m, 4H), 1.59 (m, 2H), 3.55 (m, 4H), 3.79 (s, 3H), 6.55 (d, J = 8.4 Hz, 1H), 7.08 (t, J = 7.4 Hz, 1H), 7.21 (d, J = 6.0 Hz, 1 H), 7.25 (s, 1 H), 7.48 (dd, J = 7.8, 1.7 Hz, 1H), 7.56 (m, 1 H), 7.72 (d, J = 15.6 Hz, 1H), 7.82 (d, J =8.5 Hz, 1H); MS (ESI$^+$) *m/z* 422, 424 (M+H)$^+$. Anal. calcd for $C_{21}H_{21}NCl_2SO_2$: C, 59.72; H, 5.01; N, 3.32. Found: C, 59.52; H. 4.94; N, 3.05.

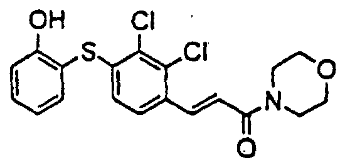

Example 405

(2-Hydroxyphenyl) [2,3-dichloro-4-(E-((4,-morpholino)carbonyl)ethenyl)phenyl]sulfide

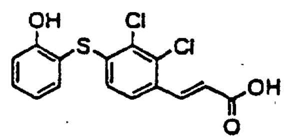

Example 405A

(2-Hydroxyphenyl) [2,3-dichloro-4-(E-(carboxy)ethenyl)phenyl]sulfide

Boron tribromide (84 mL of a 1.0M solution in $CH_2Cl_2$) was added to a suspension of Example 310C in $CH_2Cl_2$ (85 mL) at 0 °C. After addition was completed, the ice-water bath was removed, and the homogeneous dark solution was stirred for 2h before the mixture was poured into 1 N aqueous HCl (100 mL) and ice (100 g), and extracted with EtOAc (3x100 mL). The organic layers were combined, washed with brine (1x50 mL), dried ($MgSO_4$), filtered, and concentrated to a white solid (11.3 g). $^1$H NMR (DMSO-$d_6$, 300 MHz) δ 10.26 (s, 1H), 7.82 (d, *J*=15.6, 1H), 7.74 (d, *J*=8.5 Hz, 1H), 7.44 (dt, J=7.8 Hz, *J*=1.7 Hz, 1H), 7.41 (dd, *J*=7.4 Hz, *J*=1.7 Hz, 1H), 7.05 (dd, J=8.4 Hz, J=1.3 Hz, 1H), 6.94 (dt, *J*=7.8 Hz, *J*=1.4 Hz, 1H), 6.52 (d, *J*=8.2 Hz, 1H), 6.50 (d, *J*=16.0Hz, 1H); MS (APCI) *m/z* 339 (M-H)$^-$, 375 (M+Cl)$^-$.

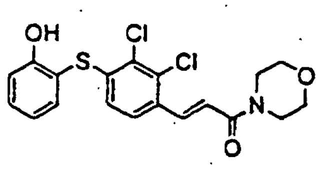

Example 405B

(2-Hydroxyphenyl) [2,3-dichloro-4-(E-((4-morpholino)carbonyl)ethenyl)phenyl]sulfide

Example 405A (11.3 g) was processed as reported in Example 310D to provide the title product (8.47 g, 62%) as a white solid. $^1$H NMR (DMSO-$d_6$, 300 MHz) δ 10.24 (s, 1H), 7.81 (d, *J*=8.9, 1H), 7.77 (d, *J*=14.9 Hz, 1H), 7.44 (dt,

*J*=6.4 Hz, *J*=1.7 Hz, 1H), 7.39 (dd, *J*=8.2 Hz, *J*=1.7 Hz, 1H), 7.05 (dd, *J*=8.1 Hz, *J*=1.0 Hz, 1H), 6.94 (dt, *J*=7.8 Hz, *J*=1.0 Hz, 1H), 6.52 (d, *J*=8.8 Hz, 1H), 6.53 (d, *J*=8.8 Hz, 1H); MS (APCI) *m/z* 410 $(M+H)^+$, 446 $(M+Cl)^+$.

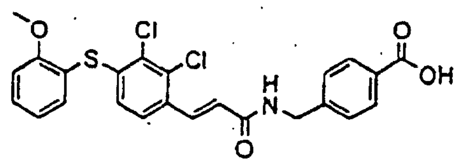

Example 406

(2-Methoxyphenyl) [2,3-dichloro-4-(E-((((4-carboxyphenyl)methyl)amino)carbonyl)ethenyl)sulfide

The title compound was prepared by the procedures described in Example 1C substituting Example 1B with (2-methoxy) [2,3-dichloro-4-( *E*-(2-carboxyethenyl)phenyl] sulfide and substituting 6-amino-1-hexanol with methyl 4-(aminomethyl)benzoate hydrochloride following by hydrolysis. $^1H$ NMR (300 MHz. DMSO-$d_6$) δ3.79 (s, 3H), 4.46 (s, 2H), 6.60 (d, J = 8.1 Hz. 1H), 6.66 (d, J = 15.6 Hz, 1H), 7.08 (t, J =8.4 Hz. 1H), 7.25 (d, J = 8.5 Hz. 1H), 7.39 (d, J = 8.5 Hz 2H), 7.51 (m, 3H), 7.75 (d. J = 15.6 Hz, 1 H), 7.90 (d, J = 8.4 Hz, 2H), 8.83 (t, 3 = 5.7 Hz, 1H), 12.90 (brs, 1H); MS ($ESI^+$) *m/z* 488,490 $(M+H)^+$. Anal. calcd for $C_{24}H_{19}NCl_2O_4S$: C, 59.02; H, 3.92; N, 2.87. Found: C, 58.97; H, 4.07; N, 2.71.

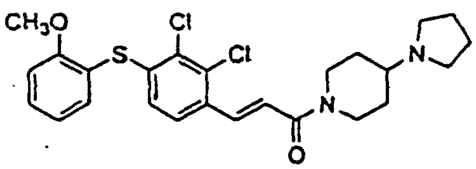

Example 407

(2-Methoxyphenyl) [2,3-dichloro-4-(E-(((4-pyrrolidin-1-yl)piperidin-1-yl)carbonyl)ethenyl)phenyl]sulfide

The title compound was prepared by the procedures described in Example 1C substituting Example 1B with (2-methoxy) [2,3-dichloro-4-(*E*-(2-carboxyethenyl)phenyl] sulfide and substituting 6-amino-1-hexanol with 4-(pyrrolidinyl)piperidine. $^1H$ NMR (300 MHz, DMSO-$d_6$) δ 1.48 (m, 2H), 1.84 (m, 2H), 2.00 (m, 2H), 2.10 (m, 2H), 2.65 (m, 1H), 3.10 (m, 3H), 3.35 (m, 1H), 3.50 (m, 1H), 3.80 (s, 3H), 4.38 (m, 2H), 4.52 (m, I H), 6.56 (d, J = 8.5 Hz, 1H), 7.08 (t, J = 7.8 Hz, I H), 7.22 (d, J = 7.8 Hz, 1H), 7.26 (d, J = 15.2 Hz, 1H), 7.48 (dd, J = 7.8, 1.7 Hz, 1H), 7.57 (t, J = 8.2 Hz, 1H), 7.76 (d, J = 15.3 Hz, 1H), 7.82 (d, J = 7.8 Hz, 1H); MS ($ESI^+$) *m/z* 491, 493 $(M+H)^+$. Anal. calcd for $C_{25}H_{28}N_2Cl_2O_2S$ 1.8 TFA: C, 49.30; H, 4.31; N, 4.02. Found: C, 49.08; H, 4.31; N, 3.97.

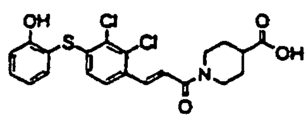

Example 408

(2-Hydroxyphenyl)[2,3-dichloro-4-(E-((4- carboxypiperidin-1-yl)carbonyl)ethenyl phenyl]sulfide

Example 405A (119 mg) was processed as detailed in Example 165 to provide the title compound as a white solid (43 mg, 28%). $^1H$ NMR (DMSO-$d_6$, 300 MHz) δ 10.23 (s, 1H), 7.81 (d, *J*=8.8, 1 H), 7.72 (d, *J*=15.2 Hz, 1H), 7.42 (dt, *J*=7.8 Hz, *J*=1.7 Hz, 1H), 7.39 (dd, *J*=7.1 Hz, *J*=1.7 Hz, 1H), 7.21.(d, *J*=15.3 Hz, 1H), 7.05 (dd, *J*=8.2 Hz, *J*= 1.0 Hz, 1H), 6.93 (dt, *J*=7.4 Hz, *J*=1.0 Hz, 1H), 6.53 (d, *J*=8.5 Hz, 1H), 4.25 (m, 1H), 4.03 (m, 2H), 2.85 (m, 2H), 1.87 (m, 2H), 1.44 (m, 2H). MS (APCI) *m/z* 452 $(M+H^+)$, 450 $(M-H^+)$, 486 $(M+Cl^-)$. Anal. calcd for $C_{21}H_{19}Cl_2NO_4S$+0.25M $H_2O$:

C, 55.21; H, 4.30; N, 3.07. Found: C, 55.26; H, 4.29; N, 2.72.

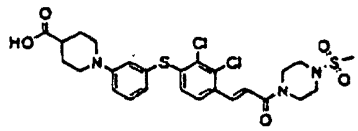

Example 409

[3-(4-Carboxypiperidin-1-yl)phenyl][2,3-dichloro-4-(*E*-((4-((methylsulfonyl)piperazin-1-yl)carbonyl)ethenyl),phenyl] sulfide

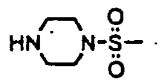

Example 409A

Piperazine methylsulfonamide

The title compound (1.65 g, 72%) was prepared as described in Example 400A, substituting dimethylsulfamoyl chloride with methanesulfonyl chloride (1.26 ml, 11.81 mmol),

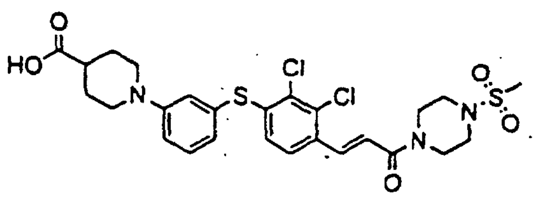

Example 409B

[3-(4-Carboxypiperidin-1-yl)phenyl] [2,3-dichloro-4-(*E*-((4-((methylsulfonyl)piperazin-1-yl)carbonyl)ethenyl)phenyl] sulfide

Example 409B (48 mg, 72%) was prepared from Example 384B as described in Example 340G, substituting methy isonipecotate with Example 409A followed by amination with ethyl isonipecotate as described in Example 396. $^1$H NMR (300 MHz, DMSO-$d_6$) δ 1.55-1.71 (m, 2H), 1.83-1.94 (m, 2H), 2.36-2.48 (m, 1H), 2.77-2.86 (m, 2H), 2.88 (s, 3H), 3.10-3.18 (m, 4H), 3.66-3.84.(m, 6H), 6.73 (d, J=8 Hz, 1H), 6.93 (d, J=7.5 Hz, 1H), 7.11 (m, 1H), 7.13 (s, 1H), 7.25 (d, J=15 Hz, 1H), 7.32-7.41 (m, 1H), 7.78 (d, J=15 Hz, 1H), 7.85 (d, J=8 Hz, 1 H); MS (ESI) *m/e* 598, 600 $(M+H)^+$; 596, 598 $(M-H)^-$.

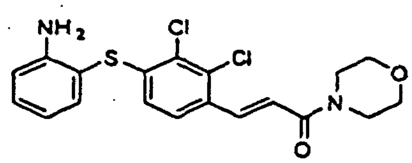

Example 410

(2-Aminophenyl) [2,3-dichloro-4-(*E*-((4-morpholinyl)carbonyl)ethenyl)phenyl sulfide

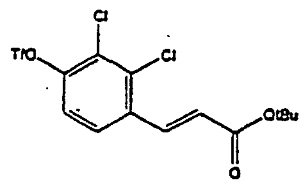

Example 410A

*tert*-Butyl 2,3-dichloro-4-((trifluoromethyl)sulfonyloxy)cinnamate

The title compound was constructed according to the procedure for Example 340D and 340E, except using *tert*-butyl acrylate instead of methyl acrylate. $^1H$ NMR (300 MHz, DMSO-$d_6$) δ 8.11(d, 1H), 7.78 (d, 1H), 7.72 (d, 1H), 6.72 (d, 1H), 1.5 (s, 9H); MS (APCI-$NH_3$) *m/e* 456 $(M+Cl)^-$.

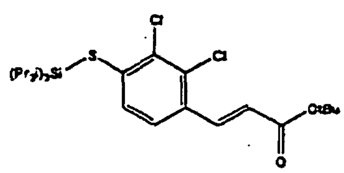

Example 410B

*tert*-Butyl 2,3-dichloro-4-((triisopropylsilyl)thio)cinnamate

Sodium hydride (3.05 g of 60% dispersion, 76 mmol) that had been rinsed with dry tetrahydrofuran (2x), was suspended in 128 mL of THF, cooled to -5°C, and slowly treated with triisopropylsilyl thiol (12.2 mL, 57 mmol), maintaining an internal temperature below 4 °C, stirred at 0 °C for 1.5 h, then added to a second flask containing Example 410A (20 g, 47.4 mmol) and tetrakistriphenylphosphine palladium (4.4 g, 3.8 mmol) in 95 mL of THF. The reaction was heated at reflux for 8 h, then allowed to cool to ambient temperature and concentrated. The resultant slurry was diluted with ethyl acetate, filtered through celite, washed with brine, dried ($Na_2SO_4$) and concentrated. The resultant black residue was flash chromatographed on silica gel with 2.5-5% acetone/hexane to provide the title compound (18.2 g, 83%). $^1H$ NMR (300 MHz, DMSO-$d_6$) δ 7.82 (d, 1H), 7.78 (d, 1H), 7.0 (d, 1H), 6.5 (d, 1 H), 1.5 (s, 9H), 1.35 (m, 3H), 1.09 (d, 18H); MS (APCI-$NH_3$) *m/e* 462 $(M+H)^+$.

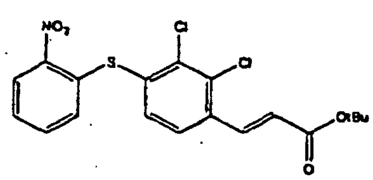

Example 410C

(2-Nitrophenyl) [2,3-dichloro-4-(*E*-((*tert*-butyloxycarbonyl)ethenyl)phenyl] sulfide

A solution of Example 410B in toluene (40 mL) was treated with cesium fluoride (600 mg, 4 mmol) followed

by 2-fluoronitrobenzene (5.03 mL, 47.4 mmol), then heated at reflux for 2 h, then allowed to cool and the mixture was concentrated under reduced pressure. The resultant dark brown slurry was diluted with ethyl acetate, washed with water (2x), 1 M NaOH,(2x), water (2x), dried ($Na_2SO_4$) and concentrated. The 21.2 grams of crude product was flash chromatographed on silica gel with 10% acetone/hexane to provide the title compound (8.92 g, 53%). $^1$H NMR (300 MHz, DMSO-$d_6$) δ 8.17 (dd, 1H),. 7.95 (d, 1H), 7.83 (d, 1H), 7.78 (m, 1H), 7.48 (m, 1H), 7.3 (dd, 1H), 7.17 (d, 1H), 6.6 (d, 1H), 1.5 (s, 3H); MS (APCI-$NH_3$) *m/e* 427 (M+H)$^+$.

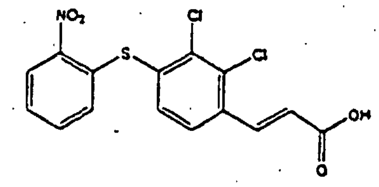

Example 410D

(2-Nitrophenyl) [2,3-dichloro-4-(*E*-((carboxy)ethenyl)phenyl] sulfide

A solution of Example 410C (3.2 g, 7.5 mmol) in dichloromethane (12 mL) at room temperature was treated with trifluoroacetic acid (4 mL), stirred 30 minutes, and concentrated to give the title compound (2.8 g, 100%) as an off-white solid. $^1$H NMR (300 MHz, DMSO-$d_6$) δ 8.16 (dd, 1H), 7.94 (d, 1H), 7.86 (d, 1H), 7.76 (m, 1H), 7.48 (m, 1H), 7.29 (dd, 1H), 7.11 (d, 1H), 6.61 (d, 1H); MS (APCI-$NH_3$) *m/e* 371 (M+H)$^+$.

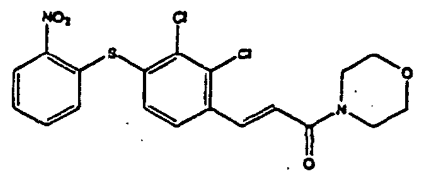

Example 410E

(2-Nitrophenyl) [2,3-dichloro-4-(*E*-((4-morpholinyl)carbonyl)ethenyl)phenyl] sulfide

A solution of Example 410D (2.7 g, 7.29 mmol) in dimethylformamide (32 mL) was treated with hydroxybenzotriazole hydrate (1.2 g, 8.0 mmol), morpholine (1.4 mL, 16 mmol) and then 1-(3-dimthylaniinopropyl)-3-ethylcarbodiimide hydrochloride (1.53 g, 8.0 mmol), stirred at room temperature for 64 hours. The heterogeneous mixture was filtered, the white solid washed with water, and then dried in a vacuum oven at 50 °C for 24 hours to provide 2.8 g (88%) of the title compound as a white powder. mp 210-213 °C; $^1$H NMR (300 MHz, d6 DMSO) δ 8.15 (dd, 1H), 8.03 (d, 1H), 7.82 (d, 1H), 7.74 (m, 1H), 7.45 (m, 1H), 7.32 (d, 1H), 7.2 (m, 2H), 3.7 (m, 2H), 3.6 (m, 6H); MS (APCI-$NH_3$) m/e 440 (M+H)$^+$.

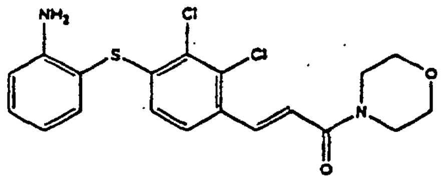

Example 410F

(2-Aminophenyl) [2,3-dichloro-4-(*E*-((4-morpholinyl)carbonyl)ethenyl)phenyl] sulfide

A solution of iron powder (1.3 g, 22.8 mmol) and ammonium chloride (292 mg, 5.46 mmol) in ethanol (9 mL) and distilled water (9 mL) at 105 °C was treated with example 410F (2 g, 4.55 mmol) in ethanol (20 mL), stirred for

one hour and then allowed to cool to room temperature. The resultant heterogeneous black mixture was filtered through a plug of Celite, rinsed through with ethyl acetate (100 mL), the filtrate washed with 1 M potassium carbonate, brine, dried ($Na_2SO_4$) and concentrated to give 1.9 g (100%) of the title compound as an off-white powder. mp 230-240 °C (dec); $^1$H NMR (300 MHz, d6 DMSO) δ 7.9 (d, 1H), 7.8 (d, 1H), 7.2 (d, 1H), 6.95 (dt, 1 H), 6.84 (m, 2H), 6.68 (d, 1H), 6.58 (dt, 1H), 5.05 (bs, 2H), 3.7 (m, 2H), 3.6 (m, 6H); MS (APCI-$NH_3$) m/e 410 $(M+H)^+$; Analytical HPLC: 4.6X250 mm Zorbax C18 column, 1.5 mL/min, 254 nm, $CH_3$ CN:$H_2O$ with 0. 1 % TFA, 0: 100 ramp to 90:10 (0-10 min), 90:10 (10-18 min), ramp to 0:100 (18-20 min), Rt = 9.2 min.

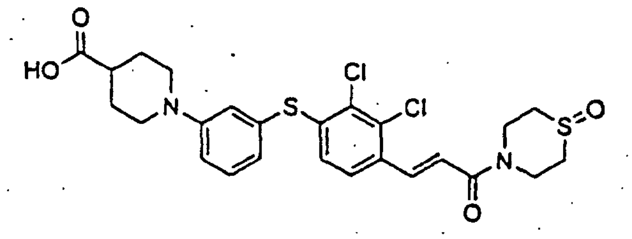

Example 411

(3-(4-carboxypiperidin-1-yl)phenyl)[2,3-dichloro-4-(*E*-((S-oxothiomorpholin-1-yl)carbonyl)ethenyl)phenyl]sulfide

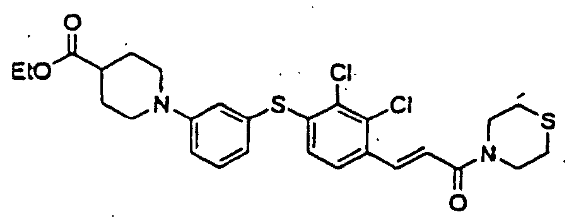

Example 411A

[3-(4-Ethoxycarbonyl)piperidin-1-yl)phenyl] [2,3-dichloro-4-(*E*-(thiomorpholinylcarbonyl)ethenyl)phenyl] sulfide

The title compound was prepared by the procedures described in Example 397B substituting morpholine with thiomorpholine. MS ($APCI^+$) *mlz* 565, 567 $(M+H)^+$.

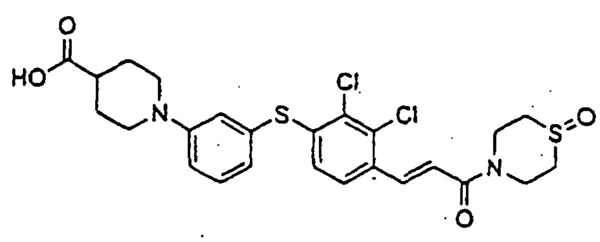

Example 411 B

(3-(4-carboxypiperidin-1-yl)phenyl)[2,3-dichloro-4-(*E*-((S-oxothiomorpholin-1-yl)carbonyl)ethenyl)phenyl] sulfide

To a solution of Example 411A (107 mg, 0.189 mmot) in $CH_2Cl_2$ (6 mL) was added mCPBA (80%, 41 mg, 0.189 mmol) at 0 °C. After stirring at the same temperature for 2 h, THF (2 mL) was added. The solution was concentrated to 1 mL, and was diluted with THF to 3 mL. Lithium hydroxide monohydrate (24 mg) in water (1 mL) was then added. The mixture was stirred at room temperature for 3 hours. The formed transparent solution was separated by

HPLC (Zorbax C-1 8) to give the title compound (68 mg). $^1$H NMR (300 MHz, DMSO-$d_6$) δ 1.64 (m, 2H), 1.90 (m, 2H), 2.41 (m, 1H), 2.86 (m, 4H), 3.62 (m, 2H), 3.95 (m, 1H), 4.18 (m, I H), 4.3 (m, 4H), 6.71 (d, J = 8.4 Hz, 1H), 6.93 (d, J = 7.5 Hz, 1H), 7.12 (d, J = 7.5 Hz, 1H), 7.13 (s, 1H), 7.28 (d, J = 15.3 Hz, 1H), 7.36 (t, J = 8.8 Hz, 1H), 7.80 (d, J = 15.3 Hz, 1H), 7.88 (d, J = 8.8 Hz, 1H); MS (APCI$^+$) *m/z* 553,555 (M+H)$^+$. Anal. calcd for $C_{25}H_{26}N_2Cl_2S_2O_4$2 TFA: C, 44.57; H, 3.61; N, 3.58. Found: C, 44.34; H, 3.76; N, 3.51.

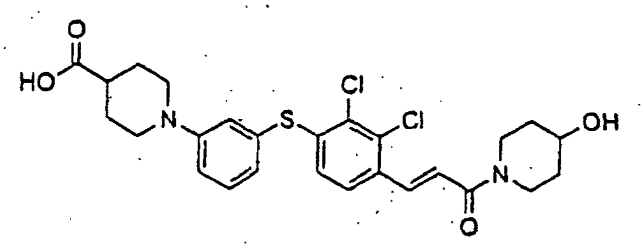

Example 412

[3-(4-Carboxypiperidin-1-yl)phenyl][2,3-dichloro-4-(*E-((*4-hydroxypiperidin-1-yl)carbonyl)ethenyl)phenyl] sulfide

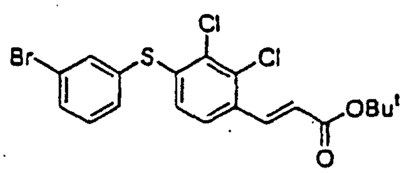

Example 412A

(3-Bromophenyl [2,3-dichloro-4-(*E*-((*tert*-butytoxycarbonyl)ethenyl)phenyl] sulfide

To a solution of Example 384B (2.35 g, 5.82 mmol) in THF (23 mL) at 5 °C was added *tert*-butyl trichloroacetimidate (2.6 mL, 14.54 mmol) and boron trifluoride-etherate (2.35 mL, 18.54 mmol). The solution was stirred at the same temperature for 10 minutes, and was then warmed up to room temperature for 5 h. The yellow solution was poured into aq. $NaHCO_3$ solution, and the mixture was extracted with ethyl acetate. The combined organic phases were washed with water, dried over anhydrous $MgSO_4$, and concentrated. The residual white solid was dissolved in $CH_2Cl_2$ and was precipitated by adding hexane. The formed suspension was filtered through silica gel, and washed with 1:8 EtOAc/hexane. The solution was concentrated and was further purified by flash chromatography (silica gel, 1:20 EtOAc/hexane) to give the title compound (2.50g, 94%). MS (APCI$^+$) m/z 461 (M+H)$^+$.

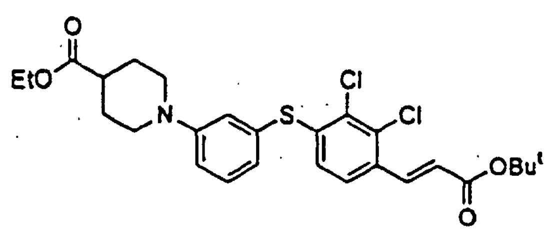

Example 412B

(3-(4-carboethoxypiperidin-1-yl)phenyl)[2,3-dichloro-4(*E*-(carbo-t-butoxyethenyl)phenyl] sulfide

A pressure tube was charged with Example 412A (589 mg, 1.28 mmol), $Pd_2(dba)_3$ (30 mg, 0.032 mmol), 2-dicyclohexylphosphanyl-2'-dimethylaminobiphenyl (26 mg, 0.064 mmol), and anhydrous $K_3PO_4$, (382 mg, 1.8 mmol),

and was purged with nitrogen. DME (4 mL) and ethyl isonipecotate (242 mg, 1.54 mmol) were added via syringe, and the mixture was purged with nitrogen again. The red reaction mixture was stirred at room temperature for 0.5 h and at 95 °C for 15 h. After the reaction mixture was cooled, the it was diluted with ethyl acetate, and washed with brine. The aqueous phase was extracted with ethyl acetate. The combined ethyl acetate solution was concentrated and the residual oil was separated by flash chromatography (silica gel, 1:6 EtOAc/hexane) to give the tide compound (523 mg, 76%). MS ($APCI^+$) $m/z$ 536 $(M+H)^+$.

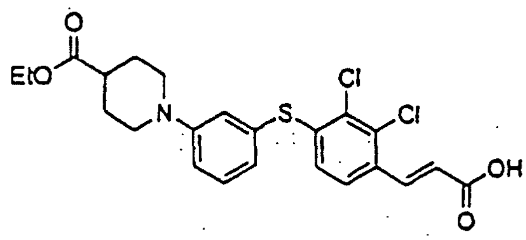

Example 412C

[3-(4-(Ethoxycarbonyl)piperidin-1-yl]phenyl] [2,3-dichloro-4-(*E*-(carboxy)ethenyl)phenyl] sulfide

To a solution of Example 412B (510 mg, 0.95 mmol) in $CH_2Cl_2$ (8 mL) at 0 °C was added trifluoroacetic acid (1.6 mL). The yellow solution was stirred at 0 °C for I h, and was warmed to room temperature for 3 h. After diluting with $CH_2Cl_2$, the solution was poured into aq. $NaHCO_3$ solution. The inorganic phase was acidified to pH 5, and was extracted with 10% MeOH in $CH_2Cl_2$. The combined organic phases were washed with water, concentrated under vacuum and dried to give the title compound (472 mg, 100%). MS ($APCI^+$) $m/z$ 480 $(M+H)^+$.

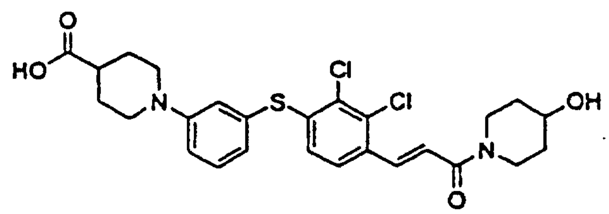

Example 412D

[3-(4-carboxypiperidin-1-yl)phenyl] [2,3-dichloro-4-(*E*-((4-hydroxypiperidin-1-yl)carbonyl)ethenyl)phenyl]sulfide

To a suspension of Example 412C (150 mg, 0.31 mmol) in DMF (3 mL) was added 4-hydroxypiperidine (63 mg, 0.62 mmol), 1-(3-dimethylaminopropyl)-3-ethylcarbodimide (120 mg, 0.62 mmol), HOBt (84 mg, 0.62 mmol) and triethylamine (87 µL, 0.62 mmol) at room temperature. The mixture was stirred at the same temperature for 15 h. Ethyl acetate was added, the mixture was washed with brine, water, and was concentrated. The residual oil was dissolved in THF (3 mL), and was added lithium hydroxide monohydrate (26 mg, 0.62 mmol) in water (1.5 mL). After stirring for 15 hours, the solution was separated by HPLC (Zorbax C-18) to give the title compound (132 mg, 55%). $^1H$ NMR (300 MHz, DMSO-$d_6$) δ 1.32 (m, 2H), 1.65 (m, 2H), 1.75 (m, 2H), 1.92 (m, 2H), 2.43 (m, 1H), 2.86 (t, J = 10.6 Hz, 2H), 3.15 (m, 1H), 3.32 (m, 1H), 3.71 (m, 3H), 3.95 (m, 2H), 6.73 (d, J = 8.5 Hz, 1H), 6.94 (d, J = 7.2 Hz, 1H), 7.13 (d, J = 7.8 Hz, 1H), 7.13 (s, 1H), 7.24 (d, J = 15.2 Hz, 1H), 7.37 (t, J = 8.1 Hz, 1 H), 7.72 (d, J = 15.2 Hz, 1H), 7.85 (d, J = 8.5 Hz, 1H); MS ($ESI^+$) $m/z$ 535, 537 $(M+H)^+$. Anal. calcd for $C_{26}H_{28}N_2Cl_2SO_4$, 0.25 TFA: C, 56.43; H, 5.05; N, 4.97. Found: C, 56.37; H, 5.00; N, 4.91.

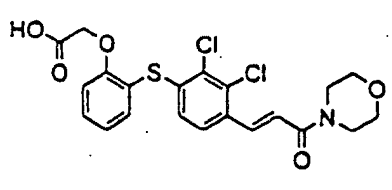

Example 413

(2-Glycoxyphenyl) [2,3-dichloro-4-(E-((4-morpholino)carbonyl)ethenyl]phenyl]sulfide

Diethyl azodicarboxylate (270 μL, 1.47 mmol) was added to a suspension of Example 405 (400 mg, 0.95 mmol), triphenylphosphine (386 mg, 1.47 mmol), and THF (2.0 mL). After 16 h, the dark orange solution was diluted with EtOAc (40 mL), washed with 1 N aqueous HCl (1x20 mL), washed with 0.2 N aqueous NaOH (1x20 mL), washed with brine (1x20 mL), dried ($MgSO_4$), filtered, and concentrated. Flash silica gel column chromatography (9:1 hexane: ethyl acetate) provided a mix of desired ester and triphenyl phosphine oxide. The mixture (200 mg) was combined with lithium hydroxide, monohydrate (34 mg, 0.81 mmol), THF (0.5 mL), and $H_2O$ (0.5 mL). After 21 h, the cloudy solution was diluted with 0.2 N aqueous NaOH (30 mL), washed with $CH_2Cl_2$ (2x15 mL), combined with I N aqueous HCl until pH<2, and extracted with EtOAc (2x20 mL). The EtOAc extracts were combined, washed with brine (1 x20 mL), dried ($MgSO_4$), filtered, and concentrated to a white solid (87 mg, 47%). $^1H$ NMR (DMSO-$d_6$, 300 MHz) δ 7.80 (d, *J*=7.8, 1H), 7.77 (d, *J*=15.3 Hz, 1H), 7.51 (dt, *J*=8.1 Hz, *J*=2.0 Hz, 1H), 7.48 (d, *J*=8.1 Hz, 1H), 7.22 (d, *J*=15.3 Hz, 1H), 7.09 (d, *J*=7.8 Hz, 1H), 7.08 (dt, *J*=7.1 Hz, *J*= 1.0 Hz, 1H), 6.71 (d, *J*=8.9 Hz, 1H), 4.77 (s, 2H), 3.66 (s, 2H), 3.58 (s, 6H); MS (APCI) *m/z* 468 $(M+H)^+$; 466 $(M-,H)^-$ 502 $(M^+Cl)^-$. Anal. calcd for $C_{21}H_{19}Cl_2NO_5S$: C, 53.85; H, 4.09; N, 2.99. Found: C, 54.07; H, 4.28, N, 2.69.

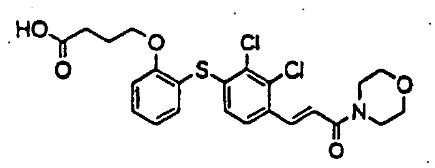

Examnle 414

(2-(4-Butyroxy)phenyl)[2,3-dichloro-4-(E-((4-morpholino)carbonyl)ethenyl)phenyl]sulfide

Ethyl 4-bromobutyrate was added to a mixture of Example 405 (300 mg, 0.731 mmol), cesium carbonate (358 mg, 1.10 mmol), and DMF (1.5 mL). After 16 h, the pale milky solution was diluted with EtOAc (30 mL), washed with 1 N aqueous HCl (2x25 mL), washed with brine (1x25 mL), dried ($M_gSO_4$), filtered, and concentrated to a white solid (326 mg, 85%) as the ethyl ester. The ethyl ester (312 mg, 0.595 mmol), THF (1.5 mL), and $H_2O$ (1.5 mL) were combined with lithium hydroxide, monohydrate (63 mg, 1.50 mmol). After 18 h, the clear solution was poured into I N aqueous HCl (25 mL) and extracted with EtOAc (2x25 mL). The organic layers were combined, dried ($M_gSO_4$), filtered, and concentrated to a white solid (247 mg, 85%). $^1H$ NMR (DMSO-$d_6$, 300 MHz) δ 7.79 (d, J=8.5, 1H), 7.77 (d, *J*=15.6 Hz, 1H), 7.51 (dt, *J*=7.5 *Hz, J*=1.7 Hz, 1H), 7.48 (dd, *J*=7.5 Hz, *J*=1.0 Hz, 1H), 7.20 (d, *J*=14.9 Hz, 1H), 7.19 (d, *J*=9.5 Hz, 1H), 7.06 (t, *J*=7.5 Hz, 1H), 6.63 (d, *J*=8.5 Hz, 1H), 4.01 (t, *J*=6.1 Hz, 2H), 3.65 (s, 2H), 3.58 (s, 6H), 2.10 (t, *J*=7.4 Hz, 2H), 1.75 (m, 2H); MS (APCI) *m/z* 496 $(M+H)^+$.

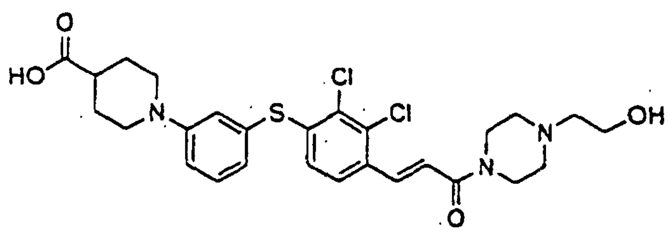

Example 415

[3-(4-Carboxypiperidin-1-yl)phenyl] [2,3-dichloro-4-( *E*-((4-hydroxyethylpiperazin-1-yl)carbonyl)ethenyl)phenyl] sulfide

The tide compound was prepared by the procedures described in Example 412 substituting 4-hydroxypiperidine with 1-hydroxyethylpiperazine. $^1H$ NMR (300 MHz, DMSO-$d_6$) δ 1.70 (m, 2H), 1.94 (m, 2H), 2.98 (m, 2H), 3.05 (m, 2H), 3.18 (m, 2H), 3.54 (m, 2H), 3.65 (m, 3 H), 3.78 (m, 2H), 6.77 (d, J = 8.8 Kz, 1 H), 7.03 (d, J = 6.8 Hz, 1H), 7.28 (d, J = 14.9 Hz, I H), 7.29 (m, 2H), 7.42 (t, J = 7.8 Hz, 1H), 7.78 (d, J =15.3 Hz, 1H), 7.86 (d, J = 8.8 Hz, 1H); MS (ESI$^+$) *m/z 564, 566* $(M+H)^+$.

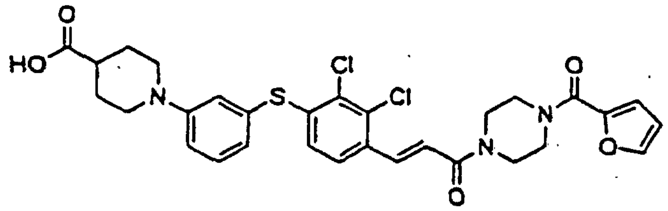

Example 416

[3-(4-Carboxypiperidin-1-yl)phenyl] [2,3-dichloro-4-(*E*-((4-furoylpiperazin-1-yl)carbonyl)ethenyl)phenyl] sulfide

The title compound was prepared by the procedures described in Example 412 substituting 4-hydroxypiperidine with 1-furoylpiperazine. $^1$H NMR (300 MHz, DMSO-$d_6$) δ 1.64 (m, 2H), 1.91 (m, 2H), 2.43 (m, 1H), 2.87 (m, 2H), 3.70 (m, 10H), 6.43 (m, 1H), 6.72 (d, J = 8.5 Hz, 1H), 6.94 (d, J = 7.8 Hz, 1H), 7.03 (d, J = 3.3 Hz, 1H), 7.13 (d, J = 7.8 Hz, 1H), 7.14 (s, 1H), 7.26 (d, J = 15.2 Hz, 1H), 7.36 (t, J =7.5 Hz, 1H), 7.77 (d, J =1 5.2 Hz, 1H), 7.86 (m, 2H); MS (ESI$^+$) *m/z* 614, 616 (M+H)$^+$. Anal. calcd for $C_{30}H_{29}N_3Cl_2SO_5$ 1.5 TFA: C, 50.45; H, 3.91; N, 5.35. Found: C, 50.53; H, 3.96; N, 5.35.

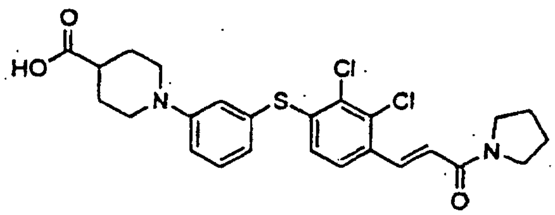

Example 417

[3-(4-Carboxypiperidin-1-yl)phenyl] [2,3-dichloro-4-(E-((pyrrolidin-1-yl)carbonyl)ethenyl)phenyl] sulfide

The title compound was prepared by the procedures described in Example 412 substituting 4-hydroxypiperidine with pyrrolidine. $^1$H NMR (300 MHz, DMSO-$d_6$) δ 1.63 (m, 2H), 1.79 (m, 2H), 1.88 (m, 4H), 2.43 (m, 1H), 2.82 (m, 2H), 3.39 (t, J = 6.7 Hz, 2H), 3.59 (t, J = 6.8 Hz, 2H), 3.68 (m, 2H), 6.71 (d, J = 8.5 Hz, 1H), 6.92 (d, J = 7.5 Hz, 1H), 6.96 (d, J = 15.3 Hz, 1H), 7.12 (d, J = 6.8 Hz, 1H), 7.13 (s, 1H), 7.35 (t, J = 8.8 Hz, 1H), 7.72 (d, J = 15.3 Hz, 1H), 7.80 (d, J = 8.5 Hz); MS (ESI$^+$) *m/z* 505, 507 (M+H)$^+$. Anal. calcd for $C_{25}H_{26}N_2Cl_2SO_3$ 0.8 TFA: C, 53.54; H, 4.53; N, 4.69. Found: C, 53.74; H, 4.40; N, 4.64.

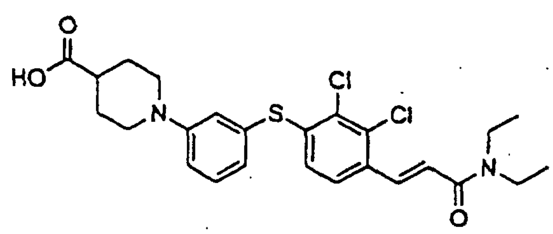

Examnte 418

[3-(4-Carboxypiperidin-1-yl phenyl][2,3-dichloro-4-(*E*-((diethylaminocarbonyl)ethenyl)phenyl] sulfide

The title compound was prepared by the procedures described in Example 412 substituting 4-hydroxypiperidine with diethylamine. $^1$H NMR (300 MHz, DMSO-$d_6$) δ 1.06(t, J = 6.7 Hz, 3H), 1.11 (t, J=6.7Hz, 3H), 1.63 (m, 2H),

1.88 (m, 2H), 2.43 (m, 1H), 2.82 (m, 2H), 3.35 (q, J = 6.7 Hz, 2H), 3.47 (q, J = 6.7 Hz, 2H), 3.70 (m, 2H), 6.68 (d, J = 8.5 Hz, 1H), 6.92 (d, J = 7.5 Hz, 1H), 7.07 (d, J = 15.2 Hz, 1H), 7.12 (d, *J* = 8.6 Hz, 1H), 7.13 (s, 1H), 7.35 (t, J = 8.8 Hz, 1H), 7.75 (d, *J* = 15.3 Hz, 1H), 7.84 (d, J = 8.5 Hz, 1H); MS (ESI$^+$) *m/z* 507 (M+H)$^+$. Anal. calcd for $C_{25}H_{28}N_2Cl_2SO_3$ 0.2 TFA: C, 57.53; H, 5.36; N, 5.28. Found: C, 57.68; H, 5.38; N, 5.33.

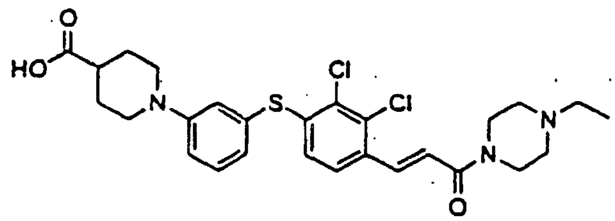

Example 419

[3-(4-Carboxypiperidin-1-yl)phenyl] [2,3-dichloro-4-(*E*-((4-ethylpiperazinyl)carbonyl)ethenyl)phenyl] sulfide

The title compound was prepared by the procedures described in Example 412 substituting 4-hydroxypiperidine with 1-ethylpiperazine. $^1$H NMR (300 MHz, DMSO-$d_6$) δ 1.22 (t, J = 7.5 Hz, 3H), 1.63 (m, 2H), 1.87 (m, 2H), 2.42 (m, 1H), 2.81 (t, J = 10.5 Hz, 2H), 3.00 (m, 2H), 3.15 (m, 2H), 3.40 (m, 1H), 3.51 (m, 2H), 3.67 (m, 2H), 4.50 (m, 2H), 6.73 (d, J = 8.8 Hz, I H), 6.89 (d, J = 7.8 Hz, 1H), 7.11 (m, 2H), 7.28 (d, J = 15.2 Hz, 1H), 7.36 (m, 1H), 7.80 (d, J = 15.2 Hz, I H), 7.86 (d, J = 8.5 Hz); MS (APCI$^+$) m/z 548, 550 (M+H)$^+$. Anal. calcd for $C_{27}H_{31}N_3Cl_2SO_3$ 2.2 TFA: C, 47.18; H, 4.19; N, 5.26. Found: C, 47.27; H, 4.27; N, 5.30.

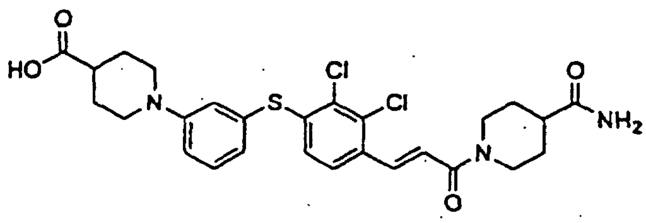

Example 420

[3-(4-Carboxypiperidin-1-yl)phenyl] [2,3-dichloro-4-(*E*-((4-(aminocarbonyl)piperidin-1-yl)carbonyl)ethenyl)phenyl] sulfide

The title compound was prepared by the procedures described in Example 412 substituting 4-hydroxypiperidine with isonipecotamide. $^1$H NMR (300 MHz, DMSO-$d_6$) δ 1.45 (m, 2H), 1.63 (m, 2H), 1.73 (m, 2H), 1.87 (m, 2H), 2.43 (m, 2H), 2.78 (m, 2H), 3.10 (m, 2H), 3.7 (m, 3H), 4.30 (m, 1H), 4.40 (m, 1H), 6.70 (d, J = 8.5 Hz, 1H), 6.78 (s, 1H), 6.92 (d, J = 7.5 Hz, 1H), 7.12 (m, 2H), 7.25 (d, J = 15.2 Hz, 1H), 7.27 (s, 1H), 7.35 (m, 1H), 7.73 (d, J = 15.2 Hz, 1H), 7.86 (d, j = 8.6 Hz, 1H); MS (APCI$^+$) m/z 562 (M+H)$^+$. Anal. calcd for $C_{27}H_{29}N_3Cl_2SO_4$ 0.2 TFA: C, 56.23; H, 5.03; N, 7.18. Found: C, 56.41; H, 4.96; N, 6.98.

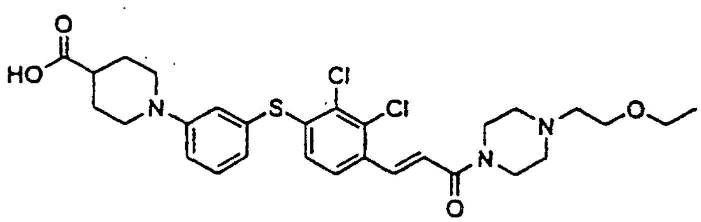

Example 421

[3-(4-Carboxypiperidin-1-yl)phenyl] [2,3-dichloro-4-(*E*-((4-(2-(ethoxyethyl)piperidin-1-yl)carbonyl)ethenyl)phenyl] sulfide

The title compound was prepared by the procedures described in Example 412 substituting 4-hydroxypiperidine with 1-(2-ethoxyethyl)piperazine. $^1H$ NMR (300 MHz, DMSO-$d_6$) δ 1.15 (t, J = 6.8 Hz, 3H), 1.63 (m, 2H), 1.90 (m, 2H), 2.42 (m, 1H), 2.81 (t, J =10.2 Hz, 2H), 3.09(m, 2H), 3.32 (m, 2H), 3.50 (q, J =6.8 Hz, 2H), 3.51 (m, 2H), 3.68 (m, 4H), 4.45 (m, 2H), 6.73 (d, J = 8.6 Hz, 1H), 6.91 (d, J = 7.5 Hz, 1H), (m, 7.11 (m, 2H, 7.26 (d, J = 15.3 Hz, 1H), 7.36 (m, 1H), 7.80 (d, J = 15.2 Hz, 1H), 7.83 (d, J = 8.5 Hz, 1H); MS (APCI$^+$) *m/z* 592 $(M+H)^+$. Anal. calcd for $C_{29}H_{35}N_3Cl_2SO_4$. 2.5 TFA: C, 46.53; H, 4.31; N, 4.79. Found: C, 46.51; H, 4.31; N, 4,77.

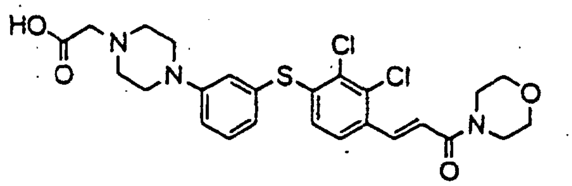

Example 422

[3-((4-Carboxymethyl)piperazin-1-yl)phenyl] [(2,3-dichloro-4-(E-(4-morpholinyl)carbonyl)ethenyl)phenyl] sulfide

The title compound (24 mg, 42%) was prepared from Example 393A as described in Example 384D, substituting morpholine with 1-((ethoxycarbonyl)methyl)piperazine, followed by hydrolysis with LiOH as described in Example 340H. $^1H$ NMR (300 MHz, MeOH-$d_4$) δ 3.54 (s, 8H), 3.69 (s, 8H), 4.1 (s, 2H), 6.77 (d, J=8.5 Hz, 1H), 7.06 (d, J=15 Hz, 1H), 7.08 (m, 1H), 7.42 (t, J=7.5 Hz, 1H), 7.59 (d, J=8.25 Hz, 1H), 7.93 (d, J=15 Hz, 1H); MS (ESI) *m/e* 536, 538 $(M+H)^+$.

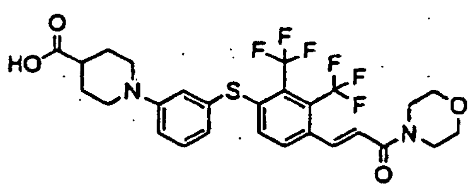

Example 423

[3-(4-Carboxypiperidin-1-yl)phenyl] [2,3-bis(trifluoromethyl)-4-(E-((4-morpholino)carbonyl)ethenyl)phenyl]sulfide

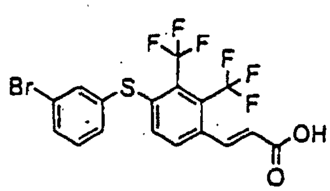

Example 423A

[3-Bromophenyl] [2,3-bis(trifluoromethyl)-4-(E-(2-carboxy)ethenyl)phenyl]sulfide

The title compound was prepared from Example 401C using the procedures described in Example 384A, followed by hydrolysis with LiOH as given in Example 340H.

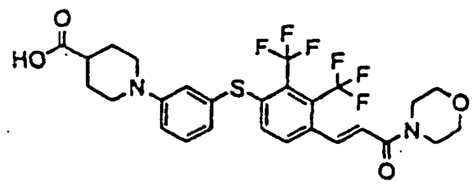

Example 423B

[3-(4-Carboxypiperidin-1-yl)phenyl][2,3-bis(trifluoromethyl)-4-(E-((4-morpholino)carbonyl)ethenyl)phenyl]sulfide

The title compound was prepared from Example 423A as described in Example 340G, substituting methy isonipecotate with morpholine, followed by amination with ethyl isonipecotate as described in Example 396, and subsequent hydrolysis according to the procedure of Example 340H. $^1$H NMR (400 MHz, DMSO-$d_6$) δ 1.5-1.64 (m, 2H), 1.67-1.75 (m, 1H), 1.87-1.96 (m, 1H), 2.49-2.57 (m, 2H), 2.82-2.91 (m, 1H), 2.99-3.06 (m, 1H), 3.46-3.54 (m, 2H), 3.54-3.62 (m, 5H), 3.63-3.72 (m, 3H), 6.87 (d, J=8 Hz, 1H), 7.06-7.13 (m, 2H), 7.16 (d, J=15 Hz, 1H). 7.25-7.36 (m, 2H), 7.66 (dd, $J_1$=15 Hz, $J_2$=4 Hz, 1H), 8.00 (d, J=8 Hz, 1H); MS (ESI) *m/e* 589 $(M+H)^+$.

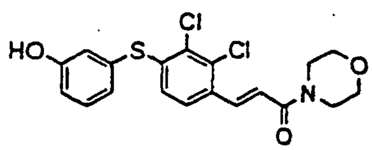

Example 424

(3-Hydroxyphenyl) [2,3-dichloro-4-(E-((4-morpholino)carbonyl)ethenyl)phenyl]sulfide

Example 310B was processed as described in Examples 310 and 405, substituting 3-methoxythiophenol for the thiol. $^1$H NMR (DMSO-$d_6$, 300 MHz) δ 9.88 (s, 1H), 7.86 (d, *J*=8.8, 1H), 7.77 (d, *J*=14.9 Hz, 1H), 7.33 (dt, *J*=7.4 Hz, *J*=1.0 Hz, 1H), 7.24 (d, *J*=14.8 Hz, 1H), 6.96 (dd, *J*=8.8 Hz, *J*=1.0 Hz, 1H), 6.90 (dd, *J*=8.8 Hz, *J*=1.0 Hz, 1H), 6.89 (s, 1H), 6.79 (d, *J*=8.5 Hz, 1H), 3.67 (s, 2H), 3.58 (s, 6H); MS (APCI) *m/z* 410 $(M+H)^+$, 427 $(M+NH_4)^+$; 408 $(M-H)^-$, 446 $(M+Cl)^-$. Anal. calcd for $C_{19}H_{17}Cl_2NO_3S0.25\ H_2O$: C, 55.01; H, 4.25; N, 3.38. Found: C, 55.15; H, 4.25; N, 3.51.

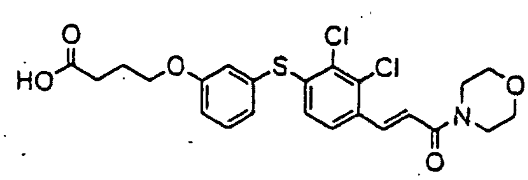

Example 425

[3-(4-Butyroxy)phenyl] [2,3-dichloro-4-(E-((4-morpholino)carbonyl)ethenyl)phenyl]sulfide

Example 424 was processed as described in Example 414 to provide the title compound as a white solid. $^1$H NMR (DMSO-$d_6$, 300 MHz) δ 7.86 (d, *J*=8.8 Hz, 1H), 7.77 (d, *J*= 15.3 Hz, 1H), 7.43 (dt, *J*=7.5 Hz, *J*=1.7 Hz, 1H), 7.24 (d, *J*=15.2 Hz, 1H), 7.11 (s, 1H), 6.79 (d, *J*=8.4 Hz, 1H), 6.73 (d, *J*=8.5 Hz, 1H), 4.02 (t, *J*=6.5 Hz, 2H), 3.67,(s, 2H),

3.58 (s, 6H), 2.37 (t, *J*=7.5 Hz, 2H), 1.95 (m, 2H); MS (APCI) *m/z* 410 (M+H$^+$), 494 (M-H)$^-$, 530 (M+Cl)$^-$.

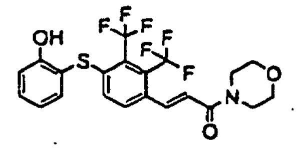

Example 426

(2-Hydroxyphenyl) [2,3-bis(trifluoromethyl)-4-(E-((4-morpholino)carbonyl)ethenyl)phenyl]sulfide

Example 401D was processed as described in Example 405 to provide the title compound as a white solid. $^1$H NMR (DMSO-$d_6$, 300 MHz) δ 10.26 (s, 1H), 7.96 (d, *J*=8.5, 1 H), 7.67 (m, 1H), 7.46 (dd, *J*=7.4 Hz, *J*=1.3 Hz, 1 H), 7.38 (dt, *J*=7.5 Hz, *J*=1.3 Hz, 1H), 7.16 (d, *J*=15.2 Hz 1 H), 7.13 (d, *J=8.5* Hz, 1 H), 7.00 (d, *J*=8.2 Hz, 1H), 6.90 (t, *J*=7.4 Hz, 2H), 3.65 (s, 2H), 3.57 (s, 6H). MS (APCI) *m/z* 478 (M+H)$^-$, 495 (M+NH$_4$)$^+$; 476 (M-H)$^-$, 512 (M+Cl)$^-$.

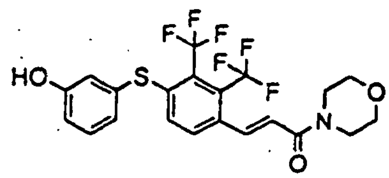

Example 427

(3-Hydroxyphenyl)[2,3-bis(trifluoromethyl)-4-(E-((4-morpholino)carbonyl)ethenyl)phenyl]sulfide

Example 401C was processed as described in Example 401 D, substituting 3-methoxythiophenol for the thiol, and in Example 405 to provide the title compound as a white solid. $^1$H NMR (DMSO-$d_6$, 300 MHz) δ 9.86 (s, 1H), 8.02 (d, *J*=8.8, 1H), 7.67 (m, 1H), 7.35 (d, *J*=9.5 Hz, 1H), 7.30 *(dt, J*=7.8 Hz, J=0.7 Hz, 1 H), 7.19 (d, *J*=15.2 Hz 1H), 6.95 (d, *J*=8.8 Hz, 1H), 6.88 *(*d, *J*=7.5 Hz, 1H), 6.85 (s, 1H), 3.67 (s, 2H), 3.58 (s, 6H). MS (APCI) *m/z* 478 (M+H$^+$), 495 (M+NH4$^+$), 476 (M-H$^+$), 512 (M+Cl$^-$).

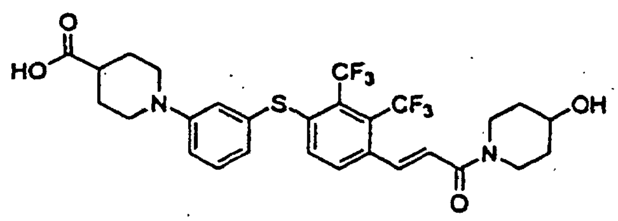

Example 428

[3-(4-Carboxypiperidin-1-yl)phenyl][2,3-ditrifluoromethyl-4-(*E*-((4-hydroxypiperidin-1.yl)carbonyl)ethenyl)phenyl] sulfide

The title compound was prepared by the procedures described in Example 412 substituting Example 384B with Example 423A. $^1$H NMR (300 MHz, DMSO-$d_6$) δ 1.31 (m, 2H), 1.63 (m, 2H), 1.71 (m, 2H), 1.91 (m, 2H), 2.42 (m, 1H), 2.82 (t, J = 10.5 Hz, 2H), 3.16 (m, 1H), 3.31 (m, 1H), 3.70 (m, 3H), 3.93 (m, 2H), 6.88 (d, J = 7.1 Hz, 1H), 7. 10 (d, J = 8.5 Hz, 1H), 7.14 (t, J = 7.1Hz, 1H), 7.23 (d, J = 8.8 Hz, 1H), 7.28 (d, J = 8.2 Hz, 1H), 7.34 (d, J= 8.2 Hz, 1H), 7.60 (dq, J = 15.2, 4.5 Hz, 1H), 8.01 (d,J = 8.8 Hz, 1H); MS (APCI$^+$) *m/z* 603 (M+H)$^+$ Anal. calcd for $C_{28}H_{28}F_6N_2O_4S$ 1.15 TFA: C, 49.60; H, 4.00; N, 3.82. Found: C, 49.65; H, 3.80; N, 3.81.

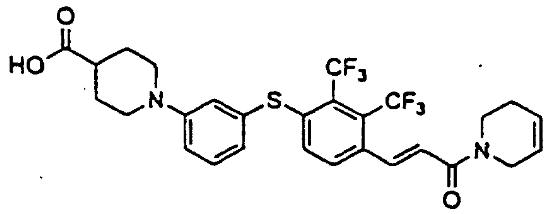

Example 429

[3-(4-Carboxypiperidin-1-yl)phenyl][2,3-ditrifluoromethyl-4-(*E*((1,2,5,6-tetrahydropyridin-1-yl)carbonyl)ethenyl) phenyl] sulfide

The title compound was prepared by the procedures described in Example 412 substituting Example 384B with Example 423A, and substituting 4-hydroxypiperidine with 1,2,5,6-tetrahydropyridine. $^1$H NMR (300 MHz, DMSO-$d_6$) δ 1.63 (m, 2H), 1.90 (m, 2H), 2.12 (m, 2H), 2.43 (m, 1H), 2.81 (t, J =10.5 Hz, 2H), 3.75 (m, 4H), 4.01 ((s, 1H), 4.15 (s, 1H), 5.73 (m, 1H), 5.84 (m, 1H), 6.87 (d, J = 7.5 Hz, 1H), 7.10. (m, 2H), 7.30 (m, 3H), 7.62 (m, 1H), 8.01 (t, J = 6.5 Hz, 1 H); MS (APCI$^+$) *m/z* 585 (M+H)$^+$. Anal. calcd for $C_{28}H_{26}F_6N_2O_3S$ 0.1 TFA: C, 56.83; H, 4.41; N, 4.70. Found: C, 56.91; H, 4.44; N, 4.60.

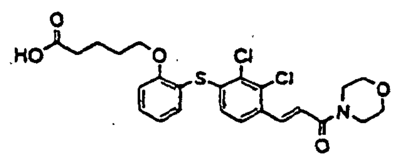

Example 430

[2-((4-Carboxy)butyloxy)phenyl][2,3-dichloro-4-(E-((4-morpholino)carbonyl)ethenyl)phenyl]sulfide

Example 405 was processed as described in Example 414, substituting ethyl 5-bromovalerate for the alkyl halide. $^1$H NMR (DMSO-$d_6$, 300 MHz) δ 7.79 (d, J=8.5, 1H), 7.77 (d, *J*=15.3 Hz, 1H), 7.51 (dt, J=8.2 Hz, *J*=1.7 Hz, 1H), 7.48 (dd, *J*=7.5 Hz; *J*=1.7 Hz, 1H), 7.20 (dd, *J*=7.5 Hz, *J*=1.7 Hz, 1H), 7.20 (d, *J*=15.6 Hz, 1H), 7.05 (dt, *J*=7.5 Hz, *J*=1.0 Hz, 1H), 6.63 (d, *J*=8.5 Hz, 1H), 3.99 (t, *J*=6.1 Hz, 2H), 3.65 (s, 2H), 3.58 (s, 6H), 2.10 (t, *J*=7.1 Hz, 2H), 1.56 (m, 2H), 1.39 (m, 2H). MS (APCI) *m/z* 510 (M+H)$^+$. Anal. calcd for $C_{24}H_{25}Cl_2NO_5S \cdot 0.75\ H_2O$: C, 55.02; H, 5.10; N, 2.67. Found: C, 54.72; H, 4.82; N, 2.77.

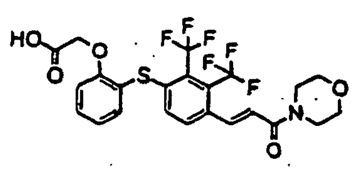

Example 431

(2-Glycoxyphenyl) [2,3-bis(trifluoromethyl)-4-(E-((4-morpholino)carbonyl)ethenyl)phenyl]sulfide

Example 426 was processed as detailed in Example 414, substituting ethyl bromoacetate for the alkyl bromide. $^1$H NMR (DMSO-$d_6$, 300 MHz) δ 7.94 (d, J=8.4, 1H), 7.66 (m, 1H), 7.50 (m, 1H), 7.47 (d, *J*=7.4 Hz, 1H), 7.28 (d, *J*=8.5 Hz, 1H), 7.16 (d, *J*=14.9 Hz, 1H), 7.07 (m, 2H), 4.74 (s, 2H), 3.65 (s, 2H), 3.57 (s, 6H): MS (APCI) m/z 536 (M+H)$^+$, 553 (M+$NH_4$)$^+$; 534 (M-H)$^+$. Anal. calcd for $C_{23}H_{19}F_6NO_5S$: C, 51.59; H, 3.58; N, 2.62. Found: C, 51.31; H, 3.63; N, 2.33.

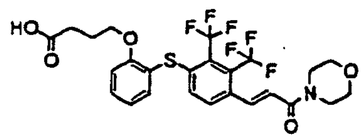

Example 432

(2-( 4-Butyroxy)phenyl)[2,3-bis(trifluoromethyl)-4-(E-((4-morpholino)carbonyl)ethenyl)phenyl]sulfide

Example 426 was processed as described in Example 414 to provide the title compound as a white solid. $^1H$ NMR (DMSO-$d_6$, 300 MHz) δ 7.92 (d, *J*=8.5, 1H), 7.65 (m, 1H), 7.59 (d, *J*=7.8 Hz, *J*=1.7 Hz, 1H), 7.50 (t, *J*=8.2 Hz, 1H), 7.15 (d, *J*=8.2 Hz, 1H), 7.12 (d, *J*=15.6 Hz, 1H), 7.09 (d, *J*=8.8 Hz, 1H), 7.07 (t, *J*=7.2 Hz, 1H), 3.92 (t, *J*=6.1 Hz, 2H), 3.65 (s, 2H), 3.57 (s, 6H), 1.99 (t, *J*=7. 1Hz, 2H), 1.63 (m, 2H); MS (APCI) *m/z* 562 $(M-H)^-$. 598 $(M+Cl)^-$.

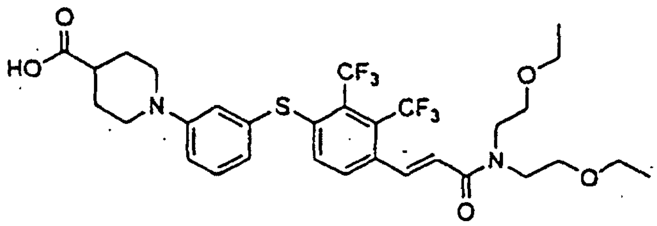

Example 433

[3-(4-Carboxypiperidin-1-yl)phenyl] [2,3-ditrifluoromethyl-4-(*E*-((bis-(2-ethoxyethyl)amino)carbonyl)ethenyl)phenyl] sulfide

The title compound was prepared by the procedures described in Example 412 substituting Example 384B with Example 423A, and substituting 4-hydroxypiperidine with bis (2-ethoxyethyl)amine. $^1H$ NMR (300 MHz, DMSO-$d_6$) δ 0.99 (t, J = 6.8 Hz, 3H), 1.09 (t, J = 6.8 Hz, 3H), 1.63 (m, 2H), 1.90 (m, 2H), 2.44 (m, 1H), 2.82 (t, J = 10.8 Hz, 2H), 3.40 (m, 4H), 3.50 (m, 6H), 3.68 (m, 4H), 6.88 (d, J = 7.5 Hz, 1H), 7.11 (m, 3H), 7.32 (m, 2H), 7.62 (dq, J = 15.2 4.5 Hz, 1H), 7.94 (d, J = 8.5 Hz, 1H); MS ($APCI^+$) *m/z* 663 $(M+H)^+$ Anal. calcd for $C_{31}H_{36}F_6N_2O_5S$ 0.7 TFA: C, 52.41; H, 4.98; N, 3.77. Found: C, 52.38; H, 4.94; N, 3.68.

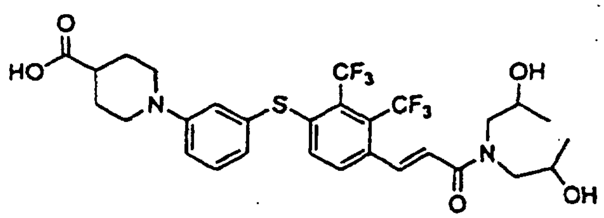

Example 434

[3-(4-Carboxypiperidin-1-yl)phenyl][2,3-bis-(trifluoromethyl)-4-(*E-(bis-(2*-hydroxypropyl)amino)carbonyl)ethenyl) phenyl] sulfide

The title compound was prepared by the procedures described in Example 412 substituting Example 384B with Example 423A, and substituting 4-hydroxypiperidine with diisopropanolamine. $^1H$ NMR (300 MHz, DMSO-$d_6$) δ 1.04 (m, 6H), 1.63 (m, 2H), 1.90 (m, 2H), 2.42 (m, 1 H), 2.83 (t, J = 10.5 Hz, 2H), 3.04 (m, 1H), 3.26 (m, 1H), 3.45 (m, 2H), 3.67 (m, 2H), 3.75 (m, 1H), 3.90 (m, 1H), 6.90 (d, J = 7.5 Hz, 1H), 7.11 (m, 3 H), 7.28 (d, J = 8.5 Hz, 1H), 7.33 (t, J = 8.2 Hz, 1H), 7.63 (m, 1H), 7.95 (dd, J = 8.5, 2.1 Hz, 1 H); MS ($APCI^+$) *m/z* 635 $(M+H)^+$. Anal. calcd for $C_{29}H_{32}F_6N_2O_5S$ 1.5 TFA: C, 47.71; H, 4.19; N, 3.48. Found: C, 47.52; H, 4.28; N, 3.40.

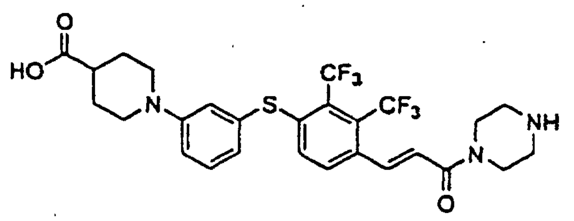

Example 435

[3-(4-Carboxypiperidin-1-yl)phenyl] [2,3-bis-(trifluoromethyl)-4-(*E*-((piperazin-1-yl)carbonyl)ethenyl)phenyl]sulfide

The title compound was prepared by the procedures described in Example 412 substituting Example 384B with Example 423A, and substituting 4-hydroxypiperidine with 1-(1,2,3,4-tetrahydrofuroyl)piperazine. $^1$H NMR (300 MHz, DMSO-$d_6$) δ 1.62 (m, 2H), 1.88 (m, 2H), 2.43 (m, 1H), 2.82 (t, J = 10.5 Hz, 2H), 3.15 (brs, 4H), 3.71 (m, 2H), 3.75 (m, 2H), 3.86 (m, 2H), 6.87 (d, J = 7.5 Hz, 1H), 7.10 (d, J = 8.5 Hz, 1H), 7.12 (d, J = 7.5 Hz, 1H), 7.20 (d, J = 15.2 Hz, 1H), 7.28 (d, J = 8.5 Hz, 1H), 7.35 (d, J = 7.8 Hz, 1H), 7.70 (dt, J = 15.2, 4.5 Hz, 1H), 7.98 (d, J= 8.5 Hz, 1H), 8.85 (br s, 1H); MS (APCI$^+$) *m*/*z* 588 $(M+H)^+$. Anal. calcd for $C_{27}H_{27}F_6N_3O_3S$ 3.3 TFA: C, 41.87; H, 3.17; N, 4.36. Found: C, 41.78; H, 3.26; N, 4.43.

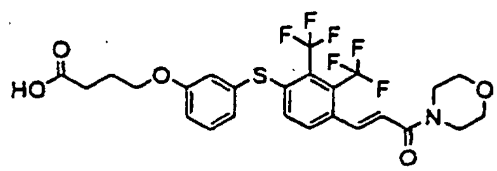

Example 436

(3-(4-Butyroxy)phenyl)[2,3-bis(trifluoromethyl)-4-(E-((4-morpholino)carbonyl)ethenyl)phenyl]sulfide

Example 427 was processed as described in Example 414 to provide the title compound as a white solid. $^1$H NMR (DMSO-$d_6$, 300 MHz) δ 8.02 (d, *J*=8.5, 1H), 7.65 (m, 1H), 7.40 (t, *J*=7.8 Hz, 1H), 7.33 (d, *J*=8.8 Hz, 1H], 7.19 (d, *J*=14.9 Hz, 1H), 7.09 (m, 3H), 4.02 (t, *J*=6.4 Hz, 2H), 3.67 (s, 2H), 3.58 (s, 6H), 2.37 (t, *J*=7.1 Hz, 2H). 1.95 (m, 2H); MS (APCI) *m*/*z* 564 $(M+H)^+$; 562 $(M-H)^+$, 598 $(M+Cl)^-$.

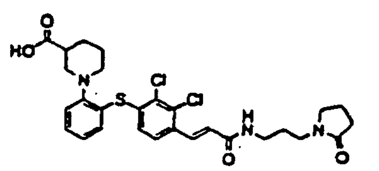

Example 437

[2-(3-Carboxypiperidin-1-yl)phenyl][2,3-dichloro-4-[*E*-[(3-(2-pyrrolidinon-1-yl)propylaminocarbonyl)ethenyl)phenyl] sulfide

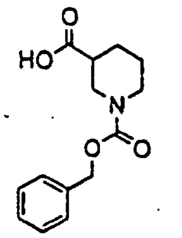

Example 437A

N-Benzyoxycarbonyl nipecotic acid

To a solution of nipecotic acid (10 g, 63.6 mmol) in I N NaOH (2.5 g in 64 ml water, 63.6 mmol) at 0 °C was alternately added benzyloxycarbonyl chloride (10.9 mL, 76.5 mmol) in diethyl ether (50 mL) and I N NaOH (5 g in 128 ml water, 127.2 mmol) in five portions. The reaction mixture was stirred at 0 °C for 2 h, and at ambient temperature for 24 h. Then this was made acidic with 10% HCl and the solid formed was filtered and dried (vacuum oven, 45 °C) to obtain the title compound (18.9 g, 113%). MS (ESI) *m*/*e* 264 $(M+H)^+$.

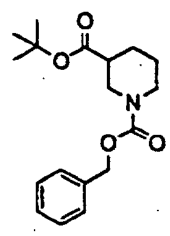

Example 437B

tert-Butyl N-benzyoxycarbonyl nipecotate

A solution of Example A (18 g, 62 mmol) in THF (250 mL, 0.25 M) was treated with trichloroacetimidate (28 mL, 15 5 mmol) and $BF_3 \cdot Et_2O$ (18 mL, 1 mL/g) at ambient temperature. After 18 h the reaction mixture was quenched with solid $NaHCO_3$ followed by water and stirred vigorously. Then the solvent was removed, and partitioned with ethyl acetate (250 mL). The organic layer was separated and washed with brine (3x80 mL), dried ($Na_2SO_4$) and evaporated to dryness under reduced pressure to obtain the crude product. The title compound (19.2 g, 96%) was obtained by flash chromatography on silica gel eluting with 20% acetone:hexane. MS (ESI) *m*/*e* 320 $(M+H)^+$.

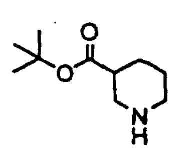

Example 437C

*tert*-Butyl nipecotate

Example 437B (19 g,59.5 mmol) was treated with 10% Pd on carbon (2 g, 10 wt %) in ethanol (237 mL, 0.25 M) to obtain the title compound (10.4 g, 94%). MS (ESI) *m/e* 186 $(M+H)^+$.

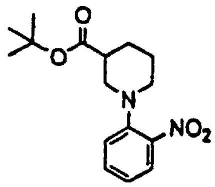

Example 437D

2-Nitro-(3-(*tert*-butyloxycarbonyl)piperidin-1-yl)benzene

To a solution of Example 437C (10.4 g, 56.1 mmol) in toluene (112 mL) was added 2-fluoronitrobenzene (6.0 ml, 56 mmol) and CsF (852 mg, 5.6 mmol). The reaction mixture was stirred under reflux conditions for 18 h, and allowed to cool to ambient temperature. The mixture was diluted with ethyl acetate (100 ml), washed with 10% HCl (2x50 ml), followed by brine (3x100 ml), then dried ($Na_1SO_4$) and evaporated in vacuo to obtain the title compound (16.5 g, 94%). MS (ESI) *m/e* 307 $(M+H)^+$.

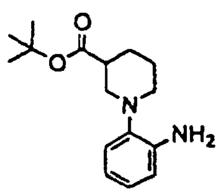

Example 437E

2-Amino-(3-*tert*-butyloxycarbonyl)piperidin-1 -yl)benzene

Example 437E (16.4 g, 53.5 mmol) was treated with 10% Pd on carbon (1.65 g, 10 wt %) in ethanol (215 ml, 0.5 M) at ambient temperature for 2 hours. The reaction mixture was filtered through celite and the filtrate was evaporated to dryness under reduced pressure to obtain the title compound (13.45 g, 91%). MS (ESI) *m/e* 277 $(M+H)^+$.

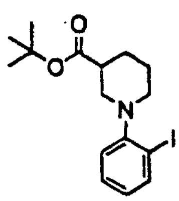

Example 437F

2-Iodo-(3-(*tert*-butyloxycarbonyl)piperidin-1-yl)benzene

Example 43 7E was dissolved in 3 N $H_2SO_4$ (195 mL, 0.25 M), cooled to. 0 °C and treated with $NaNO_2$ (3.35 g, 48.6 mmol) in water (20 mL). After 30 minutes at 0 °C potassium iodide (12.01 g, 72.8 mmol) and urea (583 mg, 9.7 mmol) in water (10 mL) were added and stirred for l h. The reaction mixture was quenched with 10% $NaHCO_3$ (50 mL) and partitioned with ethyl acetate (450 mL). The organic layer was separated and washed with 10% $NaHCO_3$ (2x100 mL), brine (2x100mL), dried ($Na_2SO_4$)and evaporated to dryness under reduced pressure. The title compound

(17.2 g, 91%) was obtained by flash chromatography on silica gel eluting with 10% acetone:hexane. $^1$H NMR (400 MHz, DMSO-$d_6$) δ 1.39 (s, 9H),6.85 (tt, $J_1$=1.5 Hz, $J_2$=7.5 Hz, 1H), 7.14 (dd, $J_1$=1.5 Hz, $J_2$=7.5 Hz, 1H), 7.37 (tt, $J_1$=1.5 Hz, $J_2$=7.5 Hz, 1H), 7.84 (dd, $J_1$=1.5 Hz, $J_2$=7.5 Hz, 1H); MS (ESI) *m/e* 388 (M+H)$^+$.

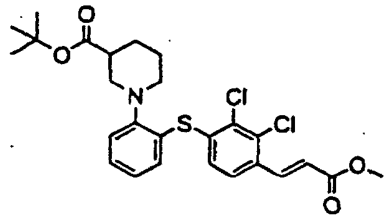

Example 437G

[2-(3-tert-Butyloxycarbonyl)piperidin-1-yl)phenyl] [2,3-dichloro-4-(*E*-(2-methoxycarbonyl)ethenyl)phenyl] sulfide

Example 437F was converted to the corresponding triisopropylsilyl thiol analogue by the method describe d for the preparation of Example 340B. Then this intermediate was reacted with Example 340E (2.94 g, 7.75 mmol) at -20 °C as described in Example 340F to obtain the title compound (2.5 g, 63%). MS (ESI) *m/e* 522, 524 (M+H)$^+$.

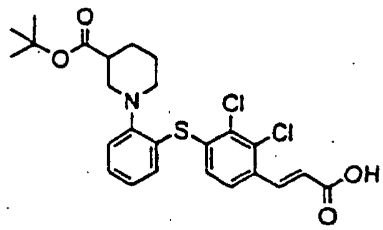

Example 437H

[2-(3-*tert*-Butyloxycarbonyl)piperidin-1-yl)phenyl] [2,3-dichloro-4-(*E*-(2-carboxy)ethenyl)phenyl] sulfide

Using the procedure for Example 340H, Example 437H was hydrolyzed to the title compound. MS (ESI) *m/e* 508, 510 (M+H)$^+$.

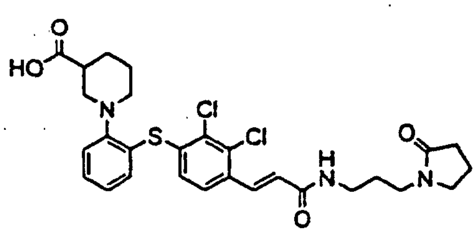

Example 4371

[2-(3-Carboxypiperidin-1-yl)phenyl] [2,3-dichloro-4-(E-[(3-(2-pyrrolidinon-1-yl)propylaminocarbonyl)ethenyl)phenyl] sulfide

The title compound (66 mg) was prepared from Example 437H (90 mg, 0.177 mmol), using the procedures described in Example 340G substituting methyl isonipecotate with 3-aminopropyl-2-pyrrolidinone followed by treatment with 20% TFA in methylene chloride as described in Example 412C. $^1$H NMR (500 MHz, DMSO-$d_6$) δ 1.27-2.43 (m, 2H), 2.60-2.72 (m, 3H), 2.84 (m, 3H), 2.17-2.30 (m, 3H), 2.62-2.73 (m, 2H), 3.08-3.23 (m, 5H), 3.29-3.38 (m, 3H), 6.62 (d, J=1 5 Hz, 1H), 6.92 (d, J=8.5 Hz, 1H), 7.12 (t, J=7.5 Hz, 1H), 7.41 (m, 1H), 7.58 (d, J=8.75 Hz, 1H), 7.70 (d, J=15 Hz, 1H), 8.21 (t, J=5 Hz, 1H); MS (ESI) *m/e* 576, 578 (M+H)$^+$.

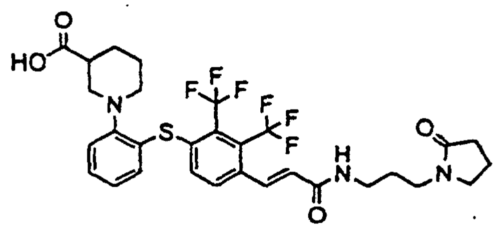

Example 438

[2-(3-Carboxypiperidin-1-yl)phenyl] [2,3-bis(trifluoromethyl)-4-(*E*-[(3-(2-pyrrolidinon-1-yl)propylaminocarbonyl) ethenyl)phenyl] sulfide

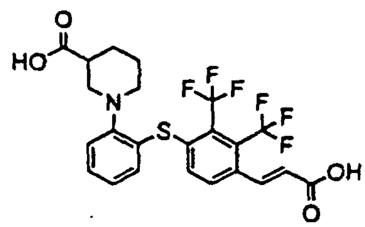

Example 438A

[2-(3-tert-Butyloxycarbonyl)piperidin-1-yl)phenyl] [2,3-bis(trifluoromethyl)-4-(*E*-(2-carboxy)ethenyl)phenyl] sulfide

The title compound (445 mg, 71%) was prepared from the reaction of Example 401C (500 mg, 1.08 mmol) with Example 437F, using the procedures described in Example 437G followed by hydrolysis as described in Example 340G. MS (ESI) *m/e* 604 $(M+H)^-$.

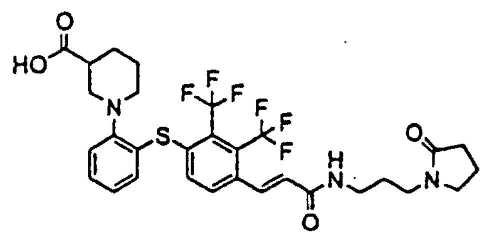

Example 438B

[2-(3-Carboxypiperidin-1-yl)phenyl [2,3-bis(trifluoromethyl)-4-(*E*-[(3-(2-pyrrolidinon-1-yl)propylaminocarbonyl) ethenyl)phenyl] sulfide

The title compound was prepared from Example 438A (110 mg, 0.191 mmol), using the procedures described in Example 340G substituting methyl isonipecotate with 3-aminopropyl-2-pyrrolidinone followed by treatment with 20% TFA in methylene chloride as described in Example 412C. $^1$H NMR (500 MHz, DMSO-$d_6$) δ 1.11-1.21 (m, 1H), 1.28-1.38 (m, 1H), 1.60-1.70 (m, 3H), 1.79-1.86 (m, 1H), 1.87-1.94 (m, 2H), 2.05-2.12 (m, 1H), 2.00 (t, J=7.5 Hz, 2H), 2.58-2.66 (m, 2H), 2.96-3.01 (m, 1H), 3.11-3.18 (m, 2H), 3.19 (t, J=6.25 Hz, 2H), 3.26 (m, 1H), 3.32 (t, J=6.25 Hz, 2H), 6.46 (d, J=15 Hz, 1H), 7.15 (t, J=7.5 Hz, 1H), 7.33 (m, 2H), 7.43 (m, 1H), 7.62 (m, 1H), 7.75 (d, J=7.5 Hz, 1H), 822 (t, J=5 Hz, 1H); MS (ESI) *m/e* 644 $(M+H)^-$.

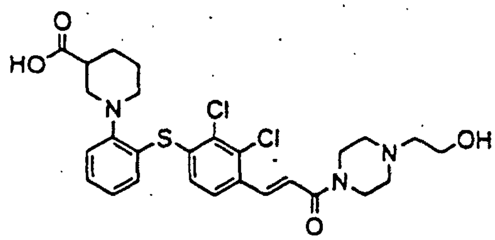

Example 439

[2-(3-Carboxypiperidin-1-yl)phenyl] [2,3-dichloro-4-(*E*-((4-(2-hydroxyethyl)piperazin-1-yl)carbonyl)ethenyl)phenyl] sulfide

The title compound was prepared from Example 437H (90 mg, 0.177 mmol), using the procedures described in Example 340G substituting methyl isonipecotate with 1-(2-hydroxyethyl)piperazine followed by treatment with 20% TFA in methylene chloride as described in Example 412C. $^1H$ NMR (500 MHz, DMSO-$d_6$) δ 1.31-1.45 (m, 2H), 1.67-1.74 (m, 1H), 1.86-1.92 (m, 1H), 2.24-2.31 (m, 1H), 2.66 (t, J=10 Hz, 1H), 2.73 (t, J=10Hz, 1H), 3.02-3.24 (m, 5H), 3.33-3.38 (m, 1H), 3.52 (m, 3H), 3.75 (t, J=5 Hz, 2H), 4.31-4.60 (m, 3H), 6.90 (d, J=9 Hz, 1H), 7.11 (t, J=7.5 Hz, 1H), 7.21 (d, J=7.5. Hz, 1H), 7.25 (d, J=7.5 Hz, 1H), 7.30 (d, J= 15 Hz, 1H), 7.42 (t, J=7.5Hz, 1H), 7.83 (d, J=15 Hz, I H), 7.85 (d, 9 Hz, 1H); MS (ESI) *m/e* 564, 566 $(M+H)^+$.

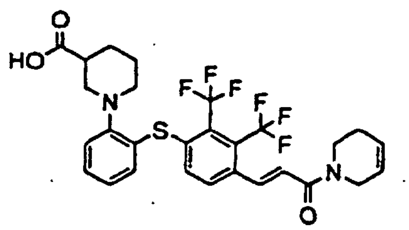

Example 440

[2-(3-Carboxypiperidin-1-yl)phenyl] [2.3-bis(trifluoromethyl)-4-(*E*-((1,2,5,6-tetrahydropyridin-1-yl)carbonyl)ethenyl) phenyl] sulfide

The title compound was prepared from Example 438A (110 mg, 0.191 mmol), using the procedures described in Example 340G substituting methyl isonipecotate with 1,2,5,6-tetrahydropyridine followed by treatment with 20% TFA in methylene chloride as described in Example 412C. $^1H$ NMR (500 MHz, DMSO-$d_6$) δ 1.19-1.27 (m, 1H), 1.30-1.40 (m, 1H), 1.64-1.70 (m, 1H), 1.80-1.86 (m, 1H), 2.04-2.18 (m, 2H), 2.60 (t; J=10 Hz, 1H), 2.68 (t, J=10 Hz, 1H), 3.03 (br d, J=10 Hz, 1H), 3.23 (br d, J=10 Hz, 1H), 3.60-3.74 (m, 2H), 3.91-4.20 (m, 3H), 5.68-5.74 (m, 1H), 5.80-5.90 (m, 1H), 7.06-7.19 (m, 2H), 7.20-7.28 (m, 2H), 7.36 (m, 1H), 7.44 (t, J=7.5 Hz, 1H), 7.62-7.71 (m, 1H), 7.94-8.04 (m; 1H); MS (ESI) *m/e* 585 $(M+H)^+$.

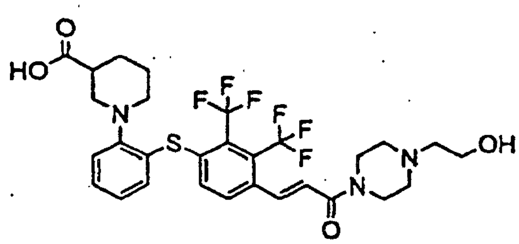

Example 441

[2-(3-Carboxypiperidin-1-yl)phenyl] [2,3-bis(trifluoromethyl)-4-E-((4-(2-hydroxyethyl)piperazin-1-yl)carbonyl)ethenyl) phenyl]sulfide

The title compound was prepared from Example 438A (110 mg, 0.191 mmol), using the procedures described in Example 340G substituting methyl isonipecotate with 1-(2-hydroxyethyl)piperazine followed by treatment with 20% TFA in methylene chloride as described in Example 412C. $^1$H NMR (500 MHz, DMSO-$d_6$) δ 1:17-1.27 (m, 1H), 1.17-1.27 (m, 1H), 1.31-1.41 (m,1H), 1.64-1.71 (m, 1H), 1.80-1.88 (m, 1H), 2.07-2.15 (m, 1H), 2.61 (t, J=10 Hz, 1H), 2.09 (t, J=10 Hz, 1H), 2.91-3.13 (m, 3H), 3.18-3.28 (m,3H), 3.44-3.58 (m, 3H), 3.75 (t, J=5 Hz, 2H), 4.29-4.58 (m,3H), 7.15 (d, J=7.5 Hz, 1H), 7.19 (d, J=10 Hz, 1H), 7.28 (d, J=7.5 Hz, 2H), 7.37 (d, J=7.5 Hz,1H), 7.45 (t, J=7.5Hz, 1H), 7.69-7.77 (m, 1H), 7.98 (d, J=7.5 Hz, 1H), 9.77 (br s, 1H); MS (ESI) *m/e* 632 $(M+H)^+$.

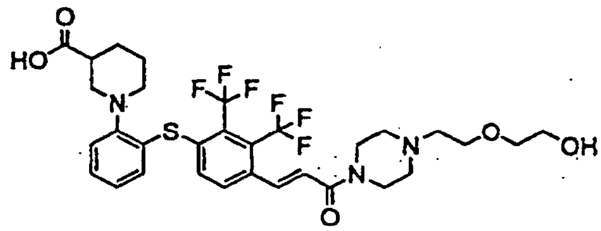

Example 442

[2-(3-Carboxypiperidin-1-yl)phenyl] [2,3-bis(trifluoromethyl)-4-(*E*-((4-(2-(hydroxyethoxy)ethyl)piperazin-1-yl)carbonyl) ethenyl)phenyl] sulfide

The title compound was prepared from Example 438A (110 mg, 0.191 mmol), using the procedures described in Example 340G substituting methyl isonipecotate with 1-[2-(2-hydroxyethoxy)ethyl]piperazine followed by treatment with 20% TFA in methylene chloride as described in Example 412C. $^1$H NMR (500 MHz, DMSO-$d_6$) δ 1.17-1.27 (m, 1H), 131-1.40 (m, 1H), 1.64-1.70 (m, 1H), 1.80-1.86 (m, 1H), 2.08-2.16(m, 1H), 2.61 (t, J=10 Hz, 1H), 2.68 (t, J=10 Hz, 1H), 3.03 (br d J=10 Hz, 1H), 3.06-3.18 (m, 2H), 3.25 (br d, J=10 Hz, 1H), 3.33 (m, 2H), 3.42-3.51 (m, 2H), 3.53-3.60 (m, 2H), 3.70-3.80 (m, 5H), 4.32-4.56 (m, 3H), 7.13.7.21 (m, 2H), 7.27 (d, J=9 Hz, 2H), 7.3 (d, J=7.5 Hz, 1H), 7.44 (t, J=7.5 Hz, 1H), 7.68-7.77 (m, 1H), 7.44-8.01 (m, 1H), 9.81 (br s, 1H); MS (ESI) *m/e* 676 $(M+H)^+$.

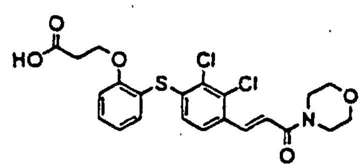

Example 443

(3-(3-Propioxy)phenyl) [2,3-dichloro-4-(E-((4-morpholino)carbonyl)ethenyl)phenyl]sulfide

β-Propiolactone (50 μL, 0.75 mmol) was added to a mixture of Example 405 (308 mg, 0.75 mmol), potassium *tert*-butoxide (750 mL, I M in THF), and THF (1.0 mL). After 18 h, the reaction was diluted with EtOAc, washed with 1 M aqueous HCl, washed with brine, dried ($MgSO_4$), filtered, and concentrated. Purification by preparative HPLC provided the title compound (72 mg, 20%) as a white solid. $^1$H NMR (DMSO-$d_6$, 300 MHz) δ 7.80 (d, *J=8.4* Hz, 1 H), 7.78 (d, *J*=15.8 Hz, I H), 7.52 (dt, *J*=8.8 Hz, *J*=1.7 Hz, 1H), 7.46 (dd, *J*=1.8 Hz, *J*=17 Hz, 1H); 7.23 (d, *J*=9.1 Hz, 1H), 7.22 (d, *J*=15.3 Hz, 1H), 7.08 (t, *J*=7.4 Hz, 1H), 6.58 (d, *J*=8.5 Hz, 1H), 4.22 (m, 2H), 4.05 (m, 2H), 3.66 (s, 2H), 3.58 (s, 6H); MS (APCI) *m/z* 482 $(M+H)^+$; 480 $(M-H)^-$.

Compounds that antagonize the interaction between ICAM-1 and LFA-1 can be identified, and their activities quantitated, using both biochemical and cell-based adhesion assays. A primary biochemical assay measures the ability of the compound in question to block the interaction between the integrin LFA-1 and its adhesion partner ICAM-1, as described below:

ICAM-1 / LFA-1 Biochemical Interaction Assay

In the biochemical assay, 100 μL of anti-LFA-1 antibody (ICOS Corporation) at a concentration of 5 μg/ml in Dulbecco's phosphate-buffered saline (D-PBS) is used to coat wells of a 96-well microtiter plate overnight at 4°C. The wells are then washed twice with wash buffer (D-PBS w/o $Ca^{++}$ or $Mg^{++}$, 0.05% Tween 20) and blocked by addition of 200 μL of D-PBS, 5% fish skin gelatin. Recombinant LFA-1 (100 μL of 0.7 μg/ml, ICOS Corporation) in D-PBS is then added to each well. Incubation continues for 1 hour at room temperature and the wells are washed twice with wash buffer. Serial dilutions of compounds being assayed as ICAM-1/LFA-1 antagonists, prepared as 10 mM stock solutions in dimethyl sulfoxide (DMSO), are diluted in D-PBS, 2mM $MgCl_2$, 1%. fish skin gelatin and 50 μL of each dilution added to duplicate wells. This is followed by addition of 50 μL of 0.8 μg/ml biotinylated recombinant ICAM- 1/Ig (ICOS Corporation) to the wells and the plates are incubated at room temperature for I hour. The wells are then washed twice with wash buffer and 100 μL of Europium-labeled Streptavidin (Wallac Oy) diluted 1:100 in Delfia assay buffer (Wallac Oy) are added to the wells. Incubation proceeds for 1 hour at room temperature. The wells are washed eight times with wash buffer and 100 μL of enhancement solution (Wallac Oy, cat. No. 1244-105) are added to each well. Incubation proceeds for 5 minutes with constant mixing. Time-resolved ffuorimetry measurements are made using the Victor 1420 Multilabel Counter (Wallac Oy) and the percent inhibition of each candidate compound is calculated using the following equation:

$$\% \text{ inhibition} = 100 \text{ X} \left\{ 1 - \frac{\text{average OD w/ compound minus background}}{\text{average OD w/o compound minus background}} \right\}$$

where "background" refers to wells that are not coated with anti-LFA-1 antibody.

Compounds of the present invention exhibit inhibitory activity In the above assay. % inhibition at 4 μM was demonstrated.

Biologically relevant activity of the compounds in this invention is confirmed using a cell-based adhesion assay, which measures their ability to block the adherence of JY-8 cells (a human EBV-transformed B cell line expressing LFA-1 on its surface) to immobilized ICAM-1, as follows:

ICAM-1 / JY-8 cell adhesion assay

For measurement of inhibitory activity in the cell-based adhesion assay, 96-well microtiter plates are coated with 70 μL of recombinant ICAM-1/Ig (ICOS Corporation) at a concentration of 5 μg/mL in D-PBS w/o $Ca^{++}$ or $Mg^{++}$ overnight at 4°C. The wells are then washed twice with D-PBS and blocked by addition of 200 μL of D-PBS, 5% fish skin gelatin by incubation for 1 hour at room temperature. Fluorescent tagged JY-8 cells (a human EBV-transformed B cell line expressing LFA-1 on its surface; 50 μL at 2 x $10^6$ cells/ml in RPMI 1640/1% fetal bovine serum) are added to the wells. For fluorescent labeling of JY-8 cells, 5 x $10^6$ cells washed once in RPMI 1640 are resuspended in 1 mL of RPMI 1640 containing 2 μM Calceiun AM (MolecularProbes), are incubated at 37°C for 30 minutes and washed once with RPMI-1640/ 1% fetal bovine serum. Dilutions of compounds to be assayed for ICAM-1/LFA-1 antagonistic activity are prepared in RPMI-1640/ 1% fetal bovine serum from 10mM stock solutions in DMSO and 50 μL are added to duplicate wells. Microtiter plates are incubated for 45 minutes at room temperatur and the wells are washed gently once with RPMI-1640/ 1% fetal bovine serum. Fluorescent intensity is measured in a fluorescent plate reader with an excitation wavelength at 485 nM and an emission wavelength at 530 nM. The percent inhibition of a candidate compound at a given concentration is calculated using the following equation:

$$\% \text{ inhibition} = 100 \text{ X} \left\{ 1 - \frac{\text{average OD w/ compound}}{\text{average OD w/o compound}} \right\}$$

and these concentration/inhibition data are used to generate dose response curves, from which $IC_{50}$ values are derived.

Compounds of the present invention exhibit blocking activity in the above assay. Inhibition at 4 μM was demonstrated.

Compounds of the present invention have been demonstrated to act via interaction with the integrin LFA-1, specifically by binding to the interaction domain (I-domain), which is known to be critical for the adhesion of LFA-1 to

a variety of cell adhesion molecules. As such, it is expected that these compounds should block the interaction of LFA-1 with other CAM's. This has in fact been demonstrated for the case of ICAM-3. Compounds of the present invention may be evaluated for their ability to block the adhesion of JY-8 cells (a human EBV-transformed B cell line expressing LFA-1 on its surface) to immobilized ICAM-3, as follows:

ICAM-3 / JY-8 cell adhesion assay

For measurement of inhibitory activity in the cell-based adhesion assay, 96-well microtiter plates are coated with 50 μL of recombinant ICAM-3/Ig (ICOS Corporation) at a concentration of 10 μg/mL in D-PBS w/o $Ca^{++}$ or $Mg^{++}$ overnight at 4°C. The wells are then washed twice with D-PBS, blocked by addition of 100 μL of D-PBS, 1% bovine serum albumin (BSA) by incubation for 1 hour at room temperature, and washed once with RPMI-1640 / 5% heat-inactivated fetal bovine serum (adhesion buffer). Dilutions of compounds to be assayed for ICAM-3/LFA-1 antagonistic activity are prepared in adhesion buffer from 10 mM stock solutions in DMSO and 100 μL are added to duplicate wells. JY-8 cells (a human EBV-transformed B cell line expressing LFA-1 on its surface; 100 μL at 0.75 x $10^6$ cells/ml in adhesion buffer) are then added to the wells. Microtiter plates are incubated for 30 minutes at room temperature; the adherent cells are then fixed with 50 μL of 14% glutaraldehyde/D-PBS and incubated for an additional 90 minutes. The wells are washed gently with $dH_2O$; 50 μL of $dH_2O$ is added, followed by 50 μL of 1% crystal violet. After 5 minutes the plates are washed 3X with $dH_2O$; 75 μL of $dH_2O$ and 225 μL of 95% EtOH are added to each well to extract the crystal violet from the cells. Absorbance is measured at 570 nM in an ELISA plate reader. The percent inhibition of a candidate compound is calculated using the following equation:

$$\% \text{ inhibition} = 100 \text{ X} \left\{ 1 - \frac{\text{average OD w/ compound}}{\text{average OD w/o compound}} \right\}$$

Compounds of the present invention exhibit blocking activity in the above assay. 100 % inhibition at 0.6 μM was demonstrated.

Additional JY-8 (ICAM/LFA-1) Cell Adhesion Assay Protocol

Reagents

- ICAM-I/Ig; ICOS
- D-PBS, Dulbecco's w/o Ca & Mg
- D-PBS, Dulbecco's *w/* Ca & Mg
- Blocking Solution: 1% non-fat dried milk in PBS w/o Ca & Mg
- RPMI 1640 media
- RPMI 1640 media with 1%FBS (RPMI-1%FBS)
- RPMI 1640 media with 50%FBS (RPMI-50%FBS)
- 1 mM Calcein AM, Molecular Probes, cat. C-1430 or C-3099
- DMSO
- JY-8 cells

Procedure

1. Coat plate (70ul/well) with 5ug/ml in D-PBS w/ Ca & Mg of ICAM-I/Ig. Cover and incubate overnight at 4°C.
2. Make compound and control dilutions using RPMI-1%FBS and RPMI-50%FBS as the diluents.
3. Decant ICAM-I/Ig coated plate(s), and wash 3X with D-PBS w/o Ca & Mg.
4. Block entire plate(s) with 150ul/well of Blocking solution. Cover and incubate for approximately **1** hour at room temperature.
**5.** Count the number of viable JY-8 cells using standard methodology. Need approximately 10-15 x10E6 cells per 96mw tray.

6. Wash cells 1 X in RPMI 1640 media without serum - centrifuging for 5 minutes at approximately 1400rpms. Remove supernate and resuspend cell pellet to 5x 10E6 cells per ml in RPMI 1640 media without serum.
7. Add 2ul of 1 mM Calcein AM for every l ml of cell suspension. Mix. Incubate for 30-60 minutes at 37 degrees C in a CO2 incubator (keeping cap of centrifuge tube loose for gas exchange).
8. Add approximately 10mls of RPMI-1%FBS, aliquot into two equal pools and centrifuge for 5 minutes at 1400rpms.
9. Remove supernate from each pool and resuspend each cell pellet to 2x10E6 cell per ml with RPMI-1 %FBS or RPMI-50%FBS.
10. Decant blocked 96mw plate(s) and wash 3X with D-PBS w/o Ca & Mg.
11. Add 50µl/well of each compound dilution or control. Add 50µl of Calcein labeled JY-8 cells to all wells. Centrifuge plate(s) briefly (2-5 seconds) at 100-150rpms. Cover and incubate for 30-60 minutes at 37 degrees C.
12. Gently wash wells 1X with approximately 150ul per well of PBS w/Ca & Mg. Remove all liquid from wells.
13. Read absorbence using reader with an excitation of 485/20 and an emission of 530/25.
14. Calculate % inhibition using the following equation:

$$\%\ \text{inhibition} = 100\ X \left\{ 1 - \frac{\text{average OD w/ compound}}{\text{average OD w/o compound}} \right\}$$

The ability of the compounds of this invention to treat arthritis can be demonstrated in a murine collagen-induced arthritis model according to the method of Kakimoto, et al., *Cell Immunol* 142: 326-337, 1992, in a rat collagen-induced arthritis model according to the method of Knoerzer, et al., *Toxicol Pathol* 25:13-19, 1997, in a rat adjuvant arthritis model according to the method of Halloran, et al., *Arthitis Rheum* 39: 810-819, 1996, in a rat streptococcal cell wall-induced arthritis model according to the method of Schimmer, et al., *J Immunol* 160: 1466-1477, 1998, or in a SCID-mouse human rheumatoid arthritis model according to the method of Oppenheimer-Marks et al., *J Clin Invest* 101: 1261-1272, 1998.

The ability of the compounds of this invention to treat Lyme arthritis can be demonstrated according to the method of Gross et al., *Science* 281, 703-706, 1998.

The ability of compounds of this invention to treat asthma can be demonstrated in a murine allergic asthma model according to the method of Wegner et al., *Science* 247:456-459, 1990, or in a murine non-allergic asthma model according to the method of Bloemen et al., *Am J Respir Crit Care Med* 153:521-529, 1996.

The ability of compounds of this invention to treat inflammatory lung injury can be demonstrated in a murine oxygen-induced lung injury model according to the method of Wegner et al., *Lung* 170:267.279, 1992, in a murine immune complex-induced lung injury model according to the method of Mulligan et al., *J Immunol* 154:1350-1363, 1995, or in a murine acid-induced lung injury model according to the method of Nagase, et al., *Am J Respir Crit Care Med* 154:504-510, 1996.

The ability of compounds of this invention to treat inflammatory bowel disease can be demonstrated in a rabbit chemical-induced colitis model according to the method of Bennet et al., *J Pharmacol Exp Ther* 280:988-1000, 1997.

The ability of compounds of this invention to treat autoimmune diabetes can be demonstrated in an NOD mouse model according to the method of Hasagawa et al., *Int Immunol* 6:831-838, 1994, or in a murine streptozotocin-induced diabetes model according to the method of Herrold et al., *Cell Immunol* 157:489-500, 1994.

The ability of compounds of this invention to treat inflammatory liver injury can de demonstrated in a murine liver injury model according to the method of Tanaka et al., *J Immunol* 151:5088-5095,1993.

The ability of compounds of this invention to treat inflammatory glomerular injury can be demonstrated in a rat nephrotoxic serum nephritis model according to the method of Kawasaki, et al., *J Immunol* 150: 1074-1083, 1993.

The ability of compounds of this invention to treat radiation-induced enteritis can be demonstrated in a rat abdominal irradiation model according to the method of Panes et al., *Gastroenterology* 108: 1761-1769, 1995.

The ability of compounds of this invention to treat radiation pneumonitis can be demonstrated in a murine pulmonary irradiation model according to the method of Hallahan et al., *Proc Natl Acad* Sci *U S A* 94:5432-6437, 1997.

The ability of compounds of this invention to treat reperfusion injury can be demonstrated in the isolated rat heart according to the method of Tamiya et al., *Immunopharmacology* 29(l): 53-63, 1995, or in the anesthetized dog according to the model of Hartman et al., *Cardiovasc Res* 30(1): 47-54, 1995.

The ability of compounds of this invention to treat pulmonary reperfusion injury can be demonstrated in a rat lung allograft reperfusion injury model according to the method of DeMeester et al., *Transplantation* 62(10): 1477-1485, 1996, or in a rabbit pulmonary edema model according to the method of Horgan et al., *Am J Physiol* 261(5): H1578-H1584, 1991.

The ability of compounds of this invention to treat stroke can be demonstrated in a rabbit cerebral embolism

stroke model according the method of Bowes et al., *Exp Neurol* 119(2): 215-219, 1993, in a rat middle cerebral artery ischemia-reperfusion model according to the method of Chopp et al., *Stroke* 25(4): 869-875,1994, or in a rabbit reversible spinal cord ischemia model according to the method of Clark et al., *Neurosurg* 75(4) : 623-627, 1991.

The ability of compounds of this invention to treat peripheral artery occlusion can be demonstrated in a rat skeletal muscle ischemia / reperfusion model according to the method of Gute et al., *Mol Cell Biochem* 179: 169-187, 1998.

The ability of compounds of this invention to treat graft rejection can be demonstrated in a murine cardiac allograft rejection model according to the method of Isobe et al., *Science* 255: 1125-1127, 1992, in a murine thyroid gland kidney capsule model according to the method of Talento et al., *Transplantation* 55: 418-422, 1993, in a cynomolgus monkey renal allograft model according to the method of Cosimi et al., *J Immunol* 144: 4604-4612, 1990, in a rat nerve allograft model according to the method of Nakao et al., *Muscle Nerve* 18: 93-102, 1995, in a murine skin allograft model according to the method of Gorczynski and Wojcik, *J Immunol* 152 : 2011-2019, 1994, in a murine corneal allograft model according to the method of He et al., *Opthalmol Vis Sci* 35: 3218-3225, 1994, or in a xenogeneic pancreatic islet cell transplantation model according to the method of Zeng et al., *Transplantation* 58:681-689, 1994.

The ability of compounds of this invention to treat graft-vs.-host disease (GVHD) can be demonstrated in a murine lethal GVHD model according to the method of Harning et al., *Transplantation* 52:842-845, 1991.

The ability of compounds of this invention to treat cancers can be demonstrated in a human lymphoma metastasis model (in mice) according to the method of Aoudjit et al., *J Immunol* 161:2333-2339, 1998.

## Claims

1. A compound of formula I

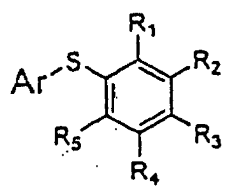

I

wherein $R_1$, $R_2$, $R_3$, $R_4$ and $R_5$ are independently selected from

a. hydrogen,
b. halogen,
c. alkyl,
d. haloalkyl,
e. alkoxy,
f. cyano,
g. nitro,
h. carboxaldehyde, and

with the proviso that at least one of $R_1$ or $R_3$ is a "*cis*-cinnamide" or a "*trans*-cinnamide", defined as

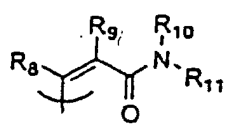

"*cis*-cinnamide"

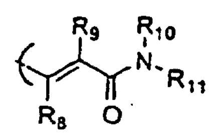

"*trans*-cinnamide",

wherein

$R_8$ and $R_9$ are independently selected from

a. hydrogen,
b. alkyl,
c. carboxy alkyl,
d. alkylaminocarbonyl alkyl, and
e. dialkylaminocarbonyl alkyl,

and
Rio and $R_{11}$ are independently selected from

a. hydrogen,
b. alkyl,
c. cycloalkyl,
d. alkoxycarbonylalkyl,
e. hydroxyalkyl,
f. an aryl group being a mono- or bicyclic carbocyclic ring system having one or two aromatic rings which can be fused to a cyclohexane, cyclohexene, cyclopentane or cyclopentene ring, and having substituents independently selected from alkyl, halogen, hydroxy and alkoxy,
g. heterocyclyl representing a 4-, 5-, 6- or 7-membered ring which contains one, two or three heteroatoms independently selected from nitrogen, oxygen and sulfur, which can be fused to one or two rings independently selected from an aryl ring as defined below, a cyclohexane ring, a cyclohexene ring, a cyclopentane ring, a cyclopentene ring or another monocyclic heterocyclic ring,
h. heterocyclylalkyl, where the heterocyclyl is as defined above,
i. heterocyclylamino, where the heterocyclyl is as defined above,
j. a heterocyclyl group as defined in g. above having substituents independently selected from alkyl, halogen, hydroxy, alkoxy, carboxy, carboxyalkyl and alkoxycarbonyl,
k. a heterocyclylalkyl group as defined in h. above having substituents independently selected from alkyl, halogen, hydroxy, alkoxy, carboxy, carboxyalkyl and alkoxycarbonyl, or

where $NR_{10}R_{11}$ is a heterocyclyl or substituted heterocyclyl group where the heterocyclyl group is as defined g. above and the substituents are independently selected from

1) alkyl,
2) alkoxy,
3) alkoxyalkyl,
4) cycloalkyl,
5) an aryl group being a mono- or bicyclic carbocyclic ring system having one or two aromatic rings which can be fused to a cyclohexane, cyclohexene, cyclopentane or cyclopentene ring,
6) a heterocyclyl group as defined above,
7) heterocyclylcarbonyl, where the heterocyclyl is as defined above,
8) heterocyclylalkylaminocarbonyl, where the heterocyclyl is as defined above,
9) hydroxy,
10) hydroxyalkyl,
11) hydroxyalkoxyalkyl,
12) carboxy,
13) carboxyalkyl,
14) carboxycarbonyl,
15) carboxaldehyde,
16) alkoxycarbonyl,
17) arylalkoxycarbonyl, where the aryl is as defined above,
18) aminoalkyl,
19) aminoalkanoyl,
20) carboxamido,
21) alkoxycarbonylalkyl,
22) carboxamidoalkyl,
23) cyano,
24) tetrazolyl,

25) tetrazolyl substituted with alkoxy, alkyl, halogen, hydroxy, carboxy, carboxyalkyl or alkoxycarbonyl,
26) alkanoyl,
27) hydroxyalkanoyl,
28) alkanoyloxy,
29) alkanyolamino,
30) alkanoyloxyalkyl,
31) alkanoylaminoalkyl,
32) sulfonate,
33) alkylsulfonyl,
34) alkysulfonylaminocarbonyl,
35) arylsulfonylaminocarbonyl, where the aryl is as defined above, and
36) heterocyclylsulfonylaminocarbonyl, where the heterocyclyl group is as defined above,

and wherein Ar is a substituted aryl or substituted heteroaryl group, which has one or two aromatic rings and which can be fused to a cyclohexane, cyclohexene, cyclopentane or cyclopentene ring, where substitutions are independently selected from

a. a heterocyclyl group as defined above having substituents independently selected from alkyl, halogen, hydroxyl, alkoxy, carboxy, carboxyalkyl and alkoxycarbonyl,
b. a heterocyclylalkyl group as defined above having substituents independently selected from carboxy, carboxyalkyl and alkoxycarbonyl,
c. carboxyalkoxy,
d. carboxythioalkoxy,
e. carboxycycloalkoxy,
f. thioalkyl; and
g. carboxyalkylamino;

or a pharmaceutically acceptable salt thereof.

**2.** A compound according to claim 1, wherein R1, is a *"cis*-cinnamide" or a *"trans*-cinnamide", and $R_3$ is hydrogen.

**3.** A compound according to claim 1 wherein $R_3$ is a "cis-cinnamide" or a "trans-cinnamide", and $R_1$ is hydrogen.

**4.** A compound according to claim 1 wherein $R_3$ is a "cis-cinnamide" or a *"trans*-cinnamide", and $R_1$, $R_8$, and $R_9$ are hydrogen.

**5.** A compound according to claim 4 wherein $R_3$ is a "cis-cinnamide".

**6.** A compound according to claim 4 wherein $R_3$ is a "trans-cinnamide".

**7.** A compound according to claim 1 wherein $R_3$ is a "cis-cinnamide" or a *"trans*-cinnamide", $R_1$, $R_2$, and $R_4$ are each independently hydrogen or alkyl; and $R_5$ is selected from halogen, haloalkyl, and nitro.

**8.** A compound according to claim 4 wherein $R_{10}$ and $R_{11}$ are each independently selected from hydrogen, alkyl, cycloalkyl, alkoxycarbonylalkyl, hydroxyalkyl, and heterocyclylalkyl.

**9.** A compound according to claim 4 wherein $NR_{10}R_{11}$ is heterocyclyl or substituted heterocyclyl.

**10.** A compound according to claim 1 wherein Ar is selected from substituted phenyl, 1,3-benzimidazol-2-one, 1,4-benzodioxane, 1,3-benzodioxole, 1-benzopyr-2-en-4-one, indole, isatin, 1,3-quinazolin-4-one, and quinoline.

**11.** A compound according to claim 1 wherein Ar is substituted benzodioxan.

**12.** A compound according to claim 1 wherein $R_3$ is a "trans-cinnamide; and Ar is selected from 1,3-benzimidazol-2-one, 1,4-benzodioxane, 1,3-benzodioxole, 1-benzopyr-2-en-4-one, indole, isatin, phenyl, 1,3-quinazolin-4-one, and quinoline.

**13.** A compound according to claim 1 wherein $R_{10}$ and $R_{11}$ are independently selected from hydrogen, alkyl, cycloalkyl,

alkoxycarbonylalkyl, hydroxyalkyl, and heterocyclylalkyl.

**14.** A compound according to claim 1 wherein $NR_{10}R_{11}$ is heterocyclyl or substituted heterocyclyl.

**15.** The compound of claim 1 with the proviso that when three of $R_1$, $R_2$, $R_4$, and R5 are hydrogen, then the remaining $R_1$, $R_2$, $R_4$, or $R_5$, is not carboxyl, 5-tetrazolyl, hydroxymethyl or carboxyl derivatized in the form of a pharmaceutically acceptable ester.

**16.** A compound according to claim 1 selected from the group consisting of:

(3-(1-(3-Carboxypiperidinyl)phenyl)[2,3-dichloro-4-(E-((1,2,5,6-tetrahydropyridin-1-yl)carbonyl)ethenyl)phenyl]sulfide;
[3-(3-Carboxylpiperidin-1-yl)phenyl] [2,3-dichloro-4-(E-[(4-carboxypiperidin-1-yl)carbonyl]ethenyl)phenyl] sulfide;
[3-(4-Carboxylpiperidin-1-yl)phenyl] [2,3-dichloro-4-(E-[(1,2,3,6-tetrahydropyridin-1-yl)carbonyl]ethenyl)phenyl] sulfide;
[3-(4-Carboxylpiperidinyl)phenyl] [2,3-dichloro-4-(E-[(4-morpholinyl)carbonyl]ethenyl)phenyl] sulfide;
3-(3-Carboxypiperidin-1-yl)phenyl] [2,3-dichloro-4-(E-[(4-morpholinyl)carbonyl]ethenyl)phenyl] sulfide;
[3-(4-Carboxypiperidin-1-yl)phenyl] [2,3-dichloro-4-(E-((4-hydroxypiperidin-1-yl)carbonyl)ethenyl)phenyl] sulfide;
(2-Glycoxyphenyl) [2,3-dichloro-4-(E-((4-morpholino)carbonyl)ethenyl)phenyl]sulfide;
(2-(4-Butyroxy)phenyl)[2,3-dichloro-4-(E-((4-morpholino)carbonyl)ethenyl)phenyl]sulfide;
[3-(4-Carboxypiperidin-1-yl)phenyl] [2,3-dichloro-4-(E-((4-hydroxyethylpiperazin-1-yl)carbonyl)ethenyl)phenyl] sulfide;
[3-(4-Carboxypiperidin-1-yl)phenyl] [2,3-dichloro-4-(E-((4-furoylpiperazin-1-yl)carbonyl)ethenyl)phenyl] sulfide;
[3-(4-Carboxypiperidin-1-yl)phenyl] [2,3-dichloro-4-(E-((pyrrolidin-1-yl)carbonyl)ethenyl)phenyl] sulfide;
[3-(4-Carboxypiperidin-1-yl)phenyl] [2,3-dichloro-4-(E-((diethylaminocarbonyl)ethenyl)phenyl] sulfide;
[3-(4-Carboxypiperidin-1-yl)phenyl] [2,3-dichloro-4-(E-((4-ethylpiperazinyl)carbonyl)ethenyl)phenyl] sulfide;
[3-(4-Carboxypiperidin-1-yl)phenyl] [2,3-dichloro-4-(E-((4-(aminocarbonyl)piperidin-1-yl)carbonyl)ethenyl) phenyl] sulfide; [3-(4-Carboxypiperidin-1-yl)phenyl] [2,3-dichloro-4-(E-((4-(2-(ethoxyethyl)piperidin-1-yl)carbonyl)ethenyl)phenyl] sulfide;
[3-(4-Carboxypiperidin-1-yl)phenyl] [2,3-bis(trifluoromethyl)-4-(E-((4-morpholino)carbonyl)ethenyl)phenyl] sulfide;
[3-(4-Butyroxy)phenyl] [2,3-dichloro-4-(E-((4-morpholino)carbonyl)ethenyl)phenyl]sulfide;
[3-(4-Carboxypiperidin-1-yl)phenyl] [2,3-bis(trifluoromethyl-4-(E-((4-hydroxypiperidin-1-yl)carbonyl)ethenyl) phenyl] sulfide;
(3-(4-Carboxypiperidin-1-yl)phenyl] [2,3-bis(trifluoromethyl-4-(E-((1,2,5,6-tetrahydropyridin-1-yl)carbonyl) ethenyl)phenyl] sulfide;
[2-((4-Carboxy)butyloxy)phenyl] [2,3-dichloro-4-(E-((4-morpholino)carbonyl)ethenyl)phenyl]sulfide;
(2-Glycoxyphenyl) [ 2,3-bis(trifluoromethyl)-4-(E-((4-morpholino)carbonyl) eth enyl) phenyl ]sulfide;
(2-(4-Butyroxy)phenyl)[ 2,3-bis(trifiuoromethyl)-4-(E-((4-morpholino)carbonyl)ethenyl)phenyl]sulfide;
[3-(4-Carboxypiperidin-1-yl)phenyl] [2,3-bis(tritluoromethyl)-4-(E-((bis-(2-ethoxyethyl)amino)carbonyl)ethenyl)phenyl] sulfide;
[3-(4-Carboxypiperidin-1-yl)phenyl] [2,3-bis-(trifluoromethyl)-4-(E-((bis-(2-hydroxypropyl)amino)carbonyl) ethenyl)phenyl] sulfide;
[3-(4-Carboxypiperidin-1-yl)phenyl] [2,3-bis-(trifluoromethyl)-4-(E-((piperazin-1-yl)carbonyl)ethenyl)phenyl] sulfide;
(3-(4-Butyroxy)phenyl)[ 2,3-bis(trifluoromethyl)-4-(E-((4-morpholino)carbonyl)ethenyl)phenyl]sulfide;
[2-(3-Carboxypiperidin-1-yl)phenyl] [2,3-dichloro-4-(E-((4-(2-hydroxyethyl)piperazin-1-yl)carbonyl)ethenyl) phenyl] sulfide;
[2-(3-Carboxypiperidin-1-yl)phenyl] [2,3-bis(trifluoromethyl)-4-(E-((1,2,5,6-tetrahydropyridin-1-yl)carbonyl) ethenyl)phenyl] sulfide;
[2-(3-Carboxypiperidin-1-yl)phenyl] [2,3-bis(trifluoromethyl)-4-(E-((4-(2-hydroxyethyl)piperazin-1-yl)carbonyl) ethenyl)phenyl] sulfide;
[2-(3-Carboxypiperidin-1-yl)phenyl] [2,3-bis(triftuoromethyl)-4-(E-((4-(2-(hydroxyethoxy)ethyl)piperazin-1-yl) carbonyl)ethenyl)phenyl] sulfide;
(3-(3-Propioxy)phenyl) [2,3-dichloro-4-(E-((4-morpholino)carbonyl)ethenyl)phenyl]sulfide;
[3-(4-Carboxypiperidin-1-yl)phenyl] [2,3-dichloro-4-(E-[(4-(dimethylaminosulfanoyl)piperazin-1-yl)carbonyl]

ethenyl)phenyl] sulfide;
[3-(4-Carboxypiperidin-1-yl)phenyl] [2,3-dichloro-4-(E-((4-((trif)uoromethylsulfonyl)piperazin-1-yl)carbonyl) ethenyl)phenyl] sulfide;
[3-(4-Carboxypiperidin-1-yl)phenyl] [2,3-dichloro-4-(E-((4-((methylsulfonyl)piperazin-1-yl)carbonyl)ethenyl) phenyl] sulfide;
(3-(4-carboxypiperidin-1-yl)phenyl)[2,3-dichloro-4-(E-((S-oxothiomorpholin-1-yl)carbonyl)ethenyl)phenyl] sulfide;
[2-(3-Carboxypiperidin-1-yl)phenyl] [2,3-dichloro-4-(E-[(3-(2-pyrrolidinon-1-yl)propylaminocarbonyl)ethenyl) phenyl] sulfide; and
[2-(3-Carboxypiperidin-1-yl)phenyl] [2,3-bis(trifluoromethyl)-4-(E-[(3-(2-pyrrolidinon-1-yl)propylaminocarbonyl)ethenyl)phenyl] sulfide.

**17.** A compound selected from the group consisting of:

(2-Methoxyphenyl) [2,3-dichloro-4-(E-(((4-hydroxylaminocarbonyl)piperidin-1-yl)carbonyl)ethenyl)phenyl] sulfide;
(2-Methoxyphenyl) [2,3-dichloro-4-(E-((N-carboxymethyl-N-phenylamino)carbonyl)ethenyl)phenyl] sulfide;
(2-Methoxyphenyl) [3-chloro-6-hydroxy-4-(E-((3-carboxypiperidin-1-yl)carbonyl)ethenyl)phenyl]sulfide;
(2-Methoxyphenyl) [2,3-dichloro-4-(E-(4-((1-(2-phenyl-1-carboxyethyl)amino)carbonyl)piperidin-1-yl)carbonyl)ethenyl)phenyl] sulfide;
(2-Methoxyphenyl) [2,3-dichloro-4-(E-(4-((1-(2-hydroxy-1-carboxyethyl)amino)carbonyl)piperidin-1-yl)carbonyl)ethenyl)phenyl]sulfide;
(3-(4-Pyrrolidin-1-yl)piperidin-1-yl)phenyl) [2,3-dichloro-4-(E-(((3-(2-pyrrolidinon-1-yl)propylamino)carbonyl) ethenyl)phenyl]sulfide;
[3-(4-(Spiro-2,2-dioxolanyl)piperidin-1-yl)phenyl] [2,3-dichloro-4-(E-((4-morpholinyl)carbonyl)ethenyl)phenyl] sulfide;
(2-(2-Carboxy)ethenyl)phenyl) [2,3-dichloro-4-(E-((4-morpholinyl)carbonyl)ethenyl)phenyl]sulfide;
[2-(4-Acetylpiperazin-1-yl)phenyl] [2,3-dichloro-4-(E-[(4-carboxypiperidin-1-yl)carbonyl]ethenyl)phenyl] sulfide; and
(2-Methoxyphenyl) [2,3-dichloro-4-(E-((((4-carboxyphenyl)methyl)amino)carbonyl)ethenyl)phenyl]sulfide.

**18.** A pharmaceutical composition comprising a compound of claim 1 or 17 in a pharmaceutically-acceptable carrier.

**19.** Use of a compound of claim 1, 16 or 17 for preparing a medicament for inhibiting inflammation.

**20.** Use of a compound of claim 1, 16 or 17 for preparing a medicament for suppressing immune response.

**21.** A compound of formula II

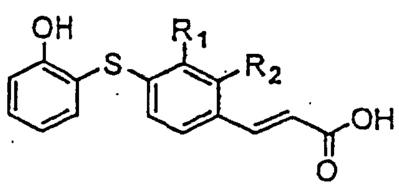

II

wherein $R_1$ and $R_2$ are independently selected from

i. hydrogen,

j. halogen,

k. alkyl,

l. haloalkyl,

m. alkoxy,

n. cyano,

o. nitro, and

p. carboxaldehyde.

**22.** A process for preparing a compound of formula II

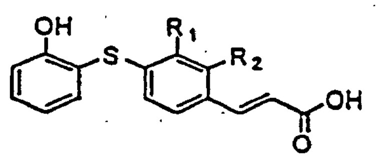

II

which comprises:

a) reacting a compound of formula II'

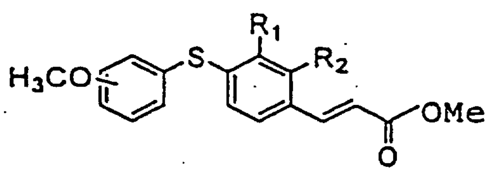

II'

with lithium hydroxide, and

b) cleaving the resulting methyl ether ,

where $R_1$ and $R_2$ are as defined in claim 21.

## Patentansprüche

**1.** Verbindung der Formel I

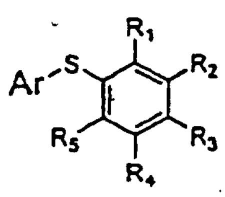

I

wobei $R_1$, $R_2$, $R_3$, $R_4$ und $R_5$ unabhängig ausgewählt sind aus

a. Wasserstoff;
b. Halogen;

c. Alkyl;
d. Halogenalkyl;
e. Alkoxy;
f. Cyano;
g. Nitro;
h. Carboxaldehyd;

mit der Maßgabe, dass wenigstens einer der Reste $R_1$ oder $R_3$ ein "cis-Cinnamid" oder ein "trans-Cinnamid" ist, die definiert sind als

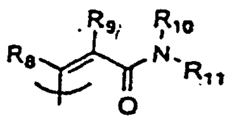
"cis-Cinnamid"

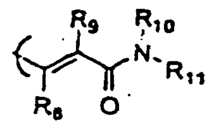
"trans-Cinnamid"

wobei
$R_8$ und $R_9$ unabhängig ausgewählt sind aus

a. Wasserstoff;
b. Alkyl;
c. Carboxyalkyl;
d. Alkylaminocarbonylalkyl; und
e. Dialkylaminocarbonylalkyl; und

$R_{10}$ und $R_{11}$ unabhängig ausgewählt sind aus

a. Wasserstoff;
b. Alkyl;
c. Cycloalkyl;
d. Alkoxycarbonylalkyl;
e. Hydroxyalkyl;
f. einer Arylgruppe, bei der es sich um ein mono- oder bicyclisches carbocyclisches Ringsystem handelt, das einen oder zwei aromatische Ringe aufweist, die mit einem Cyclohexan-, Cyclohexen-, Cyclopentanoder Cyclopentenring kondensiert sein können und Substituenten aufweist, die unabhängig aus Alkyl, Halogen, Hydroxy und Alkoxy ausgewählt sind;
g. Heterocyclyl, das einen vier-, fünf-, sechs- oder siebengliedrigen Ring darstellt, der ein, zwei oder drei Heteroatome enthält, die unabhängig aus Stickstoff, Sauerstoff und Schwefel ausgewählt sind, und der mit einem oder zwei Ringen kondensiert sein kann, die unabhängig ausgewählt sind aus einem Arylring, wie er unten definiert ist, einem Cyclohexanring, einem Cyclohexenring, einem Cyclopentanring, einem Cyclopentenring oder einem anderen monocyclischen heterocyclischen Ring;
h. Heterocyclylalkyl, wobei das Heterocyclyl wie oben definiert ist;
i. Heterocyclylamino, wobei das Heterocyclyl wie oben definiert ist;
j. einer Heterocyclylgruppe, wie sie oben unter g. definiert ist und die Substituenten aufweist, die unabhängig aus Alkyl, Halogen, Hydroxy, Alkoxy, Carboxy, Carboxyalkyl und Alkoxycarbonyl ausgewählt sind;
k. einer Heterocyclylalkylgruppe, wie sie oben unter h. definiert ist und die Substituenten aufweist, die unabhängig aus Alkyl, Halogen, Hydroxy, Alkoxy, Carboxy, Carboxyalkyl und Alkoxycarbonyl ausgewählt sind; oder

wobei $NR_{10}R_{11}$ eine Heterocyclyl- oder substituierte Heterocyclylgruppe ist, wobei die Heterocyclylgruppe wie oben unter g. definiert ist und die Substituenten unabhängig ausgewählt sind aus

1) Alkyl;
2) Alkoxy;
3) Alkoxyalkyl;
4) Cycloalkyl;

5) einer Arylgruppe, bei der es sich um ein mono- oder bicyclisches carbocyclisches System handelt, das einen oder zwei aromatische Ringe aufweist, die mit einem Cyclohexan-, Cyclohexen-, Cyclopentan- oder Cyclopentenring kondensiert sein können;
6) einer Heterocyclylgruppe, wie sie oben definiert ist;
7) Heterocyclylcarbonyl, wobei das Heterocyclyl wie oben definiert ist;
8) Heterocyclylalkylaminocarbonyl, wobei das Heterocyclyl wie oben definiert ist;
9) Hydroxy;
10) Hydroxyalkyl;
11) Hydroxyalkoxyalkyl;
12) Carboxy;
13) Carboxyalkyl;
14) Carboxycarbonyl;
15) Carboxaldehyd;
16) Alkoxycarbonyl;
17) Arylalkoxycarbonyl, wobei das Aryl wie oben definiert ist;
18) Aminoalkyl;
19) Aminoalkanoyl;
20) Carboxamido;
21) Alkoxycarbonylalkyl;
22) Carboxamidoalkyl;
23) Cyano;
24) Tetrazolyl;
25) Tetrazolyl, das mit Alkoxy, Alkyl, Halogen, Hydroxy, Carboxy, Carboxyalkyl oder Alkoxycarbonyl substituiert ist;
26) Alkanoyl;
27) Hydroxyalkanoyl;
28) Alkanoyloxy;
29) Alkanoylamino;
30) Alkanoyloxyalkyl;
31) Alkanoylaminoalkyl;
32) Sulfonat;
33) Alkylsulfonyl;
34) Alkylsulfonylaminocarbonyl;
35) Arylsulfonylaminocarbonyl, wobei das Aryl wie oben definiert ist; und
36) Heterocyclylsulfonylaminocarbonyl, wobei die Heterocyclylgruppe wie oben definiert ist; und

wobei Ar eine substituierte Aryl- oder substituierte Heteroarylgruppe ist, die einen oder zwei aromatische Ringe aufweist, welche mit einem Cyclohexan-, Cyclohexen-, Cyclopentan- oder Cyclopentenring kondensiert sein können, wobei Substituenten unabhängig ausgewählt sind aus:

a. einer Heterocyclylgruppe, wie sie oben definiert ist und die Substituenten aufweist, die unabhängig aus Alkyl, Halogen, Hydroxy, Alkoxy, Carboxy, Carboxyalkyl und Alkoxycarbonyl ausgewählt sind;
b. einer Heterocyclylalkylgruppe, wie sie oben definiert ist und die Substituenten aufweist, die unabhängig aus Carboxy, Carboxyalkyl und Alkoxycarbonyl ausgewählt sind;
c. Carboxyalkoxy;
d. Carboxythioalkyl;
e. Carboxycycloalkoxy;
f. Thioalkyl; und
g. Carboxyalkylamino;

oder ein pharmazeutisch annehmbares Salz davon.

**2.** Verbindung gemäß Anspruch 1, wobei $R_1$ ein "cis-Cinnamid" oder "trans-Cinnamid" ist und $R_3$ Wasserstoff ist.

**3.** Verbindung gemäß Anspruch 1, wobei $R_3$ ein "cis-Cinnamid" oder "trans-Cinnamid" ist und $R_1$ Wasserstoff ist.

**4.** Verbindung gemäß Anspruch 1, wobei $R_3$ ein "cis-Cinnamid" oder "trans-Cinnamid" ist und $R_1$, $R_8$ und $R_9$ Wasserstoff sind.

**5.** Verbindung gemäß Anspruch 4, wobei $R_3$ ein "cis-Cinnamid" ist.

**6.** Verbindung gemäß Anspruch 4, wobei $R_3$ ein "trans-Cinnamid" ist.

**7.** Verbindung gemäß Anspruch 1, wobei $R_3$ ein "cis-Cinnamid" oder "trans-Cinnamid" ist, $R_1$, $R_2$ und $R_4$ unabhängig Wasserstoff oder Alkyl sind und $R_5$ aus Halogen, Halogenalkyl und Nitro ausgewählt sind.

**8.** Verbindung gemäß Anspruch 4, wobei $R_{10}$ und $R_{11}$ jeweils unabhängig aus Wasserstoff, Alkyl, Cycloalkyl, Alkoxycarbonylalkyl, Hydroxyalkyl und Heterocyclylalkyl ausgewählt sind.

**9.** Verbindung gemäß Anspruch 4, wobei $NR_{10}R_{11}$ Heterocyclyl oder substituiertes Heterocyclyl ist.

**10.** Verbindung gemäß Anspruch 1, wobei Ar aus substituiertem Phenyl, 1,3-Benzimidazol-2-on, 1,4-Benzodioxan, 1,3-Benzodioxol, 1-Benzopyr-2-en-4-on, Indol, Isatin, 1,3-Chinazolin-4-on und Chinolin ausgewählt ist.

**11.** Verbindung gemäß Anspruch 1, wobei Ar substituiertes Benzodioxan ist.

**12.** Verbindung gemäß Anspruch 1, wobei $R_3$ ein "trans-Cinnamid" ist und Ar aus 1,3-Benzimidazol-2-on, 1,4-Benzodioxan, 1,3-Benzodioxol, 1-Benzopyr-2-en-4-on, Indol, Isatin, Phenyl, 1,3-Chinazolin-4-on und Chinolin ausgewählt ist.

**13.** Verbindung gemäß Anspruch 1, wobei $R_{10}$ und $R_{11}$ unabhängig aus Wasserstoff, Alkyl, Cycloalkyl, Alkoxycarbonylalkyl, Hydroxyalkyl und Heterocyclylalkyl ausgewählt sind.

**14.** Verbindung gemäß Anspruch 1, wobei $NR_{10}R_{11}$ Heterocyclyl oder substituiertes Heterocyclyl ist.

**15.** Verbindung gemäß Anspruch 1, mit der Maßgabe, dass dann, wenn drei der Reste $R_1$, $R_2$, $R_4$ und $R_5$ Wasserstoff sind, es sich bei den übrigen Resten $R_1$, $R_2$, $R_4$ oder $R_5$ nicht um Carboxy, 5- Tetrazolyl, Hydroxymethyl oder Carboxy, das in Form eines pharmazeutisch annehmbaren Esters derivatisiert ist, handelt.

**16.** Verbindung gemäß Anspruch 1, die aus der Gruppe ausgewählt ist, die aus folgenden besteht:

(3-(1-(3-Carboxypiperidinyl)phenyl))[2,3-dichlor-4-(E-((1,2,5,6-tetrahydropyridin-1-yl)carbonyl)ethenyl)phenyl]sulfid;
[3-(3-Carboxylpiperidin-1-yl)phenyl][2,3-dichlor-4-(E-[(4-carboxypiperidin-1-yl)carbonyl]ethenyl)phenyl]sulfid;
[3-(4-Carboxylpiperidin-1-yl)phenyl][2,3-dichlor-4-(E-[(1,2,3,6-tetrahydropyridin-1-yl)carbonyl]ethenyl)phenyl]sulfid;
[3-(4-Carboxylpiperidinyl)phenyl][2,3-dichlor-4-(E-[(4-morpholinyl)carbonyl]ethenyl)phenyl]sulfid;
3-(3-Carboxypiperidin-1-yl)phenyl][2,3-dichlor-4-(E-[(4-morpholinyl)-carbonyl]ethenyl)phenyl]sulfid;
[3-(4-Carboxypiperidin-1-yl)phenyl)[2,3-dichlor-4-(E-((4-hydroxypiperidin-1-yl)carbonyl)ethenyl)phenyl)sulfid;
(2-Glycoxyphenyl)[2,3-dichlor-4-(E-((4-morpholino)carbonyl)ethenyl)-phenyl]sulfid;
(2-(4-Butyroxy)phenyl)[2,3-dichlor-4-(E-((4-morpholino)carbonyl)ethenyl)phenyl]sulfid;
[3-(4-Carboxypiperidin-1-yl)phenyl] [2,3-dichlor-4-(E-((4-hydroxyethylpiperazin-1-yl)carbonyl)ethenyl)phenyl] sulfid;
[3-(4-Carboxypiperidin-1-yl)phenyl][2,3-dichlor-4-(E-((4-furoylpiperazin-1 -yl) carbonyl)ethenyl) phenyl]sulfid;
[3-(4-Carboxypiperidin-1-yl)phenyl][2,3-dichlor-4-(E-((pyrrolidin-1-yl)-carbonyl)ethenyl)phenyl]sulfid;
[3-(4-Carboxypiperidin-1-yl)phenyl) [2,3-dichlor-4-(E-((diethylaminocarbonyl)ethenyl)phenyl]sulfid;
[3-(4-Carboxypiperidin-1-yl)phenyl] [2,3-dichlor-4-(E-((4-ethylpiperazinyl)ca rbonyl)ethenyl)phenyl]sulfid;
[3-(4-Carboxypiperidin-1-yl)phenyl] [2,3-dichlor-4-(E-((4-(aminocarbonyl)piperidin-1-yl)carbonyl)ethenyl)phenyl]sulfid;
[3-(4-Carboxypiperidin-1-yl)phenyl)[2,3-dichlor-4-(E-((4-(2-ethoXyethyl)-piperidin-1-yl)carbonyl)ethenyl)phenyl]sulfid;
[3-(4-Carboxypiperidin-1-yl)phenyl][2,3-bis(trifluormethyl)-4-(E-((4-morpho lino) ca rbonyl)ethenyl) phenyl] sulfid;
[3-(4-Butyroxy)phenyl)[2,3-dichlor-4-(E-((4-morpholino)carbonyl)ethenyl)phenyl]sulfid;
[3-(4-Carboxypiperidin-1-yl)phenyl][2,3-bis(trifluormethyl-4-(E-((4-hydroxypiperidin-1-yl)carbonyl)ethenyl)

phenyl]sulfid;
[3-(4-Carboxypiperidin-1-yl)phenyl][2,3-bis(trifluormethyl-4-(E-((1,2,5,6-tetrahydropyridin-1-yl)carbonyl)ethenyl)phenyl]sulfid;
[2-((4-Carboxy)butyloxy)phenyl][2,3-dichlor-4-(E-((4-morpholino)carbonyl)ethenyl)phenyl]sulfid;
(2-Glycoxyphenyl)[2,3-bis(trifluormethyl)-4-(E-((4-morpholino)carbonyl)-ethenyl)phenyl]sulfid;
2(2-(4-Butyroxy)phenyl)[2,3-bis(trifluormethyl)]-4-(E-((4-morpholino)carbonyl)ethenyl) phenyl ]sulfid;
[3-(4-Carboxypiperidin-1-yl)phenyl][2,3-bis(trifluormethyl)-4-(E-((bis-(2-ethoxyethyl)amino)carbonyl)ethenyl) phenyl]sulfid;
[3-(4-Carboxypiperidin-1-yl)phenyl][2,3-bis(trifluormethyl)-4-(E-((bis-(2-hydroxypropy))amino)carbonyl)ethenyl)phenyl]sulfid;
[3-(4-Carboxypiperidin-1-yl)phenyl] [2,3-bis(trifluormethyl)-4-(E-((piperazin-1-yl)carbonyl)ethenyl)phenyl]sulfid;
(3-(4-Butyroxy)phenyl)[2,3-bis(trifluormethyl)-4-(E-((4-morpholino)carbonyl)ethenyl)phenyl]su!fid;
[2-(3-Carboxypiperidin-1-yl)phenyl][2,3-dichlor-4-(E-((4-(2-hydroxyethyl)piperazin-1-yl)carbonyl)ethenyl) phenyl]sulfid;
[2-(3-Carboxypiperidin-1-yl)phenyl][2,3-bis(trifluormethyl)-4-(E-((1,2,5,6-tetrahydropyridin-1-yl)carbonyl) ethenyl)phenyl]sulfid;
[2-(3-Carboxypiperidin-1-yl)phenyl][2,3-bis(trifluormethyl)-4-(E-((4-(2-hydroxyethyl)piperazin-1-yl)carbonyl) ethenyl)phenyl]sulfid;
[2-(3-Carboxypiperidin-1-yl)phenyl][2,3-bis(trifluormethyl)-4-(E-((4-(2-(hydroxyethoxy)ethyl)piperazin-1-yl) carbonyl)ethenyl)phenyl]sulfid;
(3-(3-Propoxy)phenyl)[2,3-dichlor-4-(E-((4-morpholino)carbonyl)ethenyl)phenyl]sulfid;
[3-(4-Carboxypiperidin-1-yl)phenyl] [2,3-dichlor-4-(E-[(4-(dimethylaminosulfanoyl)piperazin-1-yl)carbonyl] ethenyl)phenyl]sulfid;
[3-(4-Carboxypiperidin-1-yl)phenyl][2,3-dichlor-4-(E-((4-((trifluormethylsulfonyl)piperazin-1-yl)carbonyl)ethenyl)phenyl]sulfid;
[3-(4-Carboxypiperidin-1-yl)phenyl][2,3-dichlor-4-(E-((4-((methylsulfonyl)piperazin-1-yl)carbonyl)ethenyl) phenyl]sulfid;
(3-(4-Carboxypiperidin-1-yl)phenyl)[2,3-dichlor-4-(E-((S-oxothiomorpholin-1-yl)carbonyl)ethenyl)phenyl]sulfid;
[2-(3-Carboxypiperidin-1-yl)phenyl][2,3-dichlor-4-(E-[(3-(2-pyrrolidinon-1-yl)propylaminocarbonyl)ethenyl) phenyl]sulfid; und
[2-(3-Carboxypiperidin-1-yl)phenyl][2,3-bis(trifluormethyl)-4-(E-[(3-(2-pyrrolidinon-1-yl)propylaminocarbonyl) ethenyl)phenyl]sulfid.

**17.** Verbindung, die aus der Gruppe ausgewählt ist, die aus folgenden besteht:

(2-Methoxyphenyl)[2,3-dichlor-4-(E-(((4-hydroxylaminocarbonyl)piperidin-1-yl)carbonyl)ethenyl)phenyl]sulfid;
(2-Methoxyphenyl)[2,3-dichlor-4-(E-((N-carboxymethyl-N-phenylamino)-carbonyl)ethenyl)phenyl]sulfid;
(2-Methoxyphenyl)[3-chlor-6-hydroxy-4-(E-((3-carboxypiperidin-1-yl)carbonyl)ethenyl)phenyl]sulfid;
(2-Methoxyphenyl)[2,3-dichlor-4-(E-(4-((1-(2-phenyl-1-carboxyethyl)-amino)carbonyl)piperidin-1-yl)carbonyl) ethenyl)phenyl]sulfid;
(2-Methoxyphenyl)[2,3-dichlor-4-(E-(4-((1-(2-hydroxy-1-carboxyethyl)-amino)carbonyl)piperidin-1-yl)carbonyl)ethenyl)phenyl]sulfid;
(3-(4-Pyrrolidin-1-yl)piperidin-1-yl)phenyl)[2,3-dichlor-4-(E-(((3-(2-pyrrolidinon-1-yl)propylamino)carbonyl) ethenyl)phenyl]sulfid;
[3-(4-(Spiro-2,2-dioxolanyl)piperidin-1-yl)phenyl][2,3-dichlor-4-(E-((4-morpholinyl)carbonyl)ethenyl)phenyl] sulfid;
(2-(2-Carboxy)ethenyl)phenyl)[2,3-dichlor-4-(E-((4-morpholinyl)carbonyl)ethenyl)phenyl]sulfid;
[2-(4-Acetylpiperazin-1-yl)phenyl][2,3-dichlor-4-(E-[(4-carboxypiperidin-1-yl)carbonyl]ethenyl)phenyl]sulfid; und
(2-Methoxyphenyl)[2,3-dichlor-4-(E-((((4-carboxyphenyl)methyl)amino)-carbonyl)ethenyl)phenyl]sulfid.

**18.** Pharmazeutische Zusammensetzung, die eine Verbindung gemäß Anspruch 1 oder 17 in einem pharmazeutisch annehmbaren Träger umfasst.

**19.** Verwendung einer Verbindung gemäß Anspruch 1, 16 oder 17 zur Herstellung eines Medikaments zur Entzündungshemmung.

**20.** Verwendung einer Verbindung gemäß Anspruch 1, 16 oder 17 zur Herstellung eines Medikaments zur Unterdrükkung einer Immunantwort.

**21.** Verbindung der Formel II

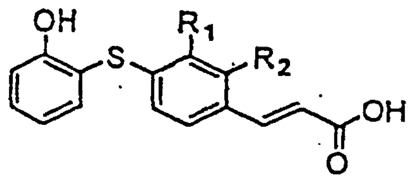

II

wobei $R_1$ und $R_2$ unabhängig ausgewählt sind aus

i. Wasserstoff;
j. Halogen;
k. Alkyl;
l. Halogenalkyl ;
m. Alkoxy;
n. Cyano;
o. Nitro; und
p. Carboxaldehyd.

**22.** Verfahren zur Herstellung einer Verbindung der Formel II

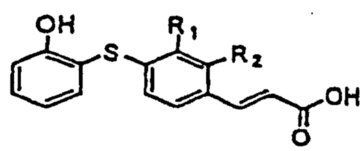

II

das folgendes umfasst:

a) Umsetzen einer Verbindung der Formel II':

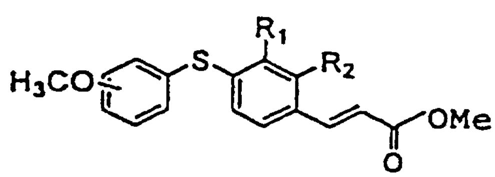

II'

mit Lithiumhydroxid; und

b) Spalten des resultierenden Methylethers, wobei $R_1$ und $R_2$ wie in Anspruch 21 definiert sind.

## Revendications

**1.** Composé de formule I

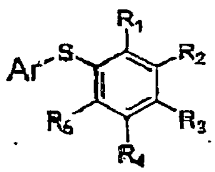

I

dans lequel $R_1$, $R_2$, $R_3$, $R_4$ et $R_5$ sont indépendamment choisis parmi

a. l'atome d'hydrogène,
b. un atome d'halogène,
c. un alkyle,
d. un alkyle substitué par un atome d'halogène,
e. un alcoxy,
f. un cyano,
g. un nitro,
h. un carboxaldéhyde, et

à la condition qu'au moins l'un de $R_1$ ou de $R_3$ soit un « *cis*-cinnamide » ou un « *trans*-cinnamide », défini tel que

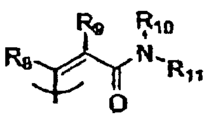

"*cis*-cinnamide"

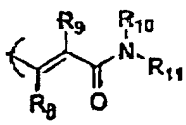

"*trans*-cinnamide",

dans lequel
$R_8$ et $R_9$ sont indépendamment choisis parmi

a. l'atome d'hydrogène,
b. un alkyle,
c. un carboxyalkyle,
d. un alkylaminocarbonylalkyle, et
e. un dialkylaminocarbonylalkyle,

et
$R_{10}$ et $R_{11}$ sont indépendamment choisis parmi

a. l'atome d'hydrogène,
b. un alkyle,
c. un cycloalkyle,
d. un alcoxycarbonylalkyle,
e. un hydroxyalkyle,
f. un groupe aryle étant un système carbocyclique mono- ou bicyclique ayant un ou deux cycles aromatiques qui peut être condensé à un cycle cyclohexane, cyclohexène, cyclopentane ou cyclopentène, et ayant des substituants indépendamment choisis parmi un alkyle, un atome d'halogène, un hydroxyle et un alcoxy,
g. un hétérocyclyle représentant un cycle à 4, 5, 6 ou 7 chaînons qui contient un, deux ou trois hétéroatomes indépendamment choisis parmi l'azote, l'oxygène et le soufre, qui peut être condensé à un ou deux cycles indépendamment choisis parmi un cycle aryle défini comme ci-dessous, un cycle cyclohexane, un cycle cyclohexène, un cycle cyclopentane, un cycle cyclopentène ou un autre cycle hétérocyclique monocyclique,
h. un hétérocyclylalkyle, dans lequel l'hétérocyclyle est défini comme ci-dessus,
i. un hétérocyclylamino, dans lequel l'hétérocyclyle est défini comme ci-dessus,
j. un groupe hétérocyclyle défini comme dans g. ci-dessus, ayant des substituants indépendamment choisis parmi un alkyle, un atome d'halogène, un hydroxyle, un alcoxy, un carboxyle, un carboxyalkyle et un alcoxycarbonyle,
k. un groupe hétérocyclylalkyle défini comme dans h. ci-dessus, ayant des substituants indépendamment choisis parmi un alkyle, un atome d'halogène, un hydroxyle, un alcoxy, un carboxyle, un carboxyalkyle et un alcoxycarbonyle, ou

dans lequel $NR_{10}R_{11}$ est un groupe hétérocyclyle ou un groupe hétérocyclyle substitué dans lequel le groupe hétérocyclyle est défini comme dans g. ci-dessus, et les substituants sont indépendamment choisis parmi

1) un alkyle,
2) un alcoxy,
3) un alcoxyalkyle,
4) un cycloalkyle,
5) un groupe aryle étant un système carbocyclique mono- ou bicyclique ayant un ou deux cycles aromatiques qui peut être condensé à un cycle cyclohexane, cyclohexène, cyclopentane ou cyclopentène,
6) un groupe hétérocyclyle défini comme ci-dessus,
7) un hétérocyclylcarbonyle, dans lequel l'hétérocyclyle est défini comme ci-dessus,
8) un hetérocyclylalkylaminocarbonyle, dans lequel l'hétérocyclyle est défini comme ci-dessus,
9) un hydroxyle,
10) un hydroxyalkyle,
11) un hydroxyalcoxyalkyle,
12) un carboxyle,
13) un carboxyalkyle,
14) un carboxycarbonyle,
15) un carboxaldéhyde,
16) un alcoxycarbonyle,
17) un arylalcoxycarbonyle, dans lequel l'aryle est défini comme ci-dessus,
18) un aminoalkyle,
19) un aminoalcanoyle,
20) un carboxamido,
21) un alcoxycarbonylalkyle,
22) un carboxamidoalkyle,
23) un cyano,
24) un tétrazolyle,
25) un tétrazolyle substitué par un alcoxy, un alkylc, un atome d'halogène, un hydroxyle, un carboxyle, un carboxyalkyle ou un alcoxycarbonyle,
26) un alcanoyle,
27) un hydroxyalcanoyle,
28) un alcanoyloxy,
29) un alcanoylamino,
30) un alcanoyloxyalkyle,
31) un alcanoylaminoalkyle,
32) un sulfonate,
33) un alkylsulfonyle,
34) un alkysulfonylaminocarbonyle,
35) un arylsulfonylaminocarbonyle, dans lequel l'aryle est défini comme ci-dessus, et
36) un hétérocyclylsulfonylaminocarbonyle, dans lequel le groupe hétérocyclyle est défini comme ci-dessus,

et dans lequel Ar est un groupe aryle ou un groupe hétéroaryle substitué, qui a un ou deux cycles aromatiques et qui peut être condensé à un cycle cyclohexane, cyclohexène, cyclopentane ou cyclopentène, dans lequel les substitutions sont indépendamment choisies parmi

a. un groupe hétérocyclyle défini comme ci-dessus, ayant des substituants indépendamment choisis parmi un alkyle, un atome d'halogène, un hydroxyle, un alcoxy, un carboxyle, un carboxyalkyle et un alcoxycarbonyle,

b. un groupe hétérocyclylalkyle défini comme ci-dessus, ayant des substituants indépendamment choisis parmi un carboxyle, un carboxyalkyle et un alcoxycarbonyle,
c. un carboxyalcoxy,
d. un carboxythioalcoxy,
e. un carboxycycloalcoxy,
f. un thioalkyle, et
g. un carboxyalkylamino ;

ou un sel pharmaceutiquement acceptable de celui-ci.

**2.** Composé selon la revendication 1, dans lequel $R_1$ est un « *cis*-cinnamide » ou un « *trans*-cinnamide », et $R_3$ est un atome d'hydrogène.

**3.** Composé selon la revendication 1, dans lequel $R_3$ est un « *cis*-cinnamide » ou un « *trans*-cinnamide », et $R_1$ est un atome d'hydrogène.

**4.** Composé selon la revendication 1, dans lequel $R_3$ est un *« cis*-cinnamide» ou un « *trans*-cinnamide », et $R_1$, $R_8$, et $R_9$ sont des atomes d'hydrogène.

**5.** Composé selon la revendication 4, dans lequel $R_3$ est un « *cis*-cinnamide ».

**6.** Composé selon la revendication 4, dans lequel $R_3$ est un « *trans*-cinnamide ».

**7.** Composé selon la revendication 1, dans lequel $R_3$ est un *« cis*-cinnamide » ou un « *trans*-cinnamide », $R_1$, $R_2$, et $R_4$ représentent, indépendamment les uns des autres, l'atome d'hydrogène ou un groupe alkyle ; et $R_5$ est choisi parmi un atome d'halogène, un alkyle substitué par un atome d'halogène, et un nitro.

**8.** Composé selon la revendication 4, dans lequel $R_{10}$ et $R_{11}$ sont chacun indépendamment choisis parmi l'atome d'hydrogène, un alkyle, un cycloalkyle, un alcoxycarbonylalkyle, un hydroxyalkyle, et un hétérocyclylalkyle.

**9.** Composé selon la revendication 4, dans lequel $NR_{10}R_{11}$ est un hétérocyclyle ou un hétérocyclyle substitué.

**10.** Composé selon la revendication 1, dans lequel Ar est choisi parmi un phényle substitué, la 1,3-benzimidazol-2-one, la 1,4-benzodioxane, le 1,3-benzodioxole, la 1-benzopyr-2-èn-4-one, l'indole, l'isatine, la 1,3-quinazolin-4-one, et la guinoline.

**11.** Composé selon la revendication 1, dans lequel Ar est la benzodioxane substituée.

**12.** Composé selon la revendication 1, dans lequel $R_3$ est un « trans-cinnamide » ; et Ar est choisi parmi la 1,3-benzimidazol-2-one, la 1,4-benzodioxane, le 1,3-benzodioxole, la 1-benzopyr-2-èn-4-one, l'indole, l'isatine, le phényle, la 1,3-quinazolin-4-one et la quinoline.

**13.** Composé selon la revendication 1, dans lequel $R_{10}$ et $R_{11}$ sont indépendamment choisis parmi l'atome d'hydrogène, un alkyle, un cycloalkyle, un alcoxycarbonylalkyle, un hydroxyalkyle, et un hétérocyclylalkyle.

**14.** Composé selon la revendication 1, dans lequel $NR_{10}R_{11}$est un hétérocyclyle ou un hétérocyclyle substitué.

**15.** Composé de la revendication 1, à la condition que lorsque trois des groupes $R_1$, $R_2$, $R_4$ et $R_5$ représentent l'atome d'hydrogène, le groupe restant $R_1$, $R_2$, $R_4$, ou $R_5$, n'est pas un carboxyle, le 5-tétrazolyle, l'hydroxyméthyle ou un carboxyle dérivé sous la forme d'un ester pharmaceutiquement acceptable.

**16.** Composé selon la revendication 1, choisi dans le groupe consistant en :

le sulfure de 3-(1-(3-carboxy)pipéridinyl)phényle et de 2,3-dichloro-4-(*E*-((1,2,5,6-tétrahydropyridin-1-yl)carbonyl)éthényl)phényle ;
le sulfure de 3-(3-carboxylpipéridin-1-yl)phényle et de 2,3-dichloro-4-(*E*-[(4-carboxypipéridin-1-yl)carbonyl] éthényl)phenyle ;
le sulfure de 3-(4-carboxylpipéridin-1-yl)phényle et de 2,3-dichloro-4-(*E*-[(1,2,3,6-tétrahydropyridin-1-yl)carbanyl]éthényl)phényle ;
le sulfure de 3-(4-carboxylpipéridinyl)phényle et de 2,3-dichloro-4-(*E*-[(4-morpholinyl)carbonyl]éthényl) phényle ;
le sulfure de 3-(3-carboxypipéridin-1-yl)phényle et de 2,3-dichloro-4-(*E*-[(4-morpholinyl)carbonyl]éthényl) phényle ;
le sulfure de 3-(4-carboxypipéridin-1-yl)phényle et de 2,3-dichloro-4-(*E*-((4-hydroxypipéridin-1-yl)carbonyl) éthényl)phényle ;
le sulfure de 2-glycoxyphényle et de 2,3-dichloro-4-(*E*-((4-morpholino)carbonyl)éthényl)phényle ;
le sulfure de 2-(4-butyroxy)phényle et de 2,3-dichloro-4-(*E*-((4-morpholino)carbonyl)éthényl)phényle] ;
le sulfure de 3-(4-carboxypipéridin-1-yl)phényle et de 2,3-dichloro-4-(*E*-((4-hydroxyéthylpipérazin-1-yl)carbonyl)éthényl)phényle ;
le sulfure de 3-(4-carboxypipéridin-1-yl)phényle et de 2,3-dichloro-4-(*E*-((4-furoylpipérazin-1-yl)carbonyl)éthényl)phényle ;
le sulfure de 3-(4-carboxypipéridin-1-yl)phényle et de 2,3-dichloro-4-(*E*-((pyrrolidin-1-yl)carbonyl)éthényl)phényle;
le sulfure de 3-(4-carboxypipéridin-1-yl)phényle et de 2,3-dichloro-4-(*E*-(diéthylarninocarbvnyl)éthényl) phényle ;
le sulfure de 3-(4-carboxypipéridin-1-yl)phényle et de 2,3-dichloro-4-(*E*-((4-éthylpipérazin-1-yl)carbonyl)éthényl)phényle ;
le sulfure de 3-(4-carboxypipéridin-1-yl)phényle et de 2,3-dichloro-4-(*E*-((4-(aminocarbonyl)pipéridin-1-yl)carbonyl)éthényl)phényle ;
le sulfure de 3-(4-carboxypipéridin-1-yl)phényle et de 2,3-dichloro-4-(*E*-(4-(2-(éthtoxyéthyl)pipénidin-1-yl)carbonyl)éthényl)phényle ;
le sulfure de 3-(4-carboxypipéridin-1-yl)phényle et de 2,3-bis(trifluorométhyl)-4-(*E*-((4-morpholino)carbonyl) éthényl)phényle ;
le sulfure de 3-(4-butyroxy)phényle et de 2,3-dichloro-4-(*E*-((4-morpholino)carbonyl)éthényl)phényle;
le sulfure de 3-(4-carboxypipéridin-1-yl)phényle et de 2,3-bis(trifluorométhyl)-4-(*E*-((4-hydroxypipéridin-1-yl) carbonyl)éthényl)phényle ;
le sulfure de 3-(4-carboxypipéridin-1-yl)phényle et de 2,3-bis(trifluorométhyl)-4-(*E*-((1,2,5,6-tétrahydropyridin-1-yl)carbonyl)éthényl)phényle ;
le sulfure de 2-((4-carboxy)butyloxy)phényle et de 2,3-dichloro-4-(*E*-((4-morpholino)carbonyl)éthényl)phényle;
le sulfure de 2-glycoxyphényle et de 2,3-bis(trifluorométhyl)-4-(*E*-((4-morpholino)carbonyl)éthényl)phenyle ;
le sulfure de 2-(4-butyroxy)phényle et de 2,3-bis(trifluorométhyl)-4-(*E* ((4-morpholino)carbonyl)éthényl)phényle,
le sulfure de 3-(4-carboxypipéridin-1-yl)phényle et de 2,3-bis(trifluorométhyl)-4-(*E*((bis-(2-éthoxyéthyl)amino) carbonyl)éthényl)phényle ;
le sulfure de 3-(4-carboxypipéridin-1-yl)phényle et de 2,3-bis-(trifluorométhyl)-4-(*E*-((bis-(2-hydroxypropyl) amino)carbonyl)éthényl)phényle ;
le sulfure de 3-(4-carboxypipéridin-1-yl)phényle et de 2,3-bis-(trifluorométhyl)-4-(*E*-((pipérazin-1-yl)carbonyl) phényle ;
le sulfure de 3-(4-butyroxy)phényle et de 2,3-bis(trifluorométhyl)-4-(*E*-((4-morpholino)carbonyl)éthényl) phényle ;
le sulfure de 2-(3-carboxypipéridin-1-yl)phényle et de 2,3-dichloro-4-(*E*-((4-(2-hydroxyéthyl)pipérazin-1-yl)carbonyl)éthényl)phényle ;
le sulfure de 2-(3-carboxypipéridin-1-yl)phényle et de 2,3-bis(trifluorométhyl)-4-(*E*-((1,2,5,6-tétrahydropyridin-1-yl)carbonyl)éthényl)phényle ;
le sulfure de 2-(3-carboxypipéridin-1-yl)phényle et de 2,3-bis(trifluorométhyl)-4-(*E*-((4-(2-hydroxyéthyl)pipérazin-1-yl)carbonyl)éthényl)phényle ;
le sulfure de 2-(3-carboxypipéridin-1-yl)phényle et de 2,3-bis(trifluorométhyl)-*4-(E-((*4-(2-(hydroxyéthoxy) éthyl)pipérazin-1-yl)carbonyl)éthényl)phényle ;
le sulfure de 3-(3-propioxy)phényle et de 2,3-dichloro-4-(E-((4-morpholino)carbonyl)éthényl)phényle ;
le sulfure de 3-(4-carboxypipéridin-1-yl)phényle et de 2,3-dichloro-4-(*E*-[(4-(diméthylaminosulfanoyl)pipérazin-1-yl)carbonyl]éthényl)phényle ;
le sulfure de 3-(4-carboxypipéridin-1-yl)phényle et de 2,3-dichloro-4-(*E*-((4-((trifluorométhylsulfonyl)pipérazin-1-yl)carbonyl)éthényl)phényle ;
le sulfure de 3-(4-carboxypipéridin-1-yl)phényle et de 2,3-dichloro-4-(*E* ((4-(méthylsulfonyl)pipérazin-1-yl)carbonyl)éthényl)phényle ;

le sulfure de 3-(4-carboxypipéridin-1-yl)phényle et de 2,3-dichloro-4-(*E*-((S-oxothiomorpholin-1-yl)carbonyl) éthényl)phényle ;
le sulfure de 2-(3-carboxypipéridin-1-yl)phényle et de 2,3-dichloro-4-(*E*-(3-(2-pyrrolidinon-1-yl)propylaminocarbonyl)éthényl)phényle; et
le sulfure de 2-(3-carboxypipéridin-1-yl)phényle et de 2,3-bis(trifluorométhyl)-4-(*E*-(3-(2-pyrrolidinon-1-yl)propylaminocarbonyl)éthényl)phényle.

**17.** Composé choisi dans le groupe consistant en :

le sulfure de 2-méthoxyphényle et de 2,3-dichloro-4-(*E*-(((4-hydroxylaminocarbonyl)pipéridin-1-yl)carbonyl) éthényl)phényle ;
le sulfure de 2-méthoxyphényle et de 2,3-dichloro-4-(*E*-((N-carboxyméthyl-N-phénylamino)carbonyl)éthenyl) phényle
le sulfure de 2-méthoxyphényle et de 3-chloro-6-hydroxy-4-(*E*-((3-carboxypipéridin-1-yl)carbonyl)éthényl) phényle ;
le sulfure de 2-méthoxyphényle et de 2,3-dichloro-4-(*E*-(4-((1-(2-phényl-1-carboxyéthylamino)carbonyl)pipéridin-1-yl)carbonyl)éthényl)phényle ;
le sulfure de 2-méthoxyphényle et de 2,3-dichloro=4-(*E*-(4-((1-(2-hydroxy-1-carboxyéthylamino)carbonyl)pipéridin-1-yl)carbonyl)éthényl)phényle ;
le sulfure de 3-(4-(pyrrolidin-1-yl)pipéridin-1-yl)phényle et de 2,3-dichloro-4-(*E*-((3-(2-pyrrolidinon-1-yl)propylamino)carbonyl)éthényl)phényle ;
le sulfure de 3-(4-(spiro-2,2-dioxolanyl)pipéridin-1-yl)phényle et de 2,3-dichloro-4-(*E*-((4-morpholinyl)carbonyl)éthényl)phényle ;
le sulfure de 2-(2-carboxyéthényl)phényle et de 2,3-dichloro-4-(*E*-((4-morpholinyl)carbonyl)éthényl)phényle ;
le sulfure de 2-(4-acétylpipérazin-1-yl)phényle et de 2,3-dichloro-4-(*E*-[(4-carboxypipéridin-1-yl)carbonyl]éthényl)phényle ; et
le sulfure de 2-méthoxyphényle et de 2,3-dichloro-4-(*E*-((((4-carboxyphényl)méthyl)amino)carbonyl)éthényl) phényle.

**18.** Composition pharmaceutique comprenant un composé de la revendication 1 ou 17 dans un véhicule pharmaceutiquement acceptable.

**19.** Utilisation d'un composé de la revendication 1, 16 ou 17 pour préparer un médicament pour l'inhibition de l'inflammation.

**20.** Utilisation d'un composé de la revendication 1, 16 ou 17 pour préparer un médicament pour la suppression de la réponse immunitaire.

**21.** Composé de formule II

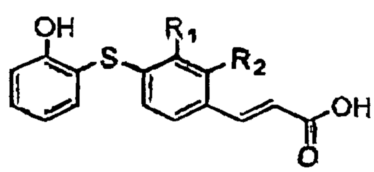

II

dans lequel $R_1$ et $R_2$ sont indépendamment choisis parmi

i. l'atome d'hydrogène,
j. un atome d'halogène,
k. un alkyle
l. un alkyle substitué par un atome d'halogène
m. un alcoxy,
n. un cyano,
o. un nitro, et

p. un carboxaldéhyde.

**22.** Procédé de préparation d'un composé de formule II ;

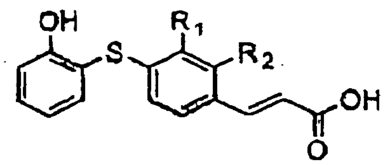

*II*

qui comprends

a) la réaction d'un composé de formule II'

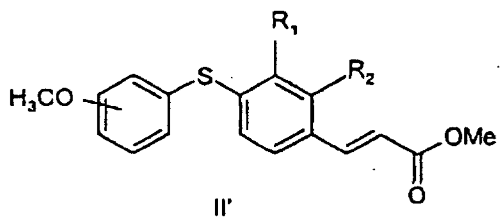

II'

avec de l'hydroxyde de lithium, et

b) coupure de l'éther méthylique en résultant ,

dans lequel $R_1$ et $R_2$ sont tels que définis à la revendication 21.